# EUROPEAN PATENT APPLICATION

(11) **EP 4 144 764 A1**
(43) Date of publication of application: **08.03.2023**
(21) Application number: 21796635.7
(22) Date of filing: 26.04.2021
(51) Int. Cl.: C07K 19/00, C07K 14/71, C07K 14/495, A61K 38/17, A61P 35/00

(54) **TGFßR2 EXTRACELLULAR DOMAIN TRUNCATED MOLECULE, FUSION PROTEIN OF TGFßR2 EXTRACELLULAR DOMAIN TRUNCATED MOLECULE AND ANTI-EGFR ANTIBODY, AND ANTI-TUMOR USE OF FUSION PROTEIN**

(30) Priority: 28.04.2020 CN 202010351280
(71) Applicant: Sinocelltech Ltd., Beijing 100176 (CN)
(72) Inventor: XIE, Liangzhi, Beijing 100176 (CN); SUN, Chunyun, Beijing 100176 (CN); GUO, Erhong, Beijing 100176 (CN)
(74) Representative: Dolphin, Kirsty Mairi
(86) International application number: PCT/CN2021/089782
(87) International publication number: WO 2021/218883

(57) **Abstract**

Provided are multiple types of TGFβR2 in truncated forms and a fusion protein constructed by TGFβR2 and EGFR antibody HPA8; also provided are a nucleic acid (comprising heavy/light chain variable regions) encoding the antibody, a vector, a pharmaceutical composition, and a kit comprising the nucleic acid; further provided is a fusion protein of the prepared truncated TGFβR2 receptor protein and a targeted EGFR and other multiple types of tumor target antibodies.

## Description

### CROSS-REFERENCING OF RELATED APPLICATIONS

This application claims the benefit of Chinese patent application 202010351280.6 filed on April 28, 2021, the contents of which are incorporated herein by reference.

### FIELD

The present invention relates to the field of tumor immunotherapeutic agents. Specifically, the invention relates to a truncated TGFβR2 (an immunomodulatory factor)extracellular domain molecule, a fusion protein comprising the truncated TGFβR2 extracellular domain molecule, and the targeting portion. The present invention comprises pharmaceutical compositions and their application as antitumor medicaments.

### BACKGROUND

Transforming growth factor-β (TGF-β) belongs to the TGF-β superfamily that regulates cell growth and differentiation, and is a pleiotropic and multifunctional cytokine that regulates cell proliferation, differentiation, and apoptosis through cell surface receptor signaling pathways in an autocrine or paracrine manner, and plays an important regulatory role in the synthesis of extracellular matrix, repair of trauma, and immune function, etc. Three isoforms, TGF-β1, TGF-β2, and TGF-β3, are present in mammals, and the most abundant and expressed isoform is TGF-β1. TGF-β initiates classical Smad and non-Smad pathways for downstream signaling by binding to TGFβR1 and TGFβR2 serine-threonine kinase receptors on the cell membrane surface.^{[1]}. In normal homeostasis *in vivo*, TGF-β signaling regulates key processes such as growth, regeneration, differentiation, etc. of various tissues. In the immune system, TGF-β triggers tolerance and suppresses inflammation. Genetic mutations can alter the TGF-β signaling in tumor-initiating cells. In the initial stages of tumorigenesis, TGF-β plays a key cancer-inhibiting role by inhibiting cell proliferation and triggering the apoptotic program. However, as tumors progress, selective pressure leads to the loss of the tumor suppressive function of TGF-β by tumor cells through different mechanisms. Acquired inactivating mutations in the TGF-β signaling pathway allow a variety of malignant cells to grow in a TGF-β-enriched environment. In addition, tumor cells somehow convert the pro-apoptotic capacity of TGF-β into pro-tumor developmental functions, such as invasion and migration capacity, and promotion of mesenchymal transition.^{[2-4]} TGF-β regulates a variety of immune cell types and functions. TGF-β controls adaptive immunity by directly promoting the proliferation of Treg cells thus inhibiting the production and function of effector T cells and antigen-presenting dendritic cells. TGF-β also inhibits NK cell function and converts macrophages and neutrophils into pro-tumor growth subtypes, promoting the formation of a tumor microenvironment with negative tumor immune regulation.^{[4]} TGF-β1 is often expressed at higher levels than normal paraneoplastic tissue in a variety of solid tumors including EGFR-positive colorectal cancer, non-small cell lung cancer, and head and neck squamous cell carcinoma. Clinical data suggest that blocking the TGF-β pathway alone is not sufficient to fully restore the immune system to suppress tumorigenesis, and therefore no TGF-β antibodies are yet available.

Epidermal growth factor receptor (EGFR) is an expression product of the proto-oncogene *C-ErbB1, a* receptor for cell proliferation and signaling of epithelial growth factor (EGF), a member of the epidermal growth factor receptor (HER) family, and a tyrosine kinase receptor with a molecular weight of 170 kDa.^{[5]} EGFR is divided into an extracellular ligand-binding region, a transmembrane region, and an intracellular kinase region. The extracellular domain of EGFR is transformed from monomer to dimer after binding to the ligand, activates the intracellular kinase region and multiple downstream signaling pathways, and plays an important role in cell growth, proliferation and differentiation.^{[6]} High expression of EGFR causes enhanced downstream signaling, increased expression of mutant EGFR receptors or ligands leads to sustained activation of EGFR, enhanced activity of the secretory loop and disruption of receptor down-regulation mechanism, etc. in turn activates genes related to tumor proliferation and differentiation and plays an important role in tumor formation and development.^{[7]} EGFR overexpression is associated with reduced survival in several cancer types, including head and neck, bladder, ovarian, cervical, and esophageal cancers. In addition, anti-EGFR medicaments have been shown to be effective in the treatment of several types of solid tumors such as colorectal, head and neck, non-small cell lung (NSCLC), and pancreatic cancers, including overall survival, progression-free survival, and overall response rates ^{[8]}. Therefore, as a clear target associated with tumor proliferation, EGFR-targeted agents have become the first-line treatment option for a variety of malignancies.

Although the signals initiated by TGF-β are different from those initiated by the EGF/EGFR pathway, the signaling pathways between the two can interact. TGF-β and EGFR signaling have been found to interact and jointly promote tumor progression in a variety of tumors. TGF-β can induce EGFR trans-activation in a highly cell type and context-specific mode. For example, TGF-β promotes the migration and invasion of breast cancer cells (MDA-MB-231, T47D, 4T1) by upregulating EGFR through the classical Smad and ERK/Sp1 signaling pathways^{[9, 10]}. In squamous cell carcinoma (A431, SCC13), TGF-β activates the EGFR pathway through H₂O₂ -dependent mechanisms to increase Erk1/2 phosphorylation levels ^{[11]}. EGF and its associated downstream signaling pathways can also regulate TGF-β signaling in different cell types. For example, in human primary ovarian cancer cells, EGF reduces the sensitivity of ovarian cancer cells to the anti-proliferative effects of TGF-β by decreasing the mRNA expression of the TGF-β-inducible cell cycle regulator p15 ^{INK4B[12]}. Oncogenic Ras in breast and lung epithelial cells reduces the cell growth inhibitory effect of TGF-β by inhibiting TGF-β-mediated signaling via negative regulation of Smad2 and Smad3 ^{[13, 14]} EGF also positively regulates Smad2 signaling in COS7 cells by increasing Smad2 phosphorylation through the ERK pathway^{[15]}.

TGF-β and EGF synergistically promote the malignant phenotype of tumors, and studies on different tissues have shown that EGF combined with TGF-β enhances epithelial to mesenchymal transition (EMT). For example, EGF and TGF-β1 promote the expression of laminin-332 (Laminin-332) to synergistically promote EMT in oral epithelial cancer.^{[16]} EGF and TGF-β1 promote EMT in intestinal epithelial cells by downregulating E-cadherin via the MAPK pathway rather than PI3K, p38MAPK, JNK, or AP-1 pathway^{[17]}. EGF and TGF-β1 induce Slug and Snail expression via Smad and MEK1/2-dependent signaling pathways, downregulate E-cadherin, and promote EMT in ovarian epithelial cells^{[18]}. EGF and TGF-β1 activate the ERK1/2 signaling pathway, synergistically upregulate Snail protein expression, and promote EMT and migration in human renal cortical proximal tubule epithelial cells (HK-2)^{[19]}. EGF enhances TGF-β-induced EMT in lung cancer (H322, H358) and pancreatic cancer (HPAF-II, CAPAN-2) cells by promoting the binding of SHP2 to GAB1.^{[20]}.

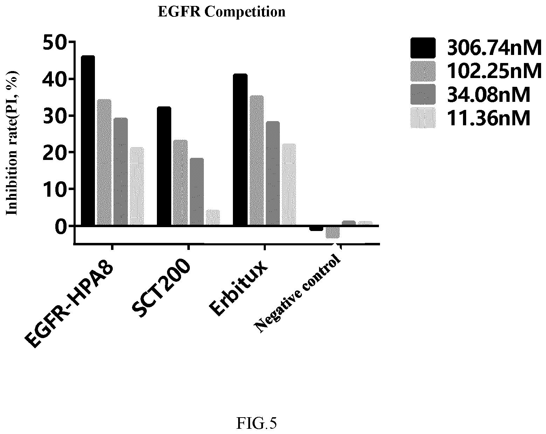

Several clinical studies have shown that elevated TGF-β levels are closely associated with the development of medicament resistance and poor prognosis. TGF-β1-induced EMT in osteosarcoma cancer stem-like cells decreases miR-499a expression, leading to increased SHKBP1 expression occurring concomitantly with a TGFβ-induced EMT-associated kinase switch to an AKT-activated EGFR-independent state, thereby reducing EGFR activity and inducing osteosarcoma resistance to EGFR kinase inhibitors (FIG. 5).^{[21]} . Treg cells are one of the main cells that produce TGF-β. The increased number of Treg in tumors of patients with head and neck squamous carcinoma treated with Cetuximab is accompanied by an increase in TGF-β content while the TGF-β content in patients with poor Cetuximab efficacy is even high ^{[22]}. Elevated TGF-β may induce resistance to EGFR antibody therapy by inhibiting the expression of relevant molecular effectors of effector cell-mediated cytotoxicity, activating the EGFR-independent AKT pathway and enhancing EMT inducition.^{[23]} In EGFR mutant non-small cell lung cancer, the classical TGF-β/Smad signaling pathway is involved in the development of PD-L1-induced tumor resistance to EGFR kinase inhibitors.^{[24]} . In breast cancer tissues, TGF-β expression was positively correlated with EGFR expression, and elevated levels of TGF-β and EGFR were associated with poor prognosis in breast cancer patients^{[9]}. In summary, EGFR and TGF-β play relatively independent and closely related roles in the process of tumor development. In addition, TGF-β is a key molecule in the development of acquired resistance in tumors against EGFR-targeted therapy. Animal studies have shown that inhibition of TGF-β improves the in vivo antitumor effect of Cetuximab on head and neck squamous cell tumor xenografts^{[23]}. These provide a theoretical basis for combining targeting TGF-β to enhance the therapeutic efficacy of EGFR antibodies against EGFR-positive tumors.

The present invention provides novel fusion proteins comprising truncated TGFβR2 that can specifically target both EGFR and TGF-β, two relatively independent and closely related signaling pathways. It is used to treat solid tumors including but not limited to gastric cancer.

### SUMMARY

In one aspect, the present invention provides a truncated TGFβR2 extracellular domain molecule that, compared to its natural form,
a) at least the amino acid residues at positions 6-16 thereof are deleted, and further optionally, the amino acid residues at positions 17- 17+n thereof are deleted, where n is an integer from 1-10; preferably, n is 2, 4, 8, 9 or 10; most preferably, n is 9; or
b) on the basis of the deletion of amino acid residues thereof at positions 6-26, furthermore, the amino acid residues thereof at positions 5, 4-5, 3-5, 2-5, 1, 1-2, 1-3, or 1-4 are deleted; or
c) the amino acid residues at positions 7-26 are deleted.

In one embodiment, the amino acid sequence comprises any of SEQ ID NO: 48-62.

In another aspect, the present invention provides a fusion protein comprising the molecules described herein.

In one embodiment, said fusion protein comprises
a) said truncated TGFβR2 extracellular domain molecule and
b) a targeting portion.

In one embodiment, said fusion protein targeting portion is a cancer cell-specific targeting portion selected from an antibody or antigen-binding fragment thereof, a functional ligand or Fc fusion protein thereof, and a receptor protein or Fc fusion protein thereof.

In one embodiment, said fusion protein targeting portion is an anti-EGFR antibody or antigen-binding fragment thereof.

In one embodiment, the N-terminal of the truncated TGFβR2 extracellular domain molecule in a said fusion protein is linked to the C-terminal of the heavy chain of the targeting portion, optionally, by a linker.

In one embodiment, said linker is preferably a G₄ S flexible peptied linker, preferably a (G₄ S)₄ peptied linker.

In a further aspect, the present invention provides an isolated binding antibody or an antigen-binding fragment thereof to EGFR, comprising
(a) a heavy chain variable region comprising heavy chain CDR1, heavy chain CDR2, and heavy chain CDR3 domains comprising SEQ ID NOs: 19, 20, and 21, respectively, and/or
(b) a light chain variable region comprising a light chain CDR1, light chain CDR2, and light chain CDR3 domain comprising SEQ ID NOs: 16, 17, and 18, respectively.

In one embodiment, said antibody or antigen-binding fragment thereof comprises
a) a heavy chain variable region comprising a sequence comprising SEQ ID NO:28 or a sequence having at least 85%, 88%, 90%, 95%, 98%, or 99% sequence identity therewith; and/or
(b) a light chain variable region comprising a sequence comprising SEQ ID NO: 29 or a sequence having at least 85%, 88%, 90%, 95%, 98%, or 99% sequence identity therewith.

In one embodiment, said antibody further comprises:
a) a heavy chain constant region, comprising a sequence comprising SEQ ID NO: 30 or a sequence having at least 85%, 88%, 90%, 95%, 98%, or 99% sequence identity therewith; and/or
b) a light chain constant region, comprising a sequence comprising SEQ ID NO: 31 or a sequence having at least 85%, 88%, 90%, 95%, 98%, or 99% sequence identity therewith.

In one embodiment, the targeting portion of the said fusion protein is selected from said anti-EGFR antibody, Trastuzumab, Bevacizumab, Ramucirumab, Ipilimumab, or Panitumumab.

In one embodiment, said fusion protein comprises
a) the amino acid sequence of a heavy chain comprises SEQ ID NO: 141 or a sequence having at least 85%, 88%, 90%, 95%, 98%, or 99% sequence identity therewith; and
b) the amino acid sequence of a light chain comprising SEQ ID NO:23 or a sequence having at least 85%, 88%, 90%, 95%, 98%, or 99% sequence identity therewith.
which comprises two heavy chains and two light chains; a disulfide bond is formed between a first light chain and a first heavy chain thereof, a disulfide bond is formed between a second light chain and a second heavy chain thereof, and a disulfide bond is formed between a first heavy chain and a second heavy chain thereof.

In one embodiment, said fusion protein has a KD value of 2.92 pM-26.3 pM, preferably 7 pM-9 pM, most preferably 8.77 pM, for binding affinity to human EGFR protein; and
has a KD value of 23 pM - 288.3 pM, preferably 64 pM - 144 pM, most preferably 96.1 pM for binding affinity to human TGF-β1 protein.

In a further aspect, the present invention provides a conjugate comprising the truncated TGFβR2 extracellular domain molecule as described herein, the fusion protein as described herein, the antibody or the antigen-binding fragment thereof as described herein, and an additional therapeutic agent, preferably said antibody or antigen-binding fragment thereof and the additional therapeutic agent are linked by a linker.

In a further aspect, the present invention provides a nucleic acid encoding the truncated TGFβR2 extracellular domain molecule as described herein, the fusion protein as described herein, the antibody or an antigen-binding fragment thereof as described herein, which is mRNA and/or DNA.

In one embodiment, said nucleic acid comprises
any one of SEQ ID NOs: 32 to 39;
any one of SEQ ID NOs: 67-84; or
any one of SEQ ID NOs: 148-163, or an functional variant thereof.

In a further aspect, the present invention provides an expression vector comprising the nucleic acid described herein.

In a further aspect, the present invention provides a host cell comprising the nucleic acid as described herein or the expression vector as described herein.

In a further aspect, the present invention provides a method for producing the truncated TGFβR2 extracellular domain molecule as described herein, the fusion protein as described herein, the antibody or an antigen-binding fragment thereof as described herein, comprising culturing the host cell as described herein under conditions suitable for expression of the preceding protein molecule, and recovering the expressed product from the culture medium.

In a further aspect, the present invention provides a pharmaceutical composition comprising
a) the truncated TGFβR2 extracellular domain molecule as described herein, the fusion protein as described herein, the antibody or an antigen-binding fragment thereof as described herein, the conjugate as described herein, the nucleic acid as described herein, or the expression vector as described herein; and
b) a pharmaceutically acceptable carrier; optionally
c) one or more other therapeutic agents.

In a further aspect, the present invention provides use of the truncated TGFβR2 extracellular domain molecule as described herein, the fusion protein as described herein, the antibody or an antigen-binding fragment thereof as described herein, the conjugate as described herein, the nucleic acid as described herein, the expression vector as described herein, or the pharmaceutical composition as described herein for the prevention and treatment of cancer, preferably for the treatment of gastric cancer.

In a further aspect, the present invention provides use of the truncated TGFβR2 extracellular domain molecule as described herein, the fusion protein as described herein, the antibody or an antigen-binding fragment thereof as described herein, the conjugate as described herein, the nucleic acid as described herein, the expression vector as described herein, or the pharmaceutical composition as described herein for the preparation of a medicament for the prevention and treatment of cancer, preferably for the treatment of gastric cancer.

In a further aspect, the present invention provides a pharmaceutical combination comprising the truncated TGFβR2 extracellular domain molecule as described herein, the fusion protein as described herein, the antibody or an antigen-binding fragment thereof as described herein, the conjugate as described herein, the nucleic acid as described herein, the expression vector as described herein, or the pharmaceutical composition as described herein, and one or more additional therapeutic agent(s).

In a further aspect, the present invention provides a kit comprising the truncated TGFβR2 extracellular domain molecule as described herein, the fusion protein as described herein, the antibody or an antigen-binding fragment thereof as described herein, the conjugate as described herein, the nucleic acid as described herein, the expression vector as described herein, or the pharmaceutical composition as described herein; preferably, further comprising a device for administering a medicament.

In a further aspect, the present invention provides a method of preventing and treating a neoplastic disease comprising administering to a subject the molecule of the truncated TGFβR2 extracellular domain molecule as described herein, the fusion protein as described herein, the antibody or an antigen-binding fragment thereof as described herein, the conjugate as described herein, the nucleic acid as described herein, the expression vector as described herein, or the pharmaceutical composition as described herein.

### BRIEF DESCRIPTION OF THE DRAWINGS

FIG. 1 Binding assay of murine antibody EGFR-mhPA8 to recombinant human EGFR-His protein.
FIG. 2 Blockage ofBlockage ofEGF binding to EGFR by the murine antibody EGFR-mhPA8.
FIG. 3 Inhibition of proliferation of MDA-MB-468 cells by murine antibody EGFR-mhPA8.
FIG. 4 Binding assay of humanized antibody EGFR-HPA8 to recombinant human EGFR-His protein.
FIG. 5 Blockage ofBlockage ofEGF binding to EGFR by humanized antibody EGFR-HPA8.
FIG. 6 Inhibition of proliferation of MDA-MB-468 cells by humanized antibody EGFR-HPA8 under different ligand conditions.
FIG. 7 Inhibition of proliferation of Fadu cells by humanized antibody EGFR-HPA8 under different ligand conditions.
FIG. 8 Effect of ADCC mediated by humanized antibody EGFR-HPA8.
FIG. 9 Effect of EGFR-HPA8 on the body weight of gastric cancer cell SNU-5 subcutaneously xenografted mice.
FIG. 10 Effect of EGFR-HPA8 on tumor volume and TGI results of gastric cancer cell SNU-5 subcutaneously xenografted mice.
FIG. 11 Effect of EGFR-HPA8 on the body weight of non-small cell lung cancer NCI-H1975 cell subcutaneously xenografted mice.
FIG. 12 Effect of EGFR-HPA8 on tumor volume and TGI results in non-small cell lung cancer NCI-H1975 cell subcutaneously xenografted mice.
FIG. 13 Schematic structure of EGFR antibody/TGFβR2 fusion protein.
FIG. 14 Detection of degradation of EGFR antibody/TGFβR2 fusion protein.
FIG. 15 Detection of degradation propensity of EGFR antibody/TGFβR2 fusion protein.
FIG. 16 Detection of the binding ability of different truncated forms of EGFR antibody/TGFβR2 fusion protein to TGF-β1 and EGFR.
FIG. 17 Detection of the ability of EGFR antibody/TGFβR2 fusion protein TGFβR2 truncated EGFR antibody/TGFβR2 fusion protein to neutralize TGF-β1.
FIG. 18 Detection of the ability of EGFR antibody/TGFβR2 fusion protein TGFβR2 truncated EGFR antibody/TGFβR2 fusion protein to neutralize TGF-β3.
FIG. 19 Assay of the ability of fusion protein 6 to bind to TGF-β1 and TGF-β3 proteins respectively.
FIG. 20 Assay of the ability of fusion protein 6 to block TGF-β1 protein or TGF-β3 protein from binding TGFβR2-Fc respectively.
FIG. 21 Assay of the binding and competition ability of fusion protein 6.
FIG. 22 Affinity assay of fusion protein 6 for recombinant human EGFR protein.
FIG. 23 Affinity assay of fusion protein 6 for recombinant human TGF-β1 protein.
FIG. 24 Neutralization effects of TGF-β1 by fusion protein 6 on Mv-1-lu cells.
FIG. 25 Neutralization effects of TGF-β1 by fusion protein 6 assayed by the reporter gene system.
FIG. 26 Inhibition of proliferation of MDA-MB-468 cells by fusion protein 6.
FIG. 27 Fusion protein 6-mediated effects of ADCC.
FIG. 28 Effect of fusion protein 6 on the body weight of non-small cell lung cancer NCI-H1975 cell subcutaneously xenografted mice.
FIG. 29 Effect of fusion protein 6 on tumor volume in non-small cell lung cancer NCI-H1975 cell subcutaneously xenografted mice.
FIG. 30 Ultrafiltration stability assay of fusion protein 6.
FIG. 31 Assay of degradation of X/TGFβR2 fusion protein.
FIG. 32 Assay of degradation propensity of X/TGFβR2 fusion protein.
FIG. 33 Assay of the binding capacity of X/TGFβR2 fusion protein to TGF-β1.
FIG. 34 Assay of the ability of X/TGFβR2 fusion protein to neutralize TGF-β1 and TGF-β3.
FIG. 35 Assay of X/TGFβR2 fusion protein binding to X-portion target.

### DETAILED DESCRIPTION

### Definition

Unless otherwise stated, all technical and scientific terms used herein have the meaning commonly understood by those of skilled persons in the art to which the present invention belongs. For the purposes of the present invention, the following terms are further defined.

When used herein and in the appended claims, the singular forms "one", "a", "another", and "said" include the plural designation of the object unless the context clearly indicates otherwise.

The term "truncated" form of a protein molecule means in which one or more amino acid residues are missing in a modified manner compared to the natural form of the protein molecule.

The term "fusion protein" refers to a protein molecule that combines two or more proteins. It is usually obtained by expressing a hybrid gene that combines the sequences of two or more genes, which are inserted into the expression vector in a form that conforms to the expression frame.

The term "antibody" refers to an immunoglobulin molecule and refers to any form of antibody that exhibits the desired biological activity. It includes, but is not limited to, monoclonal antibodies (including full-length monoclonal antibodies), polyclonal antibodies and multi-specific antibodies (e.g., bispecific antibodies), and even antibody fragments.

The term "variable region" refers to the domain in the heavy or light chain of an antibody that is involved in antibody binding to antigen. The variable regions of the heavy and light chains of natural antibodies (VH and VL, respectively) generally have a similar structure and can be further subdivided into highly variable regions (called complementary decision regions (CDR)) interspersed with more conserved regions (called framework regions (FR)).

The complementarity determining region (CDR) and the framework region (FR) of a given antibody can be identified using the Kabat system (Kabat et al: Sequences of Proteins of Immunological Interest, 5th edition, U.S. Department of Health and Human Services, PHS, NIH, NIH Publication No. 91-3242, 1991 ).

The term "constant region" refers to such amino acid sequences in the light and heavy chains of an antibody that is not directly involved in antibody-antigen binding but exhibit a variety of effector functions, such as antibody-dependent cytotoxicity.

An "antigen-binding fragment of an antibody" comprises a portion of an intact antibody molecule, retains at least some of the binding specificity of the parent antibody, and typically includes at least a portion of the antigen-binding region or variable region (e.g., one or more CDRs) of the parent antibody. Examples of antigen-binding fragments include, but are not limited to, Fv, Fab, Fab', Fab'-SH, F(ab')2, Fd fragments, Fd' fragments, single-chain antibody molecules (e.g., scFv, di-scFv or tri-scFv, bipartite antibodies or scFab), and single-domain antibodies.

The term "conjugate" refers to a biologically active protein or peptide molecule that forms a covalent or non-covalent linkage with another molecule, either a small molecule or a biomolecule.

The term "monoclonal antibody" refers to an antibody derived from a substantially homogeneous population of antibodies, i.e., said population comprising homogeneous antibodies identical to each other except for possible mutations (e.g., natural mutations) that may be present in very small amounts. Thus, the term "monoclonal" indicates the nature of said antibodies, i.e., not a mixture of unrelated antibodies. In contrast to polyclonal antibody preparations, which usually include different antibodies against different decision clusters (epitopes), each monoclonal antibody of a monoclonal antibody preparation is directed against a separate decision cluster on the antigen. In addition to their specificity, monoclonal antibody preparations have the advantage that they are usually not contaminated with other antibodies. The term "monoclonal" should not be construed as requiring any particular method of producing said antibody. The term monoclonal antibody specifically includes chimeric antibodies, humanized antibodies, and human antibodies.

The antibody "specifically binds" to a target antigen such as a tumor-associated antigen protein (herein, EGFR), i.e., binding said antigen with sufficient affinity to allow said antibody to be used as a therapeutic agent, targeting a tissue or cell expressing said antigen, and without significantly cross-reactivity with other proteins or with proteins other than the homologs and variants (e.g., mutant forms, splice variants, or protein hydrolysis truncated forms)of the antigen targets mentioned above.

The term "binding affinity" refers to the strength of the sum of non-covalent interactions between a molecule's individual binding sites and its binding partners. Unless otherwise specified, "binding affinity" as used herein refers to the intrinsic binding affinity, which reflects a 1:1 interaction between members of a binding pair (e.g., antibody and antigen). "K_{D} ", "binding rate constant kon" and "dissociation rate constant koff" are commonly used to describe the affinity between a molecule (e.g., antibody) and its binding partner (e.g., antigen) affinity, i.e., how tightly a ligand binds a particular protein. The binding affinity is influenced by non-covalent intermolecular interactions such as hydrogen bonding, electrostatic interactions, and hydrophobic and van der Waals forces between two molecules. In addition, the binding affinity between a ligand and its target molecule may be influenced by the presence of other molecules. Affinity can be analyzed by conventional methods known in the art, including the ELISA described herein.

The term "targeting portion" refers to the portion of a fusion protein that has the function of specifically binding to a target cell. The term includes antibodies and other natural (e.g., receptors, ligands) or synthetic (e.g., DARPin) molecules that specifically bind to target cells. As used herein, "specifically binds target cells" indicates that said portion preferentially binds target cells within a complex mixture.

An "isolated" biomolecule is a biomolecule that has been identified and isolated from a cell that naturally expresses the molecule. Isolated biomolecules include in situ biomolecules in recombinant cells as well as biomolecules that are typically prepared by at least one purification step.

The term "receptor" is a biochemical concept that refers to a class of molecules that **specifically recognize and bind** extracellular signals (i.e., the term "ligand") and produce a specific effect within the cell. The effects produced may last only for a short period of time, such as altering cell metabolism or cell motility. It may also be a long-lasting effect, such as up- or down-regulating the expression of a gene or genes.

"Antibody-dependent cell-mediated cytotoxicity" or "ADCC" refers to a form of cytotoxicity wherein secreted Ig binding to Fcγ receptors present on certain cytotoxic cells (e.g., NK cells, neutrophils, and macrophages) enables these cytotoxic effector cells to specifically bind to antigen-bearing target cells and subsequently kill said target cells using, for example, a cytotoxic agent. To assess the ADCC activity of the target antibody, an in vitro ADCC assay can be performed, such as the in vitro ADCC assay documented in U.S. Patent No. 5,500,362 or 5,821,337 or U.S. Patent No. 6,737,056 (Presta), the methods documented in embodiments of this application. Useful effector cells for such assays include PBMCs and NK cells.

### A truncated TGFβR2 extracellular domain molecule of the present invention, fusion proteins comprising the truncated TGFβR2 extracellular domain molecule, and the targeting portiontargeting portion

Multiple susceptibility sites exist between the N-terminal sites 7-15 of the full-length TGFβR2 extracellular domain. In order to improve the structural stability of the fusion protein, the inventors modified the N-terminal amino acid sequence of the extracellular domain of TGFβR2 by deletion of amino acids in deferent numbers. Fifteen truncated TGFβR2 extracellular domain molecules were obtained.

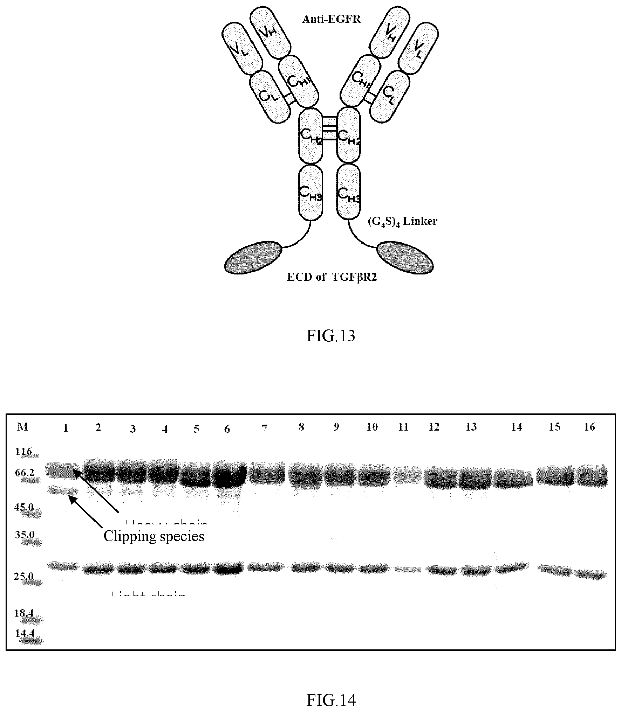

The inventors used their newly isolated EGFR antibodies EGFR-HPA8, Trastuzumab, Bevacizumab, Ramucirumab, Ipilimumab, or Panitumumab as the targeting portion of the fusion protein and the truncated TGFβR2 protein extracellular domain molecule as the immunomodulatory portion of the fusion protein. The truncated TGFβR2 protein extracellular domain molecule is linked to the C-terminal of the heavy chain of EGFR antibody by homologous recombination method, the fusion protein (EGFR/TGFβR2) comprising two chains, i.e. the light chain and heavy chain of the EGFR antibody and TGFβR2 extracellular domain , is formed, the structure of which is shown in FIG. 13. In EGFR antibody/TGFβR2 fusion protein , the amino acid of the heavy chain C-terminal of the EGFR antibody links to the extracellular domain of TGFβR2 of different amino acid deletion forms by (G₄S) ₄ Linker (SEQ ID NO:66). In addition, the lysine residue of the heavy chain C-terminal of the EGFR antibody is removed to reduce the risk of proteolysis.

In a preferred embodiment of the present invention, fusion protein 6 retains a high binding affinity for human EGFR protein, having a K_{D} value of 8.77 pM for binding affinity for human EGFR protein, a binding constant kon value of 1.68E+06 M⁻¹ s⁻¹, and a dissociation constant kdis of 1.47E-05 s⁻¹. In addition, fusion protein 6 which has the truncated TGFβR2 has a similar affinity for human TGF-β1 protein as fusion protein 1, which has full-length TGFβR2, with a k_{D} value of 96.1 pM, a binding constant kon value of 1.53E+06 M⁻¹ s⁻¹, and a dissociation constant kdis of 1.47E-04 s .⁻¹

### Nucleic acids of the present invention

The invention also relates to nucleic acids encoding the truncated TGFβR2 extracellular domain molecule of the present invention, molecules including fusion proteins comprising the truncated molecule and the targeting portiontargeting portion, or portions thereof. Some example sequences of these nucleic acid molecules are shown in the sequential list.

The nucleic acid molecules of the present invention are not limited to the sequences disclosed herein, but also include variants and other corresponding nucleic acid forms thereof, such as mRNA, cDNA, and variants thereof. The variants of the present invention may be described with reference to their physical properties in hybridization. Those of skill in the art will recognize that using nucleic acid hybridization techniques, nucleic acids can be used to identify their complements as well as their equivalents or congeners.

### Recombinant vectors and expression

The present invention also provides recombinant constructs comprising one or more nucleotide sequence(s) of the present invention. The recombinant constructs of the present invention may be used with vectors, for example, plasmid, phagemid, phage, or viral vectors, and nucleic acid molecules encoding antibodies of the present invention are inserted into said vectors.

The present truncated TGFβR2 extracellular domain molecule, targeting portiontargeting portions, fusion proteins comprising the truncated molecule and the targeting portiontargeting portion can be prepared by the recombinant expression of a nucleotide sequence encoding said molecule or protein in a host cell. Said molecule or protein may contain more than one amino acid sequence, and in order to express the plurality of amino acid sequences by recombinant methods, one or more recombinant expression vectors carrying the encoding nucleotide sequence may be transfected with the host cell so that said plurality of amino acid sequences is expressed in the said host cell. Standard recombinant DNA methodologies are used to prepare and/or obtain nucleic acids encoding said molecules or proteins, incorporate these nucleic acids into recombinant expression vectors and introduce said vectors into host cells, e.g. Sambrook, Fritsch, and Maniatis (eds.), Molecular Cloning; A Laboratory Manual, Second Edition, Cold Spring Harbor, N.Y., (1989), Ausubel, F. M. et al. (eds.) Current Protocols in Molecular Biology, Greene Publishing Associates, (1989) and those documented in U.S. Patent No. 4,816,397 by Boss et al.

Examples of prokaryotic host cells are bacteria and examples of eukaryotic host cells are yeast, insect, or mammalian cells. It should be understood that the design of an expression vector including the selection of regulatory sequences is influenced by a variety of factors, such as the selection of the host cell, the level of expression of the desired protein, and whether the expression is constitutive or inducible.

The truncated TGFβR2 extracellular domain molecules, targeting portion targeting portion and fusion proteins of the present invention can be recovered and purified from recombinant cell cultures by well-known methods, said well-known methods including, but not limited to, ammonium sulfate or ethanol precipitation, acid extraction, protein A affinity chromatography, protein G affinity chromatography, anion or cation exchange chromatography, cellulose phosphate chromatography, hydrophobic interaction chromatography, affinity chromatography, hydroxyapatite chromatography, and lectin chromatography. High-performance liquid chromatography ("HPLC") can also be used for purification.

### Use

The truncated TGFβR2 extracellular domain molecules, antibodies and fusion proteins of the present invention can be used to treat cancers, such as gastric cancer.

### Pharmaceutical compositions

One or more kinds of the truncated molecule(s), fusion proteins, antibodies, and antigen-binding fragments, truncated molecule-medicament conjugates, fusion protein-medicament conjugates, conjugates, nucleic acids, and carriers of the present invention may be prepared with at least one other chemical agent to form a pharmaceutical composition comprising the preceding active ingredient(s) described above and one or more medicament-acceptable carriers, diluents, or excipients; optionally, also may also comprise one or more other therapeutic agents.

### Reagent Kits

The present invention also relates to pharmaceutical packaging and kits comprising one or more containers, said containers containing the pharmaceutical compositions of the present invention as mentioned above. Associated with such containers may be tipped in the form prescribed by the government agency governing the manufacture, use, or distribution of the medicament or biological product, which reflects approval for human administration by the agency in which said product is manufactured, used, or distributed.

### Preparation and Storage

The pharmaceutical compositions of the present invention can be prepared in a manner known in the art, for example by conventional methods of mixing, dissolving, granulating, grinding, emulsifying, encapsulating, encapsulating, or lyophilizing.

After pharmaceutical compositions comprising compounds of the present invention formulated in an acceptable carrier have been prepared, they can be placed in appropriate containers and labeled for the treatment of the indicated condition. Such labeling would include the amount, frequency, and method of administration of the medicament.

### Medicament combinations

The combinations comprising the antibodies of the present invention described above are also combined with one or more other therapeutic agents (s), wherein the resulting combination does not cause unacceptable adverse effects.

The following embodiments are used to illustrate the invention exemplarily and are not intended to limit the invention.

### Example

### Example 1: Screening for murine antibodies that block the binding of EGF to EGFR using phage antibody display library

### 1.1 Mouse immunization and phage antibody library screening

Mice were immunized with the nucleic acid plasmids pCMV3-mFlt3L (G12FE5S01-D, constructed by Sinocelltech Limited, SEQ ID NO:142) and pCMV3-mCSF2 (G12FE5S02-D, constructed by Sinocelltech Limited, SEQ ID NO: 143). The specific method is as follows: mice were immunized with cardiotoxin 10 M intramuscularly two days before the first immunization. 20 g/leg of pCMV3-mFlt3L and pCMV3-mCSF2 mixture with a mass ratio of 1:1 was injected intramuscularly and 30 g/leg of pGS6-EGFR-TT-WPRE (Constructed by Sinocelltech Limited, SEQ ID NO:144) was injected intramuscularly three days later for each immunization, intraperitoneal injection of 5×10⁶ insect cells overexpressing EGFR for fourth and fifth immunization, with immunization intervals of 2 weeks, 2 weeks, 2 weeks, and 2 weeks, in sequence. From the third immunization onwards, blood was collected via the medial canthal plexus of the eye seven days after each immunization. The recombinant human EGFR protein (source: Sino Biological, Inc., Cat. 10001-H08B, hereinafter the same) was coated to detect the serum titre of anti-EGFR in mice. The fifth immzation serum achieved a criteria that immune serum titers reaching 8000-fold and serum titre OD>1, then an intraperitoneal booster injection using 5×10⁶ MDA-MB468 cells was performed at an interval of 20 days, and 7 days later, the mice were executed and the spleen tissues were frozen in liquid nitrogen.

Mouse spleen tissue was extracted with TriPure Isolation Reagent (source: Roche Cat. No. 11 667 165 001) for RNA extraction, and cDNA was obtained by reverse transcription with the reverse transcription kit TriPure Isolation Reagent (source: Invitrogen Cat. No. 18080-051), PCR amplification was performed to obtain the nucleotide sequences encoding the light and heavy chain variable regions of mouse antibodies, the nucleotide sequences encoding the light and heavy chain variable regions of mouse antibodies were spliced into the nucleotide sequences encoding scFv by overlap extension splicing PCR, light and heavy chain variable regions were linked by linker ^{[25]}:
TCTAGTGGTGGCGGTGGTTCGGGCGGTGGTGGAGGTGGTAGTTCTAGATCTTCC (SEQ ID NO:2),
then were ligated into the phage vector pComb3x (source: Sino Biological, Inc.)by restriction endonuclease Sfi I (source: Fermentas), and the phage display scFv antibody library for immunized mice was constructed by electrotransforming X-Blue competent cell. The recombinant human EGFR protein was coated on ELISA plates, and an anti-EGFR positive antibodies enriched phage librarywere obtained by phage antibody panning process. Subsequently, the above library was mixed with MDA-MB-468 cells (source: Cell Resource Center, Shanghai Institutes for Biological Sciences, Chinese Academy of Sciences), and phage libraries enriched for positive antibodies against MDA-MB-468 cells were screened from the above libraries according to the phage antibody panning process (O'Brien, P. M., & Aitken, R. (Eds.) , Springer Science & Business Media. 2002; ISBN: 9780896037113). Colonies of phage were selected from the enriched library, expressed, and the binding to recombinant human EGFR protein was detected by ELISA, and a high binding antibody EGFR-mhPA8 scFv antibody clone with specific binding to recombinant human EGFR was screened and was sent to a sequencing company for sequencing to obtain the nucleotide sequence of EGFR-mhPA8 scFv antibody (SEQ ID NO:3).

### 1.2 Functional assay of murine antibodies targeting EGFR

### 1.2.1 Murine antibody binding and ligand competition functions

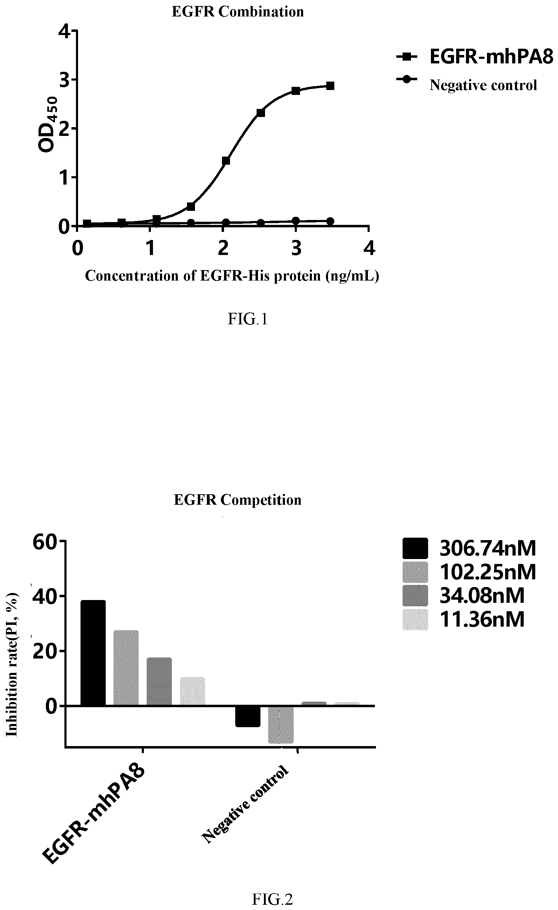

ELISA was used to detect the binding ability of the murine antibody to recombinant human EGFR protein. Recombinant human EGFR-His protein (source: Sino Biological, Inc., hereafter the same) at different concentrations (1.37 ng/mL, 4.12 ng/mL, 12.35 ng/mL, 37.04 ng/mL, 111.11 ng/mL, 333.33 ng/mL, 1000 ng/mL and 3000 ng/mL) were coated in 96-well plates, 100 L per well, were coated overnight at 4°C. The plates were washed the next day and blocked at room temperature for 1 h. EGFR-mhPA8 and the negative control antibody H7N9-R1 (source: Sinocelltech Limited, same below) were added at 13.89 nM and incubated for 1 h, after which the plates were washed to remove the unbound antibody. The secondary antibody Goat anti-hIgG F(ab)2/HRP (source: Jackson ImmunoResearch, Cat. 109-036-006, same as below) was added and incubated for 1 h, the plates were repeatedly washed and the substrate chromogenic solution was added for color development, and the OD₄₅₀ is read by a microplate reader after termination. Using the recombinant human EGFR-His protein concentration as the horizontal coordinate and the OD₄₅₀ reading as the vertical coordinate, the S-curve was fitted and the EC₅₀ of antibody binding to recombinant human EGFR-His protein was analyzed using GraphPad Prism 6.0 software. The results are shown in FIG. 1. The human-mouse chimeric antibody EGFR-mhPA8 can effectively bind recombinant human EGFR-His with an EC₅₀ of 128.7 ng/mL and R² = 1.000. Negative control H7N9-R1 did not bind to recombinant human EGFR-His protein.

This example further analyzed the ability of EGFR-mhPA8 to block EGFR ligand-receptor binding by FACS. 3 × 10⁵ MDA-MB-468 cells were added with 10 µL of the final concentration of 217.1 nM biotin-labeled EGF-Fc protein (source: Sino Biological, Inc.). EGFR-mhPA8 antibodies at final concentrations of 306.74 nM, 102.25 nM, 34.08 nM, and 11.36 nM were added after 30 min incubation at 2-8°C, and H7N9-R1 was used as the negative control antibody. Mix and incubate at 2-8°C for 20 min and then wash with PBS and centrifuge to remove unbound antibodies and ligands. Add Streptavidin-488-FITC secondary antibody (source: Sino Biological, Inc., same below) and incubate for 1 h at 2-8°C. Repeat washing and centrifugation to remove unconjugated secondary antibodies. Finally, 200 µL PBS was added to resuspend the cells and filtered through a 400 mesh filter into a flow tube for flow-through detection. The results are shown in FIG. 2, EGFR-mhPA8 can effectively block the binding of EGF-Fc protein to EGFR on MDA-MB-468 cells in a concentration gradient.

### 1.2.2 Inhibition of proliferation of MDA-MB-468 cells by murine antibodies

Breast cancer MDA-MB-468 cells highly express EGFR and also autocrine various ligand factors of EGFR. In this example, the growth inhibitory function of murine antibody on MDA-MB-468 cells was determined by WST-8 assay.

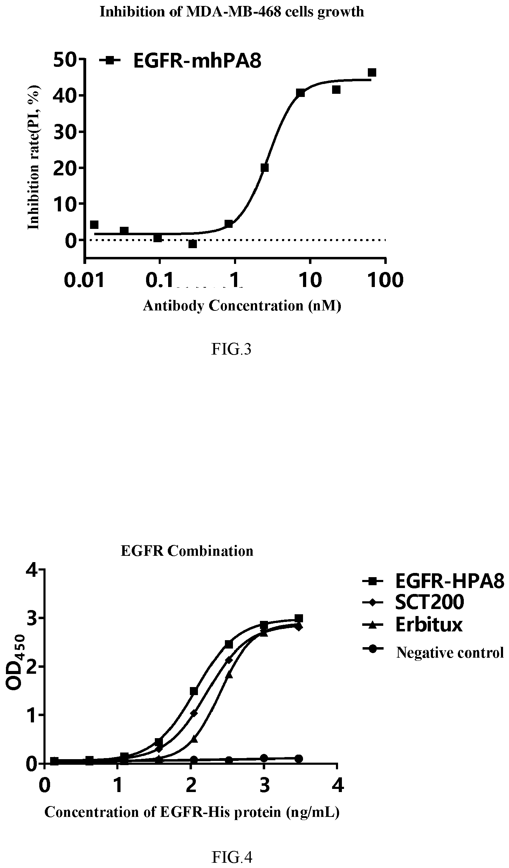

MDA-MB-468 cells were uniformly inoculated in 96-well plates at 5×10³ /well. Cells were incubated in CO₂ incubator for 3 h, and different concentrations of murine antibody EGFR-mhPA8 (66.7 nM, 22.2 nM, 7.4 nM, 2.5 nM, 0.82 nM, 0.27 nM, 0.093 nM, 0.033 nM and 0.013 nM) were added. A negative control M group (containing cells) and a blank control B group (medium only, no cells) were also used. Cells were incubated in CO₂ incubator at 37°C and 5% CO₂ for 5 days and then WST-8 was added at 15 µL/well. After 240 min of incubation, OD₄₅₀ - OD₆₃₀ was measured by a microplate reader, and the inhibition rate of mouse-derived antibody was calculated by subtracting the reading value of detection blank well B. The inhibition rate = (OD of group M - OD of the sample)/(OD of group M) × 100%, and the quantitative efficacy curve was analyzed and plotted using GraphPad Prism software, the horizontal coordinate is the antibody concentration and the vertical coordinate is the inhibition rate. As shown in FIG. 3, EGFR-mhPA8 effectively inhibited the proliferation of MDA-MB-468 and the inhibition rate increased with increasing medicament concentration in an "S" curve. EGFR-mhPA8 inhibited MDA-MB-468 cells with EC₅₀ at 2.78 nM and R² = 0.991.

### Example 2: Humanization of murine antibody EGFR-mhPA8 and ex vivo characterization of humanized antibody

### 2.1 Humanization and production of murine antibody mhPA8

The nucleotide sequence of the EGFR-mhPA8 antibody was deduced to obtain the heavy chain (SEQ ID NO:8) and light chain variable region amino acid sequence (SEQ ID NO:9) of the EGFR-mhPA8 scFv antibody. The amino acid sequences of three CDRs each for the EGFR-mhPA8 scFv light and heavy chains were determined by reference to Kabat [26] and the IMGT numbering approach, see SEQ ID NO: 10-15. According to Kabat numbering, except for the N mutation to D at position 52 in LCDR2, respective three CDRs of the above-mentioned light and heavy chains were transplanted in the subsequent humanization step and retained in the final humanized antibody EGFR-HPA8 scFv.

**Table 1 EGFR-mhPA8 light chain and heavy chain CDR sequences**

| Name | Sequence | SEQ ID |
|---|---|---|
| LCDR1 | RASENIYYSLA | SEQ ID NO:10 |
| LCDR2 | ITNGLAD | SEQ ID NO:11 |
| LCDR3 | KQSYDVPLT | SEQ ID NO:12 |
| HCDR1 | GYTFTTYYTH | SEQ ID NO:13 |
| HCDR2 | WIYPGDVNTKYNEKFKG | SEQ ID NO:14 |
| HCDR3 | AREDPGSNYFDY | SEQ ID NO:15 |

Humanization of murine antibodies was performed using the classical CDR graft method ^{[27, 28]}. The antibodies being of more than 50% amino acid sequence similarity to both the variable regions of light chain and heavy chain of murine antibody, and more than 50% amino acid sequence identity to the framework regions of the variable regions of the light chain and heavy chain of the murine antibodies to be modified respectively were selected as the humanization template library. The human antibody with the highest spatial similarity to the variable region of the antibody to be modified was selected as the humanization template. The three CDR sequences of the light or heavy chains of the murine antibody were substituted with the corresponding CDR amino acid sequences in the humanization template, and the amino acids NG, NS, NA, and NT, which are at high risk of deamidation in the mutated sequences, were subjected to mutation in order to improve the chemical stability and maintain the biological function of the antibody. The affinity of the humanized antibodies is assayed by ELISA and the humanized antibodies that maintain affinity are selected. The humanization template used for the light chain variable region grafted of EGFR-mhPA8 in this example is IGKV1-NL1^{∗}01, which has 68.4% homology with the light chain of EGFR-mhPA8, and the humanization template for the heavy chain variable region is IGHV1-69-2^{∗}01, which has 64.9% homology with the heavy chain of EGFR-mhPA8. The N52 of humanized antibody EGFR-HPA8 variable region LCDR2 prone to deamidation is mutated to D.

Since the key site of the murine framework region is essential for maintaining the stability of the CDR spatial structure, the key site should be reverse mutated to the corresponding amino acid of the murine antibody. According to the Kabat numbering, the light chain was reverse mutated to Q at position 45, I at position 48, K at position 74, and D at position 76, and the heavy chain was reverted mutated to K at position 38, I at position 48 and L at position 70. The humanized antibody EGFR-HPA8 was obtained by CDR humanization grafting and frame region reversion mutation, and its heavy chain and light chain amino acid sequences are shown in SEQ ID NO:22/23, respectively; its heavy chain and light chain amino acid sequences containing the signal peptide are shown in SEQ ID NO:24/25, respectively, containing the amino acid sequences of sequentially linked heavy chain/light chain signal peptide (SEQ ID NO:26/27), the variable region of the heavy chain/light chain of its humanized antibody (SEQ ID NO:28/29) and the constant region of its humanized antibody, that is, IgG1 heavy chain constant region/human kappa light chain constant region (SEQ ID NO:30/31); and the sequence of its humanized CDR, as detailed in Table 2.

**Table 2 EGFR-HPA8 light chain and heavy chain CDR sequences**

| Name | Sequence | SEQ ID |
|---|---|---|
| LCDR1 | RASENIYYSLA | SEQ ID NO:16 |
| LCDR2 | ITDGLAD | SEQ ID NO:17 |
| LCDR3 | KQSYDVPLT | SEQ ID NO:18 |
| HCDR1 | GYTFTTYYTH | SEQ ID NO:19 |
| HCDR2 | WIYPGDVNTKYNEKFKG | SEQ ID NO:20 |
| HCDR3 | AREDPGSNYFDY | SEQ ID NO:21 |

The the signal peptide containing EGFR-HPA8 antibody light chain nucleotide sequence (SEQ ID NO: 33) containing sequentially linked light chain signal peptide nucleotide sequences (SEQ ID NO: 35), humanized antibody light chain variable region nucleotide sequences (SEQ ID NO: 37) and human kappa light chain constant region nucleotide sequences (SEQ ID NO: 39) was amplified by splicing PCR. The above PCR products were inserted into pSTEP2 vector (source: constructed by Sinocelltech Limited, same below) by In-fusion method (double digested by *Hind III*+*Xba* I), and the correct plasmids were verified by sequencing. The nucleotide sequence of the heavy chain variable region of EGFR-HPA8 antibody (SEQ ID NO:36) was obtained by whole gene synthesis and inserted into the pSTEP2 vector (after *Sca* I + *Nhe* I double digestion)containing the nucleotide sequence of the heavy chain signal peptide (SEQ ID NO:34) and the nucleotide sequence of the heavy chain constant region of human IgG1 (SEQ ID NO:38) by In-fusion method, and the correct EGFR-HPA8 light-heavy chain expression vector was verified by sequencing. After plasmid extraction and transfection of HEK-293 cells (*fut8* knockout), the cells were cultured and expressed for 7 days, the cell supernatant was purified using affinity chromatography after centrifugation, and the chromatography medium was Protein A packing that interacts with Fc. After equilibrating the Protein A chromatography column with 50 mM Tris, 10 mM NaCl, and pH 8.0 buffer for 5-10 times the column volume, the filtered culture supernatant was added to the chromatography column for binding, and the column was drenched with 20 mM Tris, 0.3 M Arg, pH 6.5 buffer for 5-10 times the column volume, and then the column was rinsed with 0.1 M Gly, 10 mM NaCl, pH 3.5 elution buffer, and the collected samples were neutralized with 2 M Tris (pH 8.0) to obtain high-purity and high-quality ADCC-enhanced EGFR-HPA8 antibody.

Primer sequences for splicing PCR amplification of EGFR-HPA8 antibody light chain containing the signal peptide

| | |
|---|---|
| F1 (SEQ ID NO.167) | GTCACCGTCCTGACACGAAGCTTGCCGCCACC |
| R1 (SEQ ID NO.168) | ACTATAGAATAGGGCCCTCTAGA |
| F2 (SEQ ID NO.169) | GGCAAGGCTCCAAAGCTGCTGATTTAC |
| R2 (SEQ ID NO.170) | GTAAATCAGCAGCTTTGGAGCCTTGCC |
| F3 (SEQ ID NO.171) | ACCTACTACTGTATGCAGTCCTATGAT |
| R3 (SEQ ID NO.172) | ATCATAGGACTGCATACAGTAGTAGGT |

Primer sequences for whole gene synthesis of EGFR-HPA8 antibody heavy chain variable region

| | |
|---|---|
| F4 (SEQ ID NO.173) | GCTACCAGGGTGCTGAGTGAGGTCCAACTTGTCCAGTCTGGAGCAG AGGTG |
| R4 (SEQ ID NO.174) | CACTGTGGCTCCAGGCTTCTTCACCTCTGCTCC |
| F5 (SEQ ID NO.175) | CCTGGAGCCACAGTGAAGATTTCCTGTAAGGTG |
| R5 (SEQ ID NO.176) | GGTGGTGAAGGTGTAGCCAGACACCTTACAGGA |
| F6 (SEQ ID NO.177) | TACACCTTCACCACCTACTACACCCACTGGG |
| R6 (SEQ ID NO.178) | CTTGCCAGGAGCCTGCTTCACCCAGTGGGTGT |
| F7 (SEQ ID NO.179) | CAGGCTCCTGGCAAGGGATTGGAGTGGATTG |
| R7 (SEQ ID NO.180) | ATCTCCAGGGTAAATCCAGCCAATCCACTCCA |
| F8 (SEQ ID NO.181) | ATTTACCCTGGAGATGTGAACACCAAATACA |
| R8 (SEQ ID NO.182) | CCTGCCCTTGAACTTCTCATTGTATTTGGTGT |
| F9 (SEQ ID NO.183) | AAGTTCAAGGGCAGGGTGACCCTGACAGCAGAC |
| R9 (SEQ ID NO.184) | ATAGGCTGTGTCTGTGCTGGTGTCTGCTGTCAG |
| F10 (SEQ ID NO.185) | ACAGACACAGCCTATATGGAACTGTCCTCCCTG |
| R10 (SEQ ID NO.186) | GACTGCTGTGTCCTCAGACCTCAGGGAGGACAG |
| F11 (SEQ ID NO.187) | GAGGACACAGCAGTCTACTACTGTGCCAGGGAG |
| R11 (SEQ ID NO.188) | AAAGTAGTTGCTGCCAGGGTCCTCCCTGGCACA |
| F12 (SEQ ID NO.189) | GGCAGCAACTACTTTGACTACTGGGGACAAGGC |
| R12 (SEQ ID NO.190) | TGGGCCCTTGGTGCTTGCGCTGGACACTGTCACCAGGGTGCCTTGTC CCCA |

### 2.2 In vitro characterization of humanized EGFR-HPA8 antibody

### 2.2.1 Humanized antibody-specific binding and ligand competition assay

Referring to Example 1.2.1, the binding ability of the human antibody to recombinant human EGFR protein was detected by applying ELISA while setting SCT200 (documented in CN200610012002.8, same below), Erbitux (MERCK, 201621, same below) and negative control. As shown in FIG. 4, the EC₅₀ for humanized EGFR-HPA8 antibody specifically binding to recombinant human EGFR-His is at 116.6 ng/mL, R² = 1.000; EC₅₀ for SCT200 at 166.5 ng/mL, R² = 1.000; EC₅₀ for Erbitux at 253.6 ng/mL, R² = 1.000; negative control H7N9- R1 did not bind. The results indicated that EGFR-HPA8 had a better ability to bind to recombinant human EGFR-his than SCT200 and Erbitux.

Meanwhile, the ability of humanized antibody EGFR-HPA8 to block EGFR ligand-receptor binding was analyzed by FACS with reference to Example 1.2.1, setting SCT200, Erbitux, and negative control. The results are shown in FIG. 5, the ability of EGFR-HPA8 to block the binding of EGF-Fc protein to EGFR on MDA-MB-468 cells is stronger than SCT200 and Erbitux.

### 2.2.2 Inhibition of different tumor cells proliferation by humanized antibodies

### 2.2.2.1 Ability of humanized antibodies to inhibit the proliferation of MDA-MB-468 cells

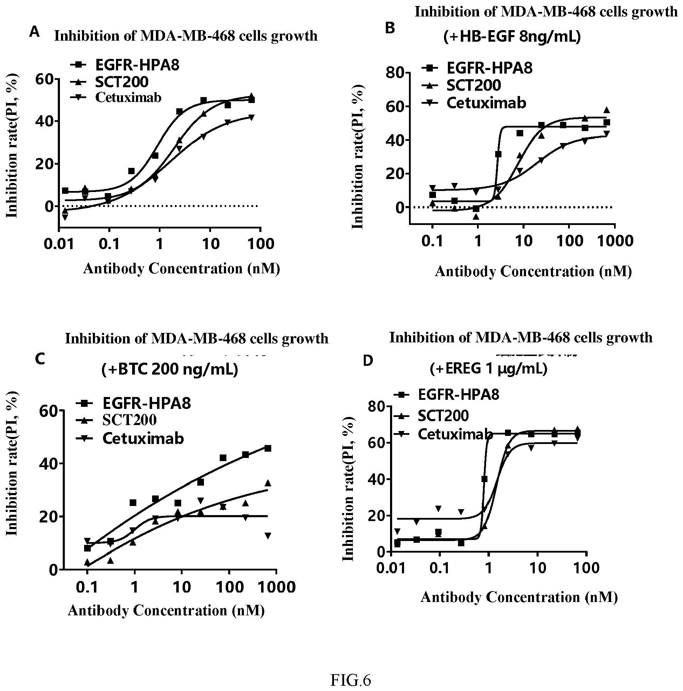

MDA-MB-468 cells were uniformly inoculated in 96-well plates at 5×10³ /well. Cells were incubated in CO₂ incubator for 3 h, and different concentrations of humanized antibody EGFR-HPA8 (666.7 nM, 222.2 nM, 74.1 nM, 24.7 nM, 8.23 nM, 2.74 nM, 0.91 nM, 0.31 nM and 0.1 nM) were added, and SCT200 and Erbitux were used as control. The ligands, HB-EGF (source: Sino Biological, Inc., same below) at a final concentration of 8 ng/mL, BTC-Fc (source: Sino Biological, Inc., same below) at a final concentration of 200 ng/mL or Fc-EREG (source: Sino Biological, Inc., same below) at a final concentration of 1 µg/mL, were then added respectively. Cells were incubated in CO₂ incubator at 37°C and 5% CO₂ for 5 days, then WST-8 was added at 15 µL/well. After 240 mins of incubation, OD₄₅₀ - OD₆₃₀ was measured by a microplate reader, and the growth inhibition rate of cells by the antibody was calculated. The addition of ligand without antibody was used as group M. The inhibition rate = (OD of group M - OD of sample)/(OD of group M) × 100%, and the quantitative efficacy curve was analyzed and plotted using GraphPad Prism software, the horizontal coordinate is the antibody concentration and the vertical coordinate is the inhibition rate. The results are shown in FIG. 6A and Table 3. EGFR-HPA8 showed better growth inhibition of MDA-MB-468 cells than SCT200 and Cetuximab control antibodies under ligand-free conditions. EGFR-HPA8 had a similar maximum inhibition rate as SCT200 but had a lower growth inhibition EC₅₀. The results of Example 2.2.1 show that the ability of EGFR-HPA8 to block the binding of EGF-Fc protein to the EGFR on MDA-MB-468 cells was stronger than SCT200 and Erbitux. In this example different ligands of EGFR were added in the MDA-MB-468 cell growth inhibition assay to further validate the ability of EGFR-HPA8 to block EGFR ligand-receptor binding at the cellular functional level. The results are shown in FIG. 6B-6D and Table 3. EGFR-HPA8 inhibited the proliferation of MDA-MB-468 cells better than SCT200 and Cetuximab under different ligand conditions.

**Table 3 EC50 and maximum neutralization rate of EGFR-HPA8 antibody to inhibit the proliferation of MDA-MB-468 cells**

| Ligand | Antibody | EC₅₀ (nM) | Maximum inhibition rate (%) |
|---|---|---|---|
| None | EGFR-HPA8 | 0.90 | 50.1 |
| | SCT200 | 1.97 | 52.3 |
| | Cetuximab | 1.59 | 44.4 |
| 8 ng/mL HB-EGF | EGFR-HPA8 | 2.64 | 47.8 |
| | SCT200 | 7.52 | 53.4 |
| | Cetuximab | 19.9 | 42.7 |
| 200 ng/mL BTC | EGFR-HPA8 | / | 87.5 |
| | SCT200 | / | 40.9 |
| | Cetuximab | / | 20.2 |
| 1 µg /mL EREG | EGFR-HPA8 | 0.81 | 65.2 |
| | SCT200 | 1.44 | 66.7 |
| | Cetuximab | 1.47 | 59.9 |

### 2.2.2.2 Ability of humanized antibodies to inhibit Fadu cell proliferation

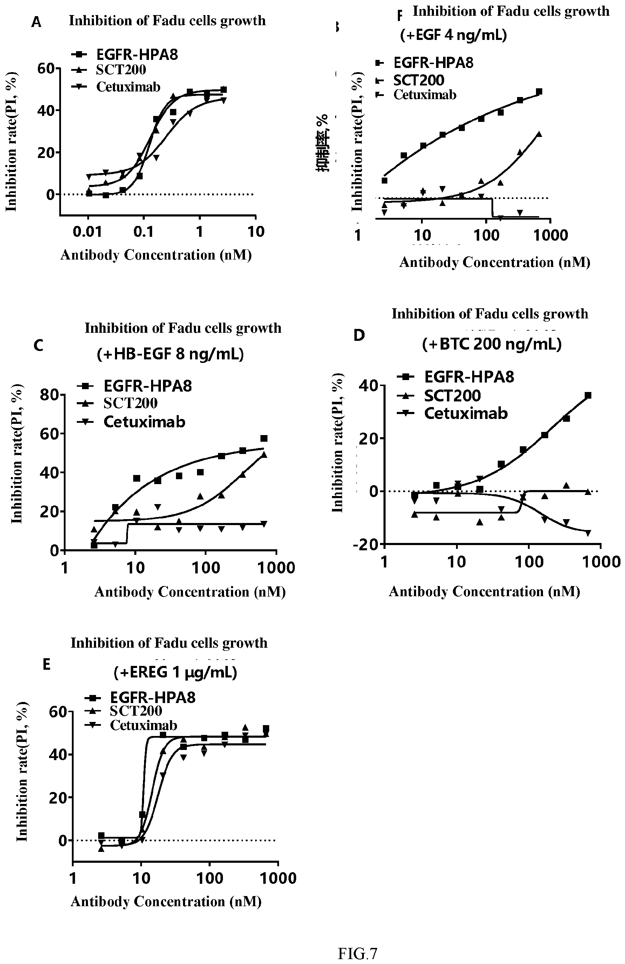

Fadu is a human pharyngeal squamous carcinoma cell strain which highly expresses EGFR and also autocrines various ligand factors of EGFR. Referring to Example 2.2.2.1, inhibition of proliferation of Fadu cells (source: Cell Resource Center, Shanghai Institutes for Biological Sciences, Chinese Academy of Sciences) by EGFR-HPA8 antibody under different ligand conditions was measured by WST-8 assay. The results are shown in FIG. 7 and Table 4. The growth inhibitory ability of EGFR-HPA8 on Fadu cells was similar to that of SCT200 and better than that of Cetuximab under the ligand-free condition, while the growth inhibitory ability of EGFR-HPA8 on Fadu cells was significantly better than that of SCT200 and Cetuximab under different ligand conditions. The results showed that the EGFR-HPA8 antibody had a better ability to inhibit EGFR ligand-receptor binding compared with SCT200 and Cetuximab.

### Table 4 EC50 and maximum neutralization rate of EGFR-HPA8 antibody to inhibit the proliferation of Fadu cells

| Ligand | Antibody | EC₅₀ (nM) | Maximum inhibition rate (%) |
|---|---|---|---|
| None | EGFR-HPA8 | 0.126 | 47.6 |
| | SCT200 | 0.129 | 49.7 |
| | Cetuximab | 0.251 | 46.0 |
| 4 ng/mL EGF | EGFR-HPA8 | / | 74.4 |
| | SCT200 | / | 68.5 |
| | Cetuximab | / | / |
| 8 ng/mL HB-EGF | EGFR-HPA8 | / | 55.8 |
| | SCT200 | / | 65.0 |
| | Cetuximab | / | 13.6 |
| 200 ng/mL BTC | EGFR-HPA8 | / | 49.5 |
| | SCT200 | / | / |
| | Cetuximab | / | / |
| 1 µg /mL EREG | EGFR-HPA8 | 10.9 | 48.4 |
| | SCT200 | 14.5 | 48.3 |
| | Cetuximab | 17.7 | 44.8 |

### 2.2.3 Humanized antibody ADCC effects

This example uses a recombinant CD16a reporter gene system to detect the humanized antibody EGFR-HPA8-mediated ADCC effects. The recombinant CD16a reporter gene system includes effector cells Jurkat-NFAT-Luc2p-CD16A and target cells expressing EGFR. When the two cells are co-cultured and the EGFR antibody is added simultaneously, the Fab fragement of the EGFR antibody binds to EGFR expressed on the surface of the target cells, and its Fc fragement can bind to the effector cells overexpressing Fcγ receptor CD16a thereby activating effector cells Jurkat-NFAT-Luc2p-CD16A and promoting NFAT-RE-mediated bioluminescence.

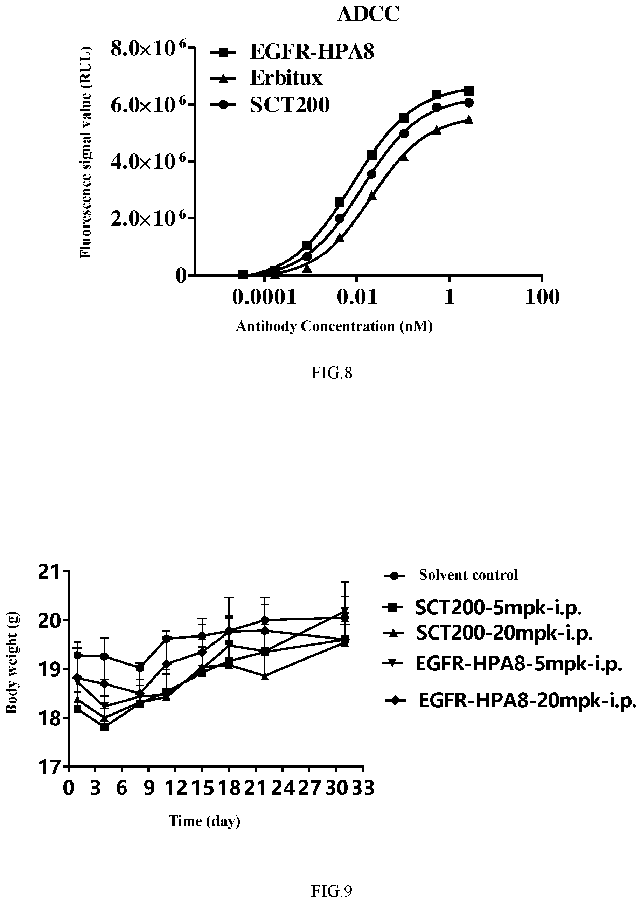

The target cells A431 cells (source: Cell Resource Center, Shanghai Institutes for Biological Sciences, Chinese Academy of Sciences) were uniformly inoculated in 96-well plates at 1×10⁴ /well. After overnight incubation, different concentrations of antibodies (2.67 nM, 0.53 nM, 0.11 nM, 0.021 nM, 0.0043 nM, 0.00085 nM, 0.00017 nM and 0.000034 nM) were added at 40 µL/well, followed by 1×10⁵ effector cells Jurkat-NFAT-Luc2p-CD16A ( Source: Sinocelltech Limited, same as below), 40 µL/well, 3 replicate wells were used for each assay. Target cells, effector cells, and negative antibody control wells were also used. Cells were incubated for 4 h in a CO₂ incubator at 37°C and 5% CO₂, and Passive Lysis 5 × Buffer, 20 µL/well, was added. The cells were freeze-thawed once, and 20 µL of supernatant per well was transferred to a 96-well white-bottom plate after shaking the plate and mixing, and the luminescence detection was performed by LB960-Microplate Luminol Detector. The quantitative efficacy curves were analyzed and plotted using GraphPad Prism software, with the horizontal coordinate being the concentration of the sample and the vertical coordinate being the RLU value. The bioluminescence intensity induction multiplier = sample RLU/negative control RLU. The results are shown in FIG. 8, EGFR-HPA8, positive controls Erbitux and SCT200 can mediate effective ADCC effects on EGFR-expressing A431 tumor cells. Among them, EGFR-HPA8 had an advantage in the half effective concentration of the effect and the induction fold, with an EC₅₀ of 0.008 nM and R² = 0.999 for the induction of ADCC.

### 2.3 Efficacy of EGFR-HPA-8 on human gastric cancer cell line SNU-5 and human non-small cell lung cancer line NCI-H1975 subcutaneously xenografted tumors in mice

Logarithmic growth stage SNU-5 cells (ATCC cell bank) were washed with PBS and digested with 0.25% trypsin, and the digest was collected followed by centrifugation at 800 r/min for 5 min. The cells were resuspended in PBS and the cell concentration was adjusted to 5.0×10⁷ cells/mL (with 50% matrix gel). Balb/c-nude mice (Beijing Viton Lever Laboratory Animal Technology Co.) were subcutaneously inoculated with 5.0×10⁶ SNU-5 cell suspensions on the right dorsum, 100 µL/per mouse. After the tumor volume reached about 170 mm³, the mice were randomly divided into seven groups according to the tumor volume, with five mice in each group. The medicament was administered by intraperitoneal injection (I.P.) on the day of grouping and was given twice a week for 7 consecutive doses. The specific dosing regimen is shown in Table 5 below.

**Table 5 Trial grouping and dosing**

| Group | Number of animals | Subject medicament | Dose(mg/kg) | Administration route | Administration frequency |
|---|---|---|---|---|---|
| 1 | 4 | Solvent control | / | Intraperitoneal injection | BIW |
| 2 | 5 | SCT200 | 5 | Intraperitoneal injection | BIW |
| 3 | 5 | SCT200 | 20 | Intraperitoneal injection | BIW |
| 4 | 5 | EGFR-HPA8 | 5 | Intraperitoneal injection | BIW |
| 5 | 5 | EGFR-HPA8 | 20 | Intraperitoneal injection | BIW |

Note: The administration volume was calculated based on the body weight of mice at 10 mL/kg.

Tumor Growth Inhibition Value (TGI) was calculated as follows: T/C (%) = T_{RTV} /C_{RTV} × 100% (T_{RTV} is RTV of treatment group; C_{RTV} is RTV of the negative control group), relative tumor volume RTV = V_{T} /V₀, V₀ is the tumor volume measured by D₀ ,the grouping and starting dosing day, V_{T} is the tumor volume measured each time. TGI (%) = 1 - T/C (%).

All experimental animals were in good condition and showed some increase in body weight throughout the the course of administration. There was no significant difference in the body weight of mice in each dosing group compared to the solvent control (P > 0.05). The changes in body weights of all animals are shown in FIG. 9 and Table 6.

**Table 6 Effect of EGFR-HPA8 on body weight in SNU-5 human gastric cancer xenografted tumor model mice**

| Group p | Subject medicament | Does (mg/kg) | Number of animals | Body weight (g) | | | Weight change thorough out the course of administration (g) |
|---|---|---|---|---|---|---|---|
| | | | | Before administration ^{a} | after 31 days of administration | P value^{b} | |
| 1 | Solvent control | / | 4 | 19.3±0.5 | 20.1±0.7 | / | 0.78 |
| 2 | SCT200 | 5 | 5 | 18.2±0.7 | 19.6±1.1 | 0.6 | 1.42 |
| 3 | SCT200 | 20 | 5 | 18.4±0.9 | 19.5±0.9 | 0.4 | 1.16 |
| 4 | EGFR-HPA8 | 5 | 5 | 18.7±1.2 | 20.2±1.0 | 0.9 | 1.44 |
| 5 | EGFR-HPA8 | 20 | 5 | 18.8±1.2 | 19.6±0.6 | 0.4 | 0.78 |

| | | | | | | | |
|---|---|---|---|---|---|---|---|
| ^{a}Mean±standard deviation ^{b}Statistical comparison of body weight in the treatment group with that in the solvent control group after 31 days of administration, t-test. | | | | | | | |

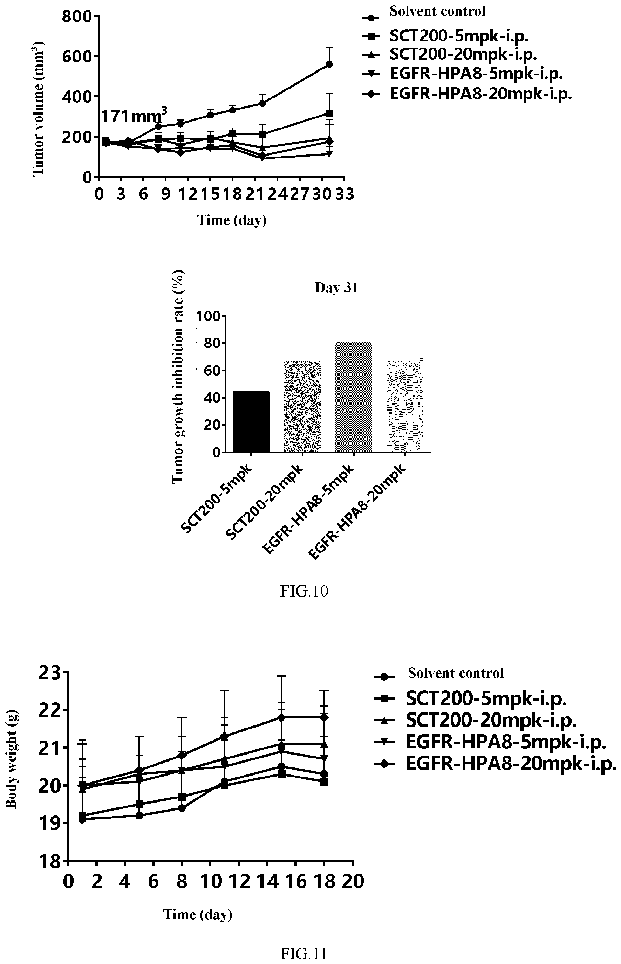

The tumor volume and TGI results for each group in the trial are shown in Table 7 and FIG. 10. After 31 days of group dosing, the mean tumor volume in the Vehicle group was 559.9 ± 144.9 mm³ and in the positive control SCT200 low-dose 5 mpk group was 317.1 ± 197.6 mm³ with a TGI of 44.3%, which was not significantly different from the Vehicle group (*P* = 11.28%). The EGFR-HPA8 low dose 5 mpk group showed better efficacy with a tumor volume of 113.8 ± 74.0 mm³ and TGI of 80.0%, a significant difference compared to the Vehicle group (*P* < 0.05), indicating that EGFR-HPA8 showed slightly better tumor-inhibiting properties than the positive control SCT200 at this dose (*P* = 0.09). The tumor volume in the positive control SCT200 20 mpk group was 191.8 ± 189.1 mm³ with a TGI of 66.5%, which was significantly different from the Vehicle group (*P <* 0.05). The tumor volume in the EGFR-HPA8 high dose 20 mpk group was 175.0 ± 175.0 mm³ with a TGI of 68.9%, which was significantly different from the Vehicle group (P < 0.05). EGFR-HPA8 and positive control SCT200 20 mpk group had a significant tumor-inhibiting effect, and there was no significant difference in tumor volume between the two administration groups (*P* = 0.9). In conclusion, EGFR-HPA8 molecules showed significant antitumor efficacy in SNU-5 human gastric cancer xenografted tumor model at both 5 mpk and 20 mpk dose levels, and the tumor-inhibiting effect was slightly better than that of SCT200 at low doses.

**Table 7 Effect of EGFR-HPA8 on tumor volume in SNU-5 human gastric cancer xenografted tumor model mice**

| Day | Tumor volume of solvent control | SCT200 5mpk | | SCT200 20 mpk | | EGFR-HPA8 5 mpk | | EGFR-HPA8 20 mpk | |
|---|---|---|---|---|---|---|---|---|---|
| | | Tumor volume^{a} | TGI (%) | Tumor volume | TGI (%) | Tumor volume | TGI (%) | Tumor volume | TGI (%) |
| 1 | 172.8±41.8 | 170.7±38.3 | / | 170.3±39.6 | / | 171.0±35.8 | / | 168.5±29.6 | / |
| 4 | 168.7±24.7 | 159.5±52.0 | 4.3 | 166.1±33.8 | 0.1 | 150.3±30.5 | 9.9 | 179.5±38.6 | -9.1 |
| 8 | 249.9±34.0 | 185.1±66.4 | 25 | 186.7±45.0 | 24.2 | 139.7±33.6 | 43.5 | 136.3±47.2 | 44 |
| 11 | 263.8±30.8 | 191.0±61.8 | 26.7 | 160.5±53.4 | 38.3 | 140.8±37.5 | 46.1 | 122.5±69.6 | 52.4 |
| 15 | 307.3±49.2 | 185.4±44.2 | 38.9 | 192.9±68.9 | 36.3 | 140.5±40.3 | 53.8 | 146.9±84.5 | 51 |
| 18 | 330.8±45.5 | 214.4±57.0 | 34.4 | 171.4±106.5 | 47.5 | 140.6±36.3 | 57 | 156.2±88.8 | 51.5 |
| 22 | 365.2±78.5 | 211.3±96.3 | 41.3 | 145.5±101.6 | 59.5 | 90.8±80.0 | 74.8 | 105.4±91.4 | 70.4 |
| 31 | 559.9±144.9 | 317.1±197.6 | 44.3 | 191.8±189.1 | 66.5 | 113.8±74.0 | 80 | 175.0±175.0 | 68.9 |

| | | | | | | | | | |
|---|---|---|---|---|---|---|---|---|---|
| ^{a}Mean±standard deviation | | | | | | | | | |

EGFR-HPA8 also showed significant tumor-inhibiting effects on the tumor of human non-small cell lung cancer cell line NCI-H1975 subcutaneous xenografted mouse model . Balb/c-nude mice were subcutaneously inoculated with NCI-H1975 cells on the right dorsum (Cell Resource Center, Shanghai Institutes for Biological Sciences, Chinese Academy of Sciences), and the Grouping and Dosing are shown in Table 8.

**Table 8 Grouping and Dosing**

| Group | Number of animals | Subject medicament | Dose(mg/kg) | Administration route | the course of administration |
|---|---|---|---|---|---|
| 1 | 4 | Solvent control | / | Intraperitoneal injection | BIW×3 |
| 2 | 5 | SCT200 | 5 | Intraperitoneal injection | BIW×3 |
| 3 | 5 | SCT200 | 20 | Intraperitoneal injection | BIW×3 |
| 4 | 5 | EGFR-HPA8 | 5 | Intraperitoneal injection | BIW×3 |
| 5 | 5 | EGFR-HPA8 | 20 | Intraperitoneal injection | BIW×3 |

| | | | | | |
|---|---|---|---|---|---|
| Note: The administration volume was10 mL/kg the body weight of mice . | | | | | |

All experimental animals were in good condition and showed some increase in body weight thoroughout the course of administration, and there was no significant difference in the body weight of mice in each administration group compared with the solvent control (P > 0.05). The changes in body weight of all animals are shown in FIG. 11 and Table 9.

**Table 9 Effect of EGFR-HPA8 on body weight in NCI-H1975 human lung cancer xenografted tumor model mice**

| Group | Subject medicament | Does (mg/kg) | Number of animals | Body weight (g) | | | Weight change thorough out the course of administration (g) |
|---|---|---|---|---|---|---|---|
| | | | | Before administration | after 31 days of administration^{a} | *P* value^{b} | |
| 1 | Solvent control | / | 6 | 19.1±1.4 | 20.3±1.5 | / | +1.2 |
| 2 | SCT200 | 5 | 6 | 19.2±1.0 | 20.1±1.2 | 0.79 | +0.9 |
| 3 | SCT200 | 20 | 6 | 19.9±0.8 | 21.1±1.0 | 0.28 | +1.2 |
| 4 | EGFR-HPA8 | 5 | 6 | 20.0±1.2 | 20.7±1.2 | 0.58 | +0.7 |
| 5 | EGFR-HPA8 | 20 | 6 | 20.0±1.1 | 21.8±0.7 | 0.05 | +1.8 |

| | | | | | | | |
|---|---|---|---|---|---|---|---|
| ^{a}Mean±standard deviation ^{b}Statistical comparison of body weight in the treatment group with that in the solvent control group after 31 days of administration, t-test. | | | | | | | |

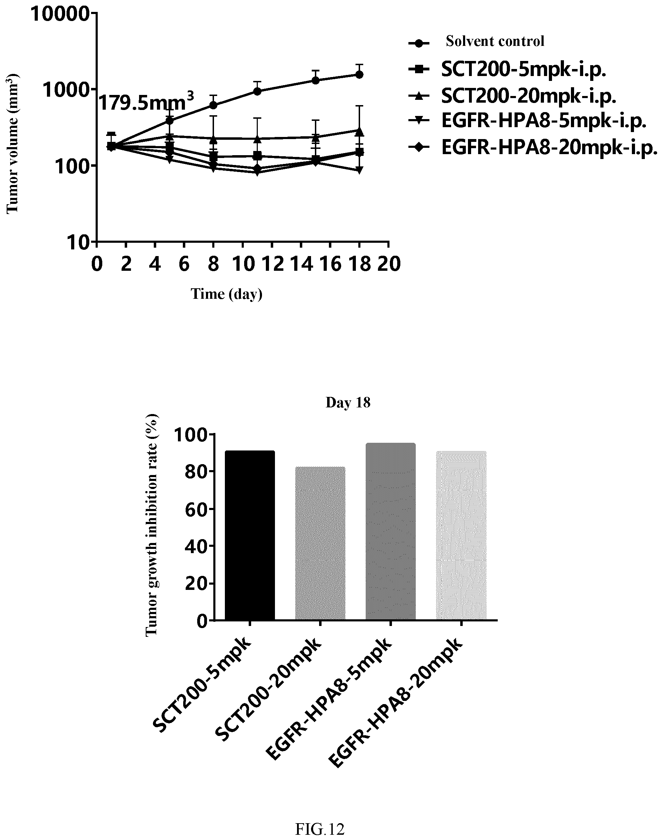

The tumor volume and TGI results for each group in the trial are shown in Table 10 and FIG. 12. After 18 days of grouping and dosing, the positive control SCT200 and EGFR-HPA8 high and low dose groups showed significant tumor-inhibiting effects and statistically significant differences in tumor volume between each administration group and the Vehicle group. The mean tumor volume in the Vehicle group on Day18 was 1564.3 ± 529.0 mm³. The tumor volumes after SCT200 5 mpk and EGFR-HPA8 5 mpk treatment were 151.9 ± 99.1 mm³ and 86.5 ± 107.5 mm³, with TGI of 90.4% and 94.5%, respectively. The tumor volumes after SCT200 20 mpk and EGFR-HPA8 20 mpk treatment were 289.8 ± 321.4 mm³ and 149.3 ± 94.9 mm³, with TGI of 81.8% and 90.4%, respectively. EGFR-HPA8 at both 5 mpk and 20 mpk doses showed a slightly better tumor-inhibiting effect than positive control SCT200, but there was no statistically significant difference. Taken together, these results suggest that the EGFR-HPA8 molecule has significant antitumor efficacy in the NCI-H1975 human non-small cell lung cancer xenografted tumor model.

**Table 10 Effect of EGFR-HPA8 on tumor volume and TGI in NCI-H1975 human lung cancer xenografted tumor model**

| Day | Tumor volume of solvent control | SCT200 5 mpk | | SCT200 20 mpk | | EGFR-HPA8 5 mpk | | EGFR-HPA8 20 mpk | |
|---|---|---|---|---|---|---|---|---|---|
| | | Tumor volume | TGI (%) | Tumor volume | TGI (%) | Tumor volume | TGI (%) | Tumor volume | TGI (%) |
| 1 | 178.5±79.6 | 181.0±90.1 | / | 181.7±86.0 | / | 179.8±76.1 | / | 178.0±70.3 | / |
| 5 | 384.6±156.5 | 173.4±82.2 | 55.5 | 242.0±201.2 | 38.2 | 119.3±75.1 | 69.2 | 150.5±54.1 | 60.8 |
| 8 | 617.6±219.5 | 130.5±65.1 | 79.2 | 226.0±220.8 | 64.1 | 91.8±72.7 | 85.3 | 104.3±46.2 | 83.1 |
| 11 | 937.7±319.8 | 133.1±87.1 | 86 | 223.7±198.0 | 76.6 | 80.9±71.3 | 91.4 | 91.5±34.9 | 90.2 |
| 15 | 1308.8±456.6 | 121.6±74.8 | 90.8 | 234.3±158.3 | 82.4 | 109.4±145.3 | 91.7 | 113.4±56.0 | 91.3 |
| 18 | 1564.3±529.0 | 151.9±99.1 | 90.4 | 289.8±321.4 | 81.8 | 86.5±107.5 | 94.5 | 149.3±94.9 | 90.4 |

| | | | | | | | | | |
|---|---|---|---|---|---|---|---|---|---|
| ^{a}Mean±standard deviation | | | | | | | | | |

### Example 3: Design and test of EGFR antibody/TGFβR2 fusion proteins containing different truncated forms of TGFβR2

### 3.1 Design of EGFR antibody/TGFβR2 fusion protein expression vectors of different truncated forms of TGFβR2 , protein expression and purification

This embodiment uses anti-EGFR antibody as the targeting portion of the fusion protein and TGFβR2 extracellular domain as the immunomodulatory part of the fusion protein. The TGFβR2 extracellular domain is linked to the C-terminal of the heavy chain of the EGFR antibody by homologous recombination, and the EGFR antibody/TGFβR2 extracellular domain fusion protein (EGFR/TGFβR2) is formed by two chains, the light chain, and the heavy chain. The structure of the fusion protein is shown in FIG. 13. The mass spectrometry results showed that there were multiple susceptibility sites between the N-terminal 7-15 sites of the full-length TGFβR2 extracellular domain. To improve the structural stability of the fusion protein, the N-terminal amino acid sequence of the extracellular domain of TGFβR2 was modified with amino acid deletions in different amino acid residue numbers in this example (SEQ ID NO:47-65). The EGFR antibody/TGFβR2 fusion protein links the amino acid of the heavy chain C-terminal of the EGFR antibody to the extracellular domain of TGFβR2 with different amino acid deletion forms via the (G₄S)₄ Linker (SEQ ID NO:66). In addition, the heavy chain C-terminal lysine of the EGFR antibody was removed to reduce the risk of proteolysis. The specific protocol of the EGFR antibody/TGFβR2 fusion protein design are shown in Table 11.

**Table 11 EGFR antibody/TGFβR2 fusion protein protocol**

| Samples | Sequence description of the TGFβR2 extracellular domain | Number of N-terminal amino acid deletions | TGFβR2 extracellular domain SEQ ID | Heavy chain SEQ ID |
|---|---|---|---|---|
| Fusion protein1 | Full length | / | SEQ ID NO:47 | SEQ ID NO:88 |
| Fusion protein2 | ECD delete (6-16) | 11 | SEQ ID NO:48 | SEQ ID NO:89 |
| Fusion protein3 | ECD delete (6-19) | 14 | SEQ ID NO:49 | SEQ ID NO:90 |
| Fusion protein4 | ECD delete (6-21) | 16 | SEQ ID NO:50 | SEQ ID NO:91 |
| Fusion protein5 | ECD delete (6-25) | 20 | SEQ ID NO:51 | SEQ ID NO:92 |
| Fusion protein6 | ECD delete (6-26) | 21 | SEQ ID NO:52 | SEQ ID NO:93 |
| Fusion protein7 | ECD delete (6-27) | 22 | SEQ ID NO:53 | SEQ ID NO:94 |
| Fusion protein8 | ECD delete (7-26) | 20 | SEQ ID NO:54 | SEQ ID NO:95 |
| Fusion protein9 | ECD delete (5-26) | 22 | SEQ ID NO:55 | SEQ ID NO:96 |
| Fusion protein10 | ECD delete (4-26) | 23 | SEQ ID NO:56 | SEQ ID NO:97 |
| Fusion protein11 | ECD delete (3-26) | 24 | SEQ ID NO:57 | SEQ ID NO:98 |
| Fusion protein12 | ECD delete (2-26) | 25 | SEQ ID NO:58 | SEQ ID NO:99 |
| Fusion protein13 | ECD delete (1,6-26) | 22 | SEQ ID NO:59 | SEQ ID NO:100 |
| Fusion protein14 | ECD delete (1-2,6-26) | 23 | SEQ ID NO:60 | SEQ ID NO:101 |
| Fusion protein15 | ECD delete (1-3,6-26) | 24 | SEQ ID NO:61 | SEQ ID NO:102 |
| Fusion protein16 | ECD delete < 1-4,6-26) | 25 | SEQ ID NO:62 | SEQ ID NO:103 |
| Fusion protein17 | ECD delete (1-19) | 19 | SEQ ID NO:63 | SEQ ID NO:104 |
| Fusion protein18 | ECD delete (1-21) | 21 | SEQ ID NO:64 | SEQ ID NO:105 |
| Fusion protein19 | ECD delete (1-26) | 26 | SEQ ID NO:65 | SEQ ID NO:106 |

In the above protocol, the target gene was amplified by PCR or Overlap-PCR, ligated to the expression vector by in-fusion, and the plasmids were extracted separately after sequencing varification, and transiently transferred to HEK-293 cells (*fut8* knockout) and cultured until day 7. Centrifuge and collect the supernatant. The cell supernatant after centrifugation was purified using Protein A affinity chromatography to obtain ADCC-enhanced EGFR antibody/TGFβR2 fusion protein.

### 3.2 Degradation of EGFR antibody/TGFβR2 fusion proteins containing different truncated forms of TGFβR2

The purity as well as the degradation of the expression product was analyzed by reduced SDS-PAGE. The different truncated EGFR antibody/TGFβR2 fusion proteins 1-16 purified in Example 3.1 were subjected to the reduced SDS-PAGE. Specific steps of reduced SDS-PAGE: (1) SDS-PAGE preparation: 3.9% concentrating gel, 13% separating gel; (2) samples were boiled at 100°C for 2 min, centrifuged, and then sampled with 8 µg; (3) constant current of 40 mA, electrophoresis time of 1 h. The results are shown in FIG. 14. The molecular weight of the light chain of EGFR antibody/TGFβR2 fusion protein was about 25 KDa, the molecular weight of the heavy chain was about 66 KDa, and the molecular weight of the clipping species was between 45-66 KDa. The results showed that there were obvious clipping species in the extracellular domain of TGFβR2, while the different truncated forms fusion proteins of the TGFβR2 extracellular domain had substantially fewer bands resulted from the degradation than the full-length forms fusion proteins of the TGFβR2 extracellular domain. Thus, the different truncated forms of the TGFβR2 extracellular domain prepared in the present invention significantly enhanced the stability of the TGF-β receptor-containing antibody fusion protein.

### 3.3 Degradation tendency of EGFR antibody/TGFβR2 fusion proteins containing different truncated forms of TGFβR2

The 293E cell supernatant which accelerates fusion proteins processing is used to further evaluate the stability of different truncated forms of EGFR antibody/TGFβR2 fusion proteins . 293E cell expression system, which is often used to express antibodies, expresses a variety of host cell proteins (HCPs) and proteases required for cell growth. Therefore, the stability of antibodies can be assessed by observing the degradation propensity of fusion proteins in the supernatant of 293E cells.

The purified fusion protein was mixed with the supernatant of 293E cells cultured for 10 days at a volume ratio of 1:0.3, and the final concentration of fusion protein was about 1 mg/mL. The mixed samples were shaken and mixed well and incubated at 37°C for 48 h. A control of incubation without cell supernatant was also used. The purity of the samples and the content of clipping species were detected by reduced SDS-PAGE, and the fusion protein heavy chain purity was calculated by BandScan software.

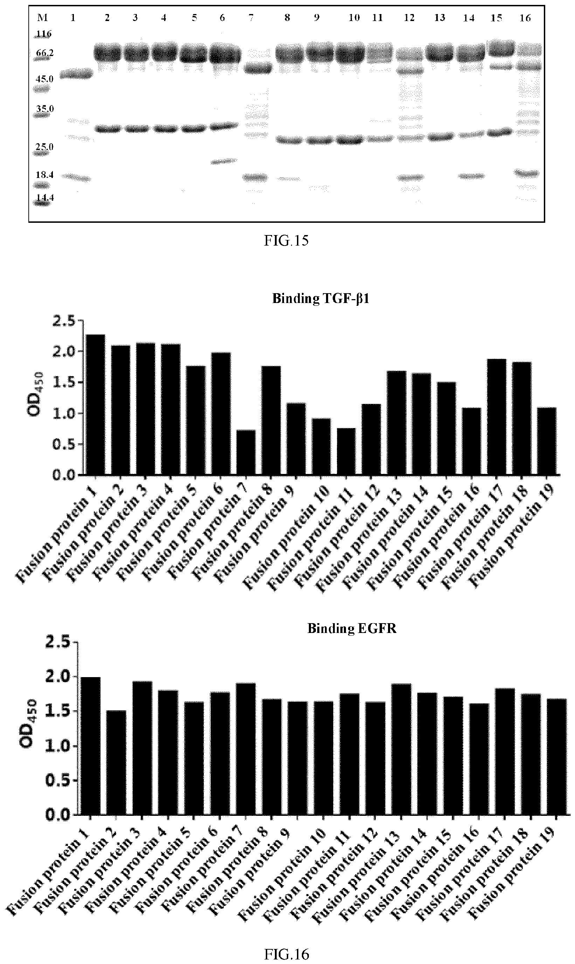

The results of the assay are shown in FIG. 15, and the fracture percentage in each sample is shown in Table 12. The results showed that the percentage of sheared bodies of TGFβR2 truncated form fusion protein (fusion protein 2-16) in the control group were all less than 4.0%, which was much lower than that of TGFβR2 full-length form fusion protein (fusion protein 1) at 24.8%. After incubation with the same cell supernatant for 48 h at 37°C, all of the TGFβR2 full-length form fusion proteins (fusion protein 1) were sheared in the experimental group, with a percentage of 100%. The truncated forms of TGFβR2 fusion proteins (fusion proteins 2-16) contained different percentages of clipping species but were significantly better than the full-length control, wherein fusion protein 2, fusion protein 6, fusion protein 9, fusion protein 10, and fusion protein 13 showing the best performance, with the percentage of clipping species content less than 3.0%.

**Table 12 Degradation ratio of EGFR antibody/TGFβR2 fusion proteins containing different truncated forms of TGFβR2**

| Samples | Experimental group | | Control group | |
|---|---|---|---|---|
| | Percentage of heavy chain % | Percentage of clipping species % | Percentage of heavy chain % | Percentage of clipping species % |
| Fusion protein1 | 0 | 100 | 75.2 | 24.8 |
| Fusion protein2 | 97.3 | 2.7 | 96.8 | 3.2 |
| Fusion protein3 | 96.4 | 3.6 | 96.1 | 3.9 |
| Fusion protein4 | 91.2 | 8.8 | 97.6 | 2.4 |
| Fusion protein5 | 91.0 | 9.0 | 98.1 | 1.9 |
| Fusion protein6 | 97.2 | 2.8 | 97.2 | 2.8 |
| Fusion protein7 | 6.8 | 93.2 | 97.2 | 2.8 |
| Fusion protein8 | 87.7 | 12.3 | 97.5 | 2.5 |
| Fusion protein9 | 98.0 | 2.0 | 98.6 | 1.4 |
| Fusion protein10 | 98.3 | 1.7 | 99.0 | 1.0 |
| Fusion protein11 | 95.1 | 4.9 | 99.2 | 0.8 |
| Fusion protein12 | 40.8 | 59.2 | 99.7 | 0.3 |
| Fusion protein13 | 97.5 | 2.5 | 96.8 | 3.2 |
| Fusion protein14 | 93.9 | 6.1 | 100 | 0 |
| Fusion protein15 | 81.6 | 18.4 | 99.1 | 0.9 |
| Fusion protein16 | 30.0 | 70.0 | 99.0 | 1.0 |

The above results indicate that the EGFR antibody/TGFβR2 fusion proteins of different truncated forms of TGFβR2 are more resistant to protease degradation than TGFβR2 fusion protein 1 of the full-length form.

### 3.4 Binding assay of EGFR antibody/TGFβR2 fusion protein of different truncated forms of TGFβR2 for TGF-β

One hundred ng/mL TGF-β1 and 40 ng/mL EGFR-His proteins were coated on 96-well plates at 100 µL/well, respectively, overnight at 4 °C. The plates were washed the next day, blocked at room temperature for 1 h, and 2 µg/mL of EGFR antibody/TGFβR2 fusion protein of different truncated forms of TGFβR2 was added at 100 µL/well. Washed the plate after 1 h incubation to remove unbound antibody, added secondary antibody Goat anti-hIgG Fc/HRP and incubated and repeated the wash. Finally, the substrate chromogenic solution was added for color development, and the OD₄₅₀ was read by the a microplate reader after termination. The results are shown in FIG. 16. The binding ability of EGFR antibody/TGFβR2 fusion protein with different truncated forms of TGFβR2 to TGF-β1 was different, but the binding ability to EGFR was similar.

### 3.5 Neutralizing assay of EGFR antibody/TGFβR2 fusion protein of different truncated forms of TGFβR2 for TGF-β

TGF-β regulates cellular functions by regulating the transcription of multiple target genes. Plasminogen activator inhibitor 1 (PAI-1) is an important target of the TGF-β1/Smad signaling pathway downstream, and activates Smad3 binds to the cis-acting element of the PAI-1 promoter region to regulate the expression of PAI-1. The element containing the PAI-1 promoter region is inserted in a specific form into a luciferase-containing vector and transferred into HepG2 cells. In this reporter gene system, the addition of exogenous TGF-β protein initiates the expression of the luciferase reporter gene and luminescence in the presence of substrate. When an exogenous TGF-β antibody is added, it neutralizes the TGF-β protein, blocks the binding of TGF-β to TGFβR2, inhibits the downstream signaling pathway, and finally inhibits the expression of the luciferase reporter gene. Therefore, the in vitro efficacy of the TGF-β antibody neutralizing TGF-β can be determined by detecting the intensity of the light signal.

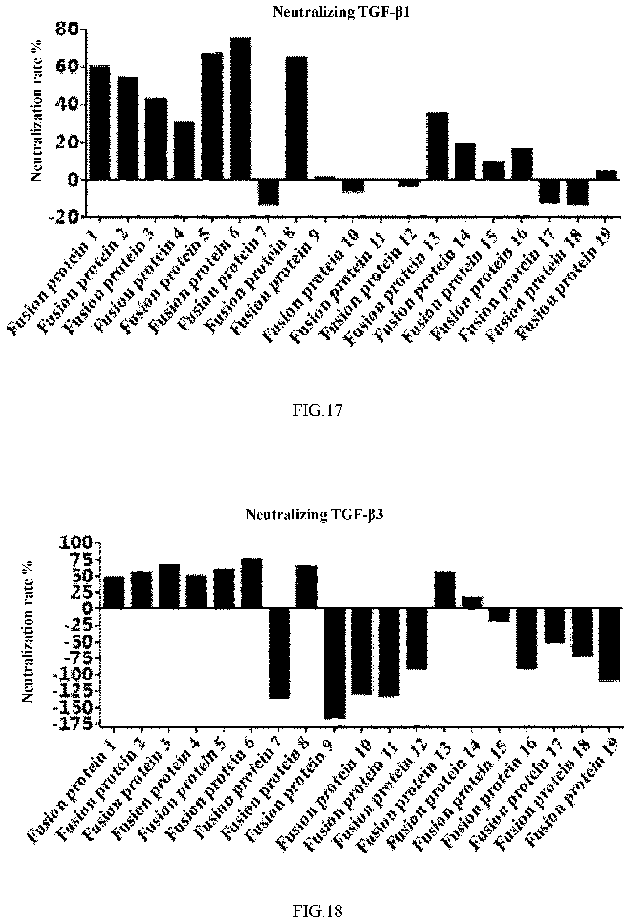

The 96-well plates were uniformly inoculated with HepG2-3TP-Luc2p-puro cells (source: Sinocelltech Limited, same below) at 30,000 cells/well. After overnight monolayer culture, the medium in the 96-well plate was discarded and replaced with a DMEM medium containing 0.5% FBS and incubated for 6 h at 37°C in a 5% CO₂ incubator. The medium in the 96-well plate was discarded and 4 ng/mL TGF-β1 protein was added, along with EGFR antibody/TGFβR2 fusion protein at a final concentration of 0.02 µg/ml, and incubated for 18 h at 37°C in a 5% CO₂ incubator. The negative control M group (containing cells and TGF-β1) and the negative control M' group (containing cells without TGF-β1) were used at the same time. Finally, 5 × Lysis buffer was added and 10 µL of cell samples were taken to detect the bioluminescence intensity value (RLU), and the neutralization rate of the EGFR antibody/TGFβR2 fusion protein was calculated. Neutralization rate (%) = (RLU value of M Group - RLU value of the sample)/( OD value of M Group - OD value of M' Group) × 100%. The concentration of antibodies was used as the horizontal coordinate and the antibody neutralization rate was used as the vertical coordinate, and the quantitative efficacy curves were analyzed and plotted using GraphPad Prism software. As shown in FIG. 17, EGFR antibody/TGFβR2 fusion protein 2, fusion protein 6, fusion protein 8, fusion protein 13, fusion protein 14, and fusion protein 16 containing the truncated forms of TGFβR2 had a certain ability to neutralize TGF-β1, among which fusion protein 2 had similar neutralization ability as fusion protein 1 containing the full-length form of TGFβR2, while fusion protein 5, fusion protein 6 and fusion protein 8 showed better neutralization ability than fusion protein 1 at this concentration. Fusion protein 6 exhibited the strongest ability to neutralize TGF-β1. The remaining EGFR antibody/TGFβR2 fusion proteins containing the truncated form of TGFβR2 had essentially no or weak neutralizing ability.

The TGF-β3 protein has a high affinity for TGFβR2 and can activate TGF-β downstream signaling. The ability of the fusion protein to neutralize TGF-β3 (20 ng/mL final concentration) was assayed by applying the reporter gene system in this example. The results are shown in FIG. 18. Fusion protein 2, fusion protein 4, and fusion protein 13 had a similar ability to neutralize TGF-β3 as fusion protein 1, while fusion protein 3, fusion protein 5, fusion protein 6, and fusion protein 8 had a better ability to neutralize TGF-β3 than fusion protein 1. Fusion protein 6 also showed the strongest ability to neutralize TGF-β3.

Based on the above analysis of the stability and neutralization ability of fusion proteins containing TGFβR2 truncated forms, the present invention prefers TGFβR2 truncated forms of fusion proteins 2 ~ 6, 8, 13, 14, and 16, more preferably TGFβR2 truncated forms of fusion proteins 2, 5, 6 and 8, and most preferably TGFβR2 truncated forms of fusion protein 6.

### Example 4: In vitro biological property of fusion protein 6, an EGFR antibody/TGFβR2 fusion protein of TGFβR2 truncated form

### 4.1 assay of binding ability of EGFR antibody/TGFβR2 fusion protein

### 4.1.1 Properties of EGFR antibody/TGFβR2 fusion protein binding and competing for TGF-β

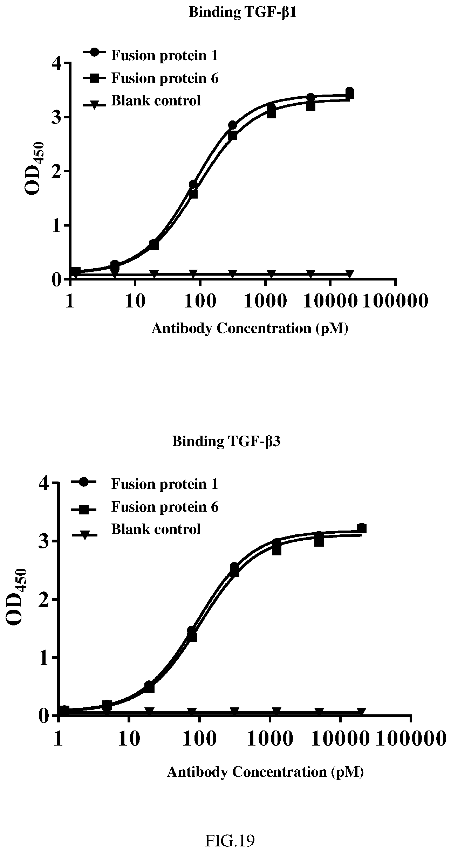

TGF-β1 protein and TGF-β3 protein at a final concentration of 2 µg/mL were coated on 96-well plates at 100µ L/well, respectively, and coated overnight at 4 °C. The plates were washed the next day, blocked at room temperature for 1 h, and incubated with different concentrations (1.22 pM, 4.88 pM, 19.53 pM, 78.13 pM, 312.5 pM, 1250 pM, 500 pM, 2000 pM) of EGFR antibody/TGFβR2 fusion protein 6 for 1 h. After that, the plates were washed to remove unbound antibodies, and incubated with secondary antibody Goat anti-hIgG₅₀² F(ab)2/HRP and were repeatedly washed, the substrate chromogenic solution was added for color development, and the OD₄₅₀ was read by a microplate reader after termination. The results are shown in FIG. 19. The binding ability of fusion protein 6 for TGF-β1 and TGF-β3 proteins was similar to that of fusion protein 1, with an EC₅₀ of 91 pM and R²=0.998 for binding to TGF-β1 protein, and with an EC₅₀ of 102 pM and R²=0.998 for binding to TGFβ3 protein.

This example further analyzes the ability of fusion protein 6 to compete with TGF-β1 protein or TGF-β3 protein for binding to TGFβR2-Fc protein at the protein level.

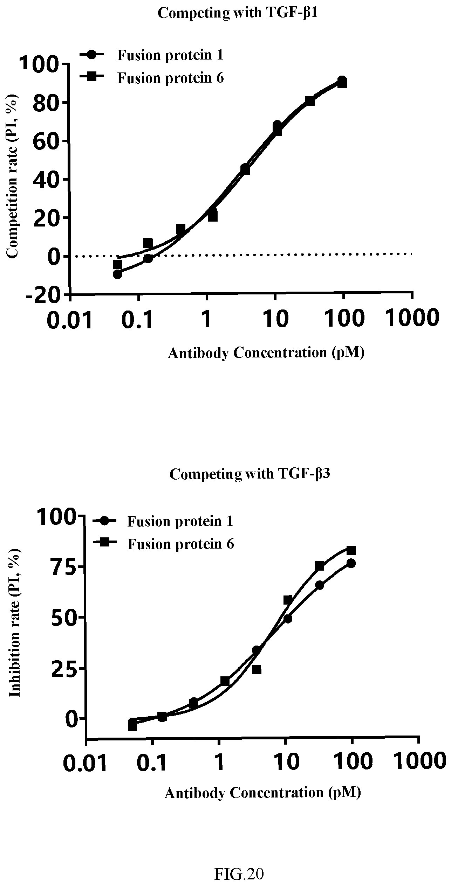

TGF-β1 protein at a final concentration of 0.2 µg/mL or TGF-β3 protein at 0.5 µg/mL was coated on a 96-well plate at 100 µL/well and overnight at 4 °C. The plates were washed the next day, blocked at room temperature for 1 h, and 100 µL of EGFR antibody/TGFβR2 fusion protein at different concentrations (0.05 nM, 0.14 nM, 0.42 nM, 1.25 nM, 3.75 nM, 11.24 nM, 33.71 nM, 101.12 nM) were added with 100 µL of the final concentration of 0.2 µg/mL (TGF-β1 competition) or 1 µg/mL (TGF-β3 competition) of biotin-labeled TGFβR2-Fc protein (protein source: Sino Biological, Inc. and biotin-labeled by Sinocelltech Limited, same below). Positive control wells with TGFβR2-Fc protein only were also used. The plate was washed after 1 h incubation, and the plate was repeatedly washed after 1 h incubation by adding detection secondary antibody Streptavidin/HRP. Finally, the substrate chromogenic solution was added for color development, and after termination, the a microplate reader read OD₄₅₀. The competitive inhibition rate PI% of the fusion protein was calculated based on the OD₄₅₀ value, and the inhibition rate PI (%) = (OD₄₅₀ value of positive wells - OD₄₅₀ value of sample wells)/OD₄₅₀ value of positive wells × 100%. The results are shown in FIG. 20, and fusion protein 6 has a similar ability to block the binding of TGF-β1 protein or TGF-β3 protein to TGFβR2-Fc as fusion protein 1.

### 4.1.2 Binding properties of EGFR antibody/TGFβR2 fusion protein for EGFR

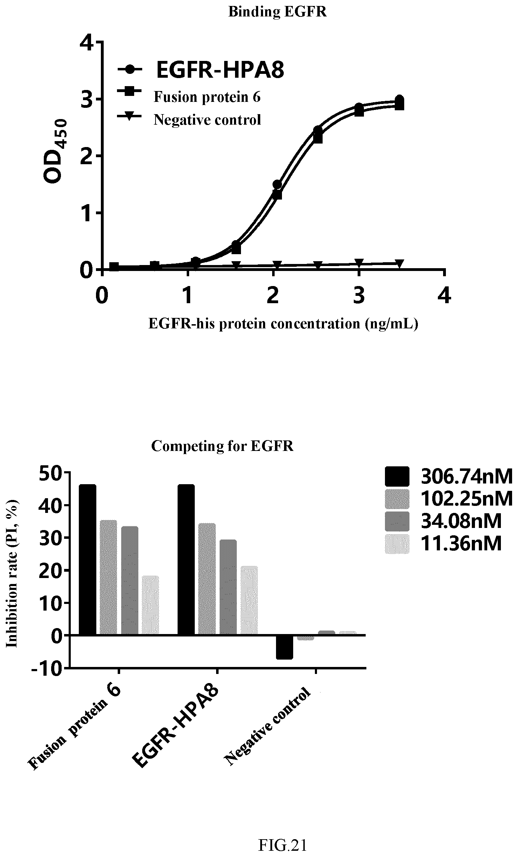

Referring to Example 1.2.1, the binding ability of the fusion protein to recombinant human EGFR protein was measured by ELISA. As shown in FIG. 21, the binding ability of fusion protein 6 to EGFR and the ability to compete with EGF to bind to EGFR was similar to that of EGFR-HPA8, with a binding EC₅₀ of 133.1 ng/mL and R² = 1.000.

### 4.2 EGFR antibody/TGFβR2 fusion protein binding affinity assay

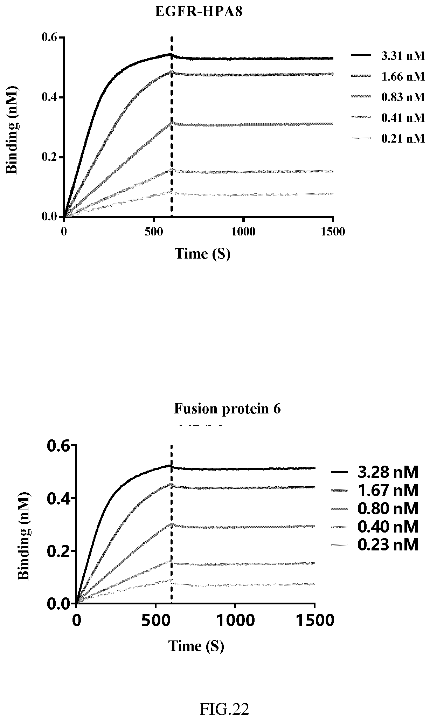

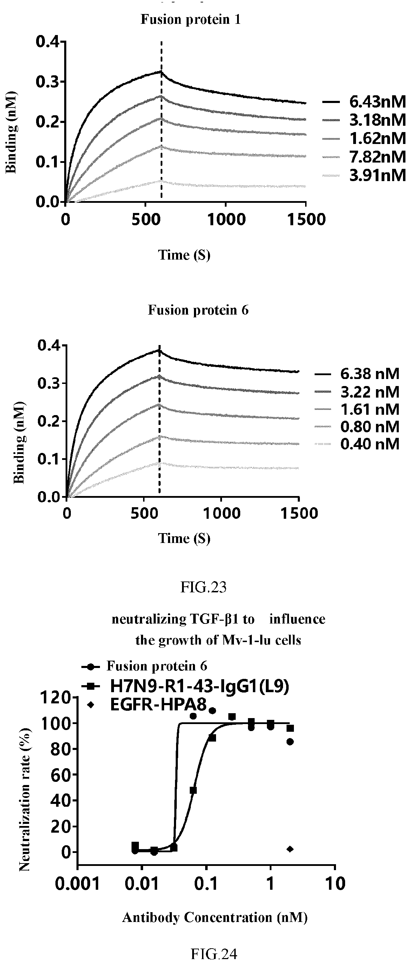

In this example, the affinity of EGFR antibody/TGFβR2 fusion protein binding to biotinylated recombinant human EGFR protein and TGF-β1 protein was determined using a biomolecular interaction analysis system (model: OctetRED96e, manufacturer: Fortebio) with EGFR-HPA8 and H7N9-R1, respectively, as negative controls. Affinity parameters were derived by fitting multiple concentration point binding and dissociation curves, and the results are shown in Tables 13 and 14, and the specific kinetic characteristic parameter curves are shown in Figures 22 and 23.

The results showed that fusion protein 6 retained a higher binding affinity to human EGFR protein compared to the monoclonal antibody EGFR-HPA8, with a KD value of 8.77 pM, a binding constant kₒₙ value of 1.68E+06 M⁻¹ s^{-1,} and a dissociation constant k_{dis} of 1.47E-05 s⁻¹. Furthermore, the truncated form of TGFβR2 fusion protein 6 has a similar affinity to human EGFR protein to the full-length form of TGFβR2 fusion protein 1 with a k_{D} value of 96.1 pM, a binding constant kₒₙ value of 1.53E+06 M s⁻¹⁻¹, and a dissociation constant k_{dis} of 1.47E-04 s .⁻¹

**Table 13 Affinity of truncated EGFR antibody/TGFβR2 fusion protein to recombinant human EGFR protein**

| Sample | K_{D} (M) | kon (M⁻¹s⁻¹) | kdis (s⁻¹) |
|---|---|---|---|
| EGFR-HPA8 | 6.90E-12 | 1.59E+06 | 1.10E-05 |
| Fusion protein 6 | 8.77E-12 | 1.68E+06 | 1.47E-05 |

**Table 14 Affinity of truncated EGFR antibody/TGFβR2 fusion protein to recombinant human TGF-β1 protein**

| Sample | K_{D} (M) | kon (M⁻¹s⁻¹) | kdis (s⁻¹) |
|---|---|---|---|
| Fusion protein 1 | 1.73E-10 | 1.45E+06 | 2.51E-04 |
| Fusion protein 6 | 9.61E-11 | 1.53E+06 | 1.47E-04 |

The above results indicate that fusion protein 6 has a good affinity with both human EGFR and TGF-β1.

### 4.3 Assay of EGFR antibody/TGFβR2 fusion protein neutralizing TGF-β

TGF-β1 can inhibit the proliferation of Mv-1-1u cells, so the ability of EGFR antibody/TGFβR2 fusion protein to neutralize TGF-β1 can be detected using the WST-8 assay.

Mv-1-lu cells (source: Cell Resource Center, Shanghai Institutes for Biological Sciences, Chinese Academy of Sciences) were inoculated uniformly with 50 µL/well in a 96-well plate at a cell inoculation density of 1×10³ /well. Cells were incubated in a CO₂ incubator for about 3 h to adhere to the well wall, and then EGFR antibody/TGFβR2 fusion protein samples at different concentrations (0.0078 nM, 0.0156 nM, 0.0313 nM, 0.0625 nM, 0.125 nM, 0.25 nM, 0.5 nM, 1 nM, 2 nM) diluted in 1640 medium containing 10% FBS was added with 50 µL/well. Finally, the TGF-β1 factor at a final concentration of 1 ng/mL was added with 10 µL/well. Positive control M group (containing cells and TGF-β1), negative control M' group (containing cells without TGF-β1), and blank control B group (adding only culture medium without cells) were also used. Cells were incubated in CO₂ incubator at 37°C and 5% CO₂ for 5 days and then WST-8 was added at 10 µL/well. The samples were left for 180 min and the reading of OD₄₅₀ -OD₆₃₀ was measured by a microplate reader, and the neutralization rate of EGFR antibody/TGFβR2 fusion protein was calculated by subtracting the reading of blank control B. Neutralization rate (%) = (OD of M' group - OD of the sample)/(OD of M' group - OD of M group) × 100%, and the quantitative efficacy curve was analyzed and plotted using GraphPad Prism software, the horizontal coordinate is the antibody concentration and the vertical coordinate is the inhibition rate. As shown in FIG. 24, both fusion protein 6 and the control product H7N9-R1-43-IgG1 (L9) of TGFβR2 (source: Sinocelltech Limited for biotin labeling, same below) could effectively neutralize the inhibition of Mv-1-lu proliferation by TGF-β1 in a dose-dependent relationship, and the half effective concentration of fusion protein 6 was smaller than that of the control product H7N9- R1-43-IgG1 (L9), indicating a better neutralizing activity of the molecule. EGFR-HPA8 did not neutralize TGF-β1, suggesting that it is the TGFβR2 portion of fusion protein 6 that shows a neutralizing effect on TGF-β1.

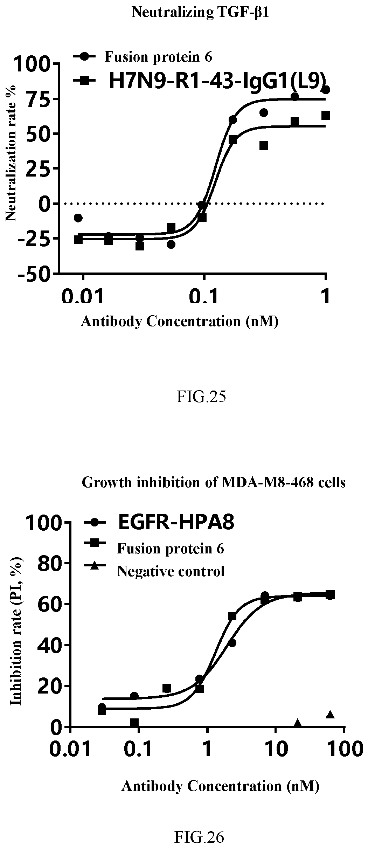

The ability of fusion protein 6 to neutralize TGF-β was further assayed by using the reporter gene system in this example, referring to Example 3.5. The results are shown in FIG. 25, and both fusion protein 6 and the control product H7N9-R1-43-IgG1 (L9) of TGFβR2 can effectively neutralize TGF-β1 in a dose-dependent relationship, and the maximum neutralization rate of fusion protein 6 ( 74.8%) was much higher than that of the control product H7N9-R1-43-IgG1 (L9) (55.3%), further indicating the excellent neutralizing activity of fusion protein 6.

### 4.4 Assay of cell proliferation inhibitory activity of EGFR antibody/TGFβR2 fusion protein

The EGFR antibody/TGFβR2 fusion protein inhibition on the growth of MDA-MB-468 cells was assayed by the WST-8 method according to Example 1.2.2 to determine its EGFR portion property. As shown in FIG. 26, the ability of fusion protein 6 to inhibit the proliferation of MDA-MB-468 cells was similar to that of EGFR-HPA8, and the inhibition rate increased with the increase of medicament concentration in an "S" curve. The control product H7N9-R1-43-IgG1 (L9) with TGFβR2 function did not inhibit the proliferation of MDA-MB-468 cells, indicating that fusion protein 6 inhibited the proliferation of tumor cells by its EGFR portion.

### 4.5 ADCC effect of EGFR antibody/TGFβR2 fusion protein

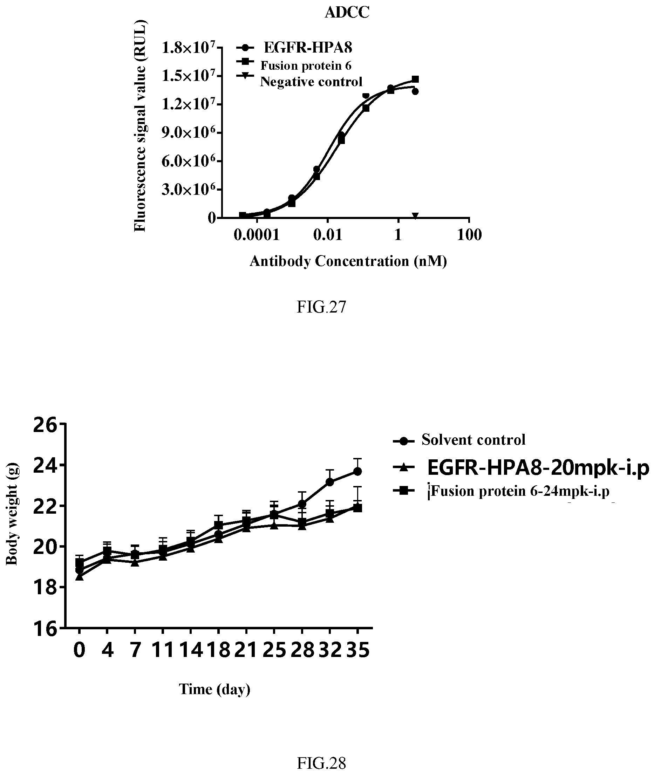

The ADCC effect mediated by EGFR antibody/TGFβR2 fusion protein on EGFR-expressing cells was assayed with reference to Example 2.2.3. The results are shown in FIG. 27. In the concentration range of 0.00004-3 nM, fusion protein 6 and anti-EGFR antibody EGFR-HPA8 can mediate similar ADCC effects on EGFR-expressing tumor cells A431. The control product H7N9-R1-43-IgG1 (L9) with TGFβR2 function had no ADCC effect on A431 cells, indicating that it is the EGFR portion of fusion protein 6 that mediates the ADCC function in this experimental system.

### Example 5: Pharmacodynamic study on TGFβR2 truncated form (fusion protein 6) EGFR antibody/TGFβR2 fusion protein in NCI-H1975 subcutaneous graft tumor model

Balb/c-nu mice were subcutaneously inoculated with 1×10⁶ NCI-H1975 cells on the right side of the rib cage. When the tumor volume reached about 300 mm³, the animals were randomly grouped by tumor volume to 6 animals in each group and administered. Dosing was started on the day of the grouping, the medicament was administered by intraperitoneal injection (I.P.) twice a week for 10 consecutive doses, and was discontinued after the last dose to observe tumor recurrence. The specific dosing regimen is shown in Table 15 below.

**Table 15 Grouping and Dosing**

| Group | Number of animals | Subject medicament | Dose(mg/kg) | Administration route | the course of administration |
|---|---|---|---|---|---|
| 1 | 6 | Vehicle | / | Intraperitoneal injection | BIW×5w |
| 2 | 6 | EGFR-HPA8-Ae0 | 20 | Intraperitoneal injection | BIW×5w |
| 3 | 6 | Fusion protein 6 | 24^{b} | Intraperitoneal injection | BIW×5w |

| | | | | | |
|---|---|---|---|---|---|
| Note: The administration volume was10 mL/kg the body weight of mice . | | | | | |

The animals in each group were in a good general condition such as activity and feeding during the course of administration, and their body weight increased to some extent. There was no significant difference in body weight between the dosing group and the solvent control group after dosing (*P* > 0.05). The changes in body weights of all animals are shown in Table 16 and FIG. 28.

**Table 16 Effect of fusion protein 6 on the body weight of H1975 non-small cell lung cancer subcutaneous xenografted tumor mice**

| Group p | Subject medicament | Number of animals | Body weight (g) | | | Weight change thoroughout the course of administration (g) |
|---|---|---|---|---|---|---|
| | | | Before administration | after 35 days of administration^{a} | *P* value^{b} | |
| 1 | Solvent control | 6 | 18.8±0.2 | 23.7±0.6 | / | +4.3 |
| 2 | EGFR-HPA8 | 6 | 18.5±0.5 | 22.0±0.9 | 0.164 | +2.9 |
| 3 | Fusion protein 6 | 6 | 19.2±0.4 | 21.9±0.4 | 0.030 | +2.4 |

| | | | | | | |
|---|---|---|---|---|---|---|
| ^{a}Means ± standard deviations. ^{b}Statistical comparison of body weight in the treatment group with that in the solvent control group after 35 days of administration, t-test. | | | | | | |

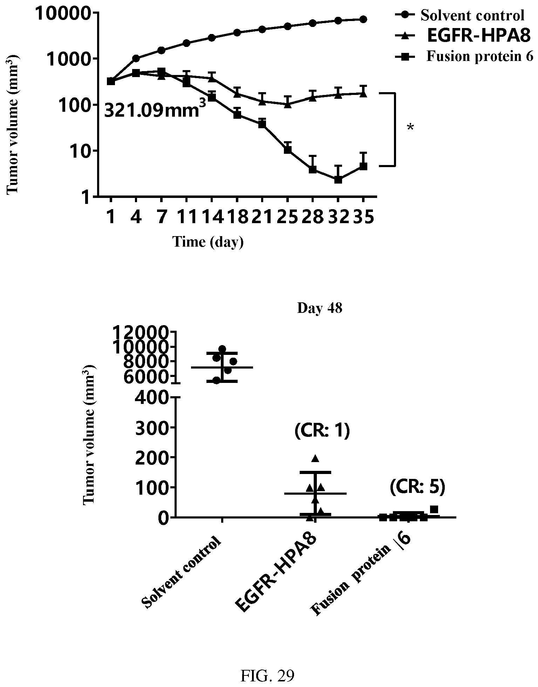

The tumor volume results for each group in the trial are shown in Table 17 and FIG. 29. After 35 days of treatment in groups, the mean tumor volume in the solvent control group was 7150.78 ± 780.4 mm³. Five of the six mice in the fusion protein 6 administration group had complete tumor disappearance (CR) with a mean tumor volume of 4.55 ± 4.55 mm³ and a TGI of 99.9%, which was significantly different from that of the solvent control group (P < 0.001). In contrast, only one mouse in the EGFR-HPA8 administration group showed complete tumor disappearance, with a mean tumor volume of 79.44 ± 28.65 mm³ and a TGI of 98.9%. The results indicated that fusion protein 6 had a significant inhibitory effect on NCI-H1975 non-small cell lung cancer subcutaneous transplanted tumors and the tumor-inhibiting effect was superior to that of EGFR-HPA8 at the same molar dose (*P* = 0.237).

**Table 17 Effect of fusion protein 6 on tumor volume in mice in H1975 non-small cell lung cancer xenografted tumor model**

| Day | Tumor volume of solvent control (mm³) | Tumor volume of EGFR-HPA8 (mm³) | TGI (%) | *P*^{a} | Tumor volume of fusion protein 6 (mm³) | TGI (%) | *P*^{b} |
|---|---|---|---|---|---|---|---|
| 1 | 324.77±37.33 | 323.06±34.66 | - | 0.9739 | 323.20±30.48 | - | 0.9747 |
| 4 | 1004.24±100.19 | 479.20±57.44 | 52.00% | 0.0011 | 490.36±38.50 | 50.90% | 0.0007 |
| 7 | 1506.18±138.21 | 416.80±57.56 | 72.20% | 0 | 528.37±88.27 | 64.70% | 0.0001 |
| 11 | 2167.62±204.64 | 425.54±82.69 | 80.30% | 0 | 288.30±78.16 | 86.60% | 0 |
| 14 | 2825.99±217.47 | 299.87±60.52 | 89.30% | 0 | 143.14±47.80 | 94.90% | 0 |
| 18 | 3685.00±626.56 | 152.98±48.45 | 95.80% | 0 | 60.65±24.72 | 98.30% | 0 |
| 21 | 4315.02±336.11 | 118.78±33.71 | 97.20% | 0 | 37.65±11.93 | 99.10% | 0 |
| 25 | 5031.82±414.57 | 64.63±16.11 | 98.70% | 0 | 10.36±4.92 | 99.80% | 0 |
| 28 | 5895.36±504.19 | 77.86±18.00 | 98.70% | 0 | 3.86±3.86 | 99.90% | 0 |
| 32 | 6722.83±477.48 | 85.01±27.00 | 98.70% | 0 | 2.33±2.33 | 100.00% | 0 |
| 35 | 7150.78±780.4 | 79.44±28.65 | 98.90% | 0 | 4.55±4.55 | 99.90% | 0 |
| 39 | 7959.47±1094.45 | 112.85±39.69 | 98.60% | 0 | 6.53±6.53 | 99.90% | 0 |
| 42 | 7862.70±1213.66 | 161.12±59.62 | 97.90% | 0.0001 | 5.39±5.39 | 99.90% | 0.0001 |
| 47 | 8411.14±1224.57 | 231.39±90.64 | 97.20% | 0.0001 | 5.26±5.26 | 99.90% | 0 |

| | | | | | | | |
|---|---|---|---|---|---|---|---|
| ^{a} Statistical comparison of tumor volumes in the EGFR-HPA8-Ae0 treatment group with those in the solvent control group, t-test. ^{b}Statistical comparison of tumor volumes in the fusion protein 6 treatment group with those in the solvent control group, t-test. | | | | | | | |

### Example 6: Stability Analysis of TGFβR2 Truncated Form (Fusion Protein 6) EGFR antibody/TGFβR2 fusion protein

### 6.1 Ultrafiltration stability analysis of EGFR antibody/TGFβR2 fusion protein

EGFR antibody/TGFβR2 fusion protein samples were concentrated to a concentration of about 10 mg/mL using ultrafiltration in 100 mM Glycine, 10 mM NaCl, 50 mM Tris, pH 7.5 buffer. The concentrated samples were tested for purity and stability of the ultrafiltered samples by reduced SDS-PAGE and molecular sieve chromatography (SEC-HPLC, Agilent 1260 liquid chromatography system, TSK-G3000SWXL column). EC-HPLC operation steps: (1) mobile phase: 200 mM NaH₂PO₄, 100mM Arginine, pH 6.5; (2) the loading volume was 80 µg; (3) the analysis time was 30 min, the flow rate was 0.5 mL/min, and the column temperature was 25 °C; (4) the purity was calculated according to the normalization method of peak area.

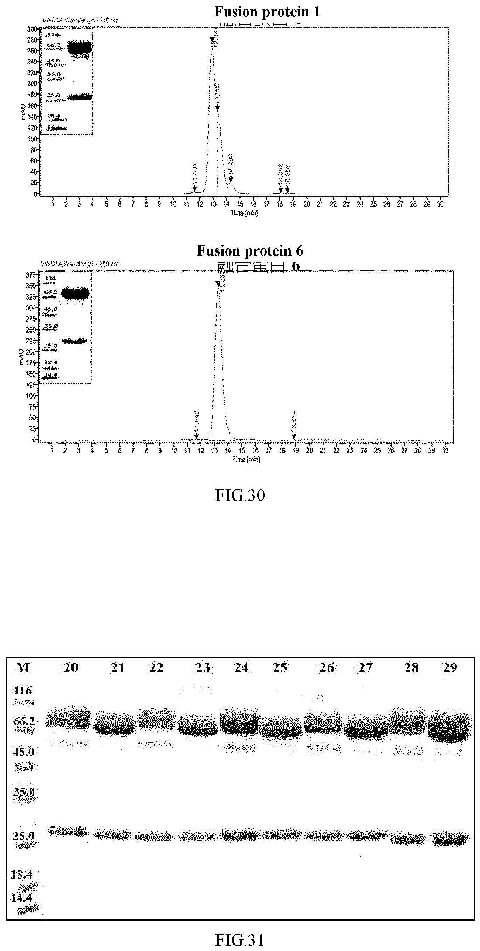

The results of the purity test after sample concentration are shown in FIG. 30, and the sample purity and fragment ratios are shown in Table 18. The results showed that the TGFβR2 truncated fusion protein 6 was less likely to break after concentration and had higher ultrafiltration stability compared with the full-length TGFβR2 fusion protein.

**Table 18 SEC results of ultrafiltration stability of EGFR antibody/TGFβR2 fusion protein**

| Sample | SEC (%) | | | Reduced SDS-PAGE Heavy chain (%) |
|---|---|---|---|---|
| | Monomers | Fragments | Fragment increase % | |
| Fusion protein 1 | 68.7 | 24.8 | 24.8 | 92.3 |
| Fusion protein 6 | 99.8 | 0.1 | 0.1 | 95.6 |

### 6.2 Thermal stability analysis of EGFR antibody/TGFβR2 fusion protein

The thermal stability of the samples was measured by differential scanning fluorimetry (DSF) using an UNcle system (Unchained Labs, model: UNCLE-0330). Operation steps: (1) The sample volume was 9 µL; (2) The experimental parameters were set: the temperature range was 25°C to 95°C, and the heating rate was 0.3°C/min; (3) the UNcle Analysis software was applied to analyze the data, the midpoint value of the internal fluorescence change curve under UV266 was taken as Tm, and the aggregation onset temperature of the aggregation change curve formed by the static light scattering signal under UV266/Blue473 was taken as Tagg266 and Tagg473.

The results of the thermal stability assay of fusion protein 6 were shown in Table 19, which exhibited good thermal stability.

**Table 19 Tm assay results of fusion protein 6**

| Sample | Tm (CH2) | Tm (Fab) | Tagg266 | Tagg473 |
|---|---|---|---|---|
| Fusion protein 6 | 69.7 | 80.8 | 73 | 74.7 |

### 6.3 Thermal accelerated stability analysis of EGFR antibody/TGFβR2 fusion protein

After the samples were stored at 45°C for 1 week, the accelerated stability of the samples was analyzed by SEC-HPLC and SDS-PAGE, and the procedure was the same as 6.1.

The results of thermal accelerated stability of fusion protein 6 are shown in Table 20. After 1 week of storage at 45°C, the SEC purity of fusion protein 6 decreased by 0.7%, but the purity was still high, the level of aggregates increased less and the level of fragments did not change, which showed good thermal accelerated stability.

**Table 20 Thermal acceleration stability assay results of fusion protein 6**

| Sample | Treatment condition | SEC (%) | | |
|---|---|---|---|---|
| | | Aggregates | Monomers | Fragments |
| Fusion protein 6 | No treatment | 0 | 100 | 0 |
| | 1-week storage at 45°C | 0.6 | 99.3 | 0 |
| | Range of variation | 0.6 | 0.7 | 0 |

### 6.4 Freeze-thaw stability analysis of EGFR antibody/TGFβR2 fusion protein

The samples were stored at -80°C for 3 h and then transferred to 45°C for 1 h to thaw, and so on for five repeated freeze-thaws. The freeze-thaw stability of the samples was analyzed by SEC-HPLC, and the procedure was the same as in 6.1.

The results of freeze-thaw stability of fusion protein 6 are shown in Table 21. After five repeated freeze-thaws, the SEC purity of fusion protein 6 did not change significantly, and the levels of aggregates and fragments did not increase significantly, which showed good freeze-thaw stability.

**Table 21 Freeze-thaw stability assay results of fusion protein 6**

| Sample | Treatment condition | SEC (%) | | |
|---|---|---|---|---|
| | | Aggregates | Monomers | Fragments |
| Fusion protein 6 | No treatment | 0.1 | 99.8 | 0.1 |
| | repeated freeze-thaws | 0.2 | 99.6 | 0.2 |
| | Range of variation | 0.1 | 0.2 | 0.1 |

### 6.5 Shaking stability of EGFR antibody/TGFβR2 fusion protein

The samples were placed in deep-well plates and shaken on a vortex shaker at 800 rpm for 24 h. The samples were analyzed by SEC-HPLC with the same procedure as in 6.1. The results are shown in Table 22, which indicates that the SEC monomer purity of the samples did not change significantly after 24 h of shaking, and the levels of aggregates and fragments did not increase significantly, indicating that fusion protein 6 has good shaking stability.

**Table 22 Shaking stability assay results of fusion protein 6**

| Sample | Treatment condition | SEC (%) | | |
|---|---|---|---|---|
| | | Aggregates | Monomers | Fragments |
| Fusion protein 6 | T0 | 0.3 | 99.7 | 0 |
| | Shaking | 0.3 | 99.7 | 0 |

### Example 7: Design and property assay of solid tumor antigen-targeting antibody X/TGFβR2 fusion protein , wherein the TGFβR2 portion is truncated forms of TGFβR2 (fusion protein 6)

### 7.1 Design of TGFβR2 X antibody / truncated TGFβR2 fusion protein expression vector, expression, and purification

To further validate the structural stability and the ability to neutralize TGF-β1 of the elected TGFβR2 truncated form in Example 3.2, this Example uses a variety of solid tumor antigens as the targeting portion of the fusion protein and the TGFβR2 extracellular domain (full-length and ECD delete (6-26) fusion protein 6) as the immunomodulatory portion of the fusion protein to form the X antibody/ TGFβR2 extracellular domain fusion protein (X/TGFβR2 fusion protein). Similarly, in the X/TGFβR2 fusion protein, the C-terminal amino acid of the X antibody heavy chain links to the TGFβR2 extracellular domain via the (G₄S)₄ Linker. In addition, the C-terminal lysine of the X antibody heavy chain was removed to reduce the risk of proteolytic cleavage. The construction protocol of the X/TGFβR2 fusion protein is shown in Table 23.

**Table 23 X/TGFβR2 fusion protein design protocol**

| Sample | Targeting antibody | TGFβR2 ECD | Heavy chain SEQ ID |
|---|---|---|---|
| Fusion protein 20 | Trastuzumab | Full length | SEQ ID NO:127 |
| Fusion protein 21 | Trastuzumab | ECD delete (6-26) | SEQ ID NO:128 |
| Fusion protein 22 | Bevacizumab | Full length | SEQ ID NO:130 |
| Fusion protein 23 | Bevacizumab | ECD delete (6-26) | SEQ ID NO:131 |
| Fusion protein 24 | Ramucirumab | Full length | SEQ ID NO:133 |
| Fusion protein 25 | Ramucirumab | ECD delete (6-26) | SEQ ID NO:134 |
| Fusion protein 26 | Ipilimumab | Full length | SEQ ID NO:136 |
| Fusion protein 27 | Ipilimumab | ECD delete (6-26) | SEQ ID NO:137 |
| Fusion protein 28 | Panitumumab | Full length | SEQ ID NO:139 |
| Fusion protein 29 | Panitumumab | ECD delete (6-26) | SEQ ID NO:140 |

PCR or Overlap-PCR amplifies the target gene and ligates it to the expression vector by in-fusion. The recombinant expression vector was validated by sequencing and the plasmids were extracted, transiently transferred to HEK-293 cells (*fut8* knockout), cultured for 7 days and the supernatant was collected by centrifugation. The obtained cell supernatant was purified using Protein A affinity chromatography to purify the fusion protein.

### 7.2 Degradation of X antibody /truncated TGFβR2 fusion proteins

The purity, as well as the degradation of the X/TGFβR2 fusion protein, was assayed by reduced SDS-PAGE. The results are shown in FIG. 31. For the different X/TGFβR2 fusion proteins, the expressed samples of the elected truncated form of TGFβR2 extracellular domain were significantly more stable and had fewer degradation bands than the full-length TGFβR2 extracellular domain control samples. The stability of the samples should be attributed to the present truncated forms of TGFβR2 extracellular domain, no matter what kinds of the targeting portion, i.e. the antibody is used.

### 7.3 Degradation tendency of X antibody/truncated TGFβR2 fusion protein

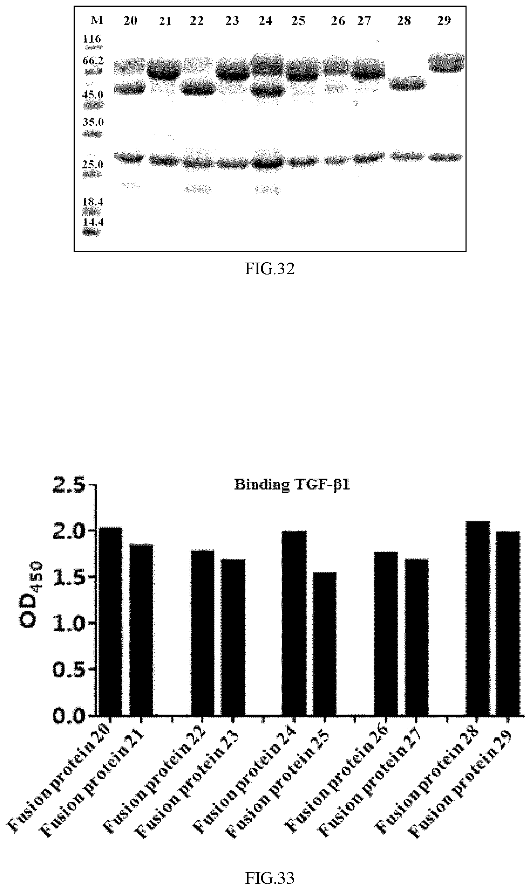

In this example, 293E cell supernatant was used for the accelerated treatment of the fusion protein, and the degradation stability of X/TGFβR2 fusion protein with the elected truncated TGFβR2 extracellular domain was further determined as in Example 3.3. The results of the sample purity assay are shown in FIG. 32, and the sample purity and the percentage of shearers are shown in Table 24. The results indicate that X/TGFβR2 fusion proteins with preferred TGFβR2 extracellular domain truncated form is more resistant to protease degradation than the full-length form of TGFβR2 fusion protein.

**Table 24 Degradation percentage of X/TGFβR2 fusion proteins with truncated TGFβR2**

| Samples | Experimental group | | Control group | |
|---|---|---|---|---|
| | Percentage of heavy chain % | Percentage of clipping species % | Percentage of heavy chain % | Percentage of clipping species % |
| Fusion protein 20 | 15.2 | 84.8 | 94.4 | 5.6 |
| Fusion protein 21 | 98.1 | 1.9 | 96.7 | 3.3 |
| Fusion protein 22 | 4.9 | 94.9 | 80 | 20 |
| Fusion protein 23 | 99.8 | 0.2 | 96.2 | 3.8 |
| Fusion protein 24 | 24.7 | 74.8 | 70.7 | 29.3 |
| Fusion protein 25 | 96.8 | 3.2 | 96.9 | 3.1 |
| Fusion protein 26 | 85.4 | 14.6 | 84.4 | 15.6 |
| Fusion protein 27 | 97.2 | 2.8 | 97.1 | 2.9 |
| Fusion protein 28 | 0 | 100 | 38.8 | 61.2 |
| Fusion protein 29 | 97.1 | 2.9 | 98.5 | 98.5 |

To assay the stability of the X/TGFβR2 fusion protein with the elected truncated TGFβR2 extracellular domain under certain concentration conditions, its ultrafiltration stability was analyzed using the method of Example 6.1. The results are shown in Table 25. The X/TGFβR2 fusion protein with the preferred truncated extracellular domain of TGFβR2 is less prone to degradation after concentration, and the percentage of clipping species content is less than 4.0% (SDS-PAGE purity), which has stronger ultrafiltration stability than the X/TGFβR2 fusion protein with the full-length extracellular domain of TGFβR2.

**Table 25 SEC and SDS degradation assay of concentrated X/TGFβR2 fusion protein**

| Fusion protein No. | SEC-HPLC | | | Reduced SDS-PAGE clipping species (%) |
|---|---|---|---|---|
| | Intact protein % | Fragments % | Fragments increase% | |
| Fusion protein 20 | 93.1 | 1.5 | -1.1 | 19.8 |
| Fusion protein 21 | 94.3 | 0.5 | 0.1 | 0.8 |
| Fusion protein 22 | 67.8 | 23.7 | 6.9 | 10.8 |
| Fusion protein 23 | 91.3 | 0.9 | -0.3 | 3.2 |
| Fusion protein 24 | 82.8 | 16.6 | 16 | 9.1 |
| Fusion protein 25 | 98.5 | 0.6 | 0.1 | 3.3 |
| Fusion protein 26 | 67.2 | 32 | 12.3 | 14.2 |
| Fusion protein 27 | 97.6 | 0.8 | 0.1 | 3.7 |
| Fusion protein 28 | 23 | 77 | 50.2 | 51.1 |
| Fusion protein 29 | 97.5 | 0.1 | 0.1 | 2.5 |

In summary, the superior stability of the fusion protein containing the preferredtruncated TGFβR2 extracellular domain was further validated by the fusion proteins with multiple kinds of solid tumor antigens as the targeting portion.

### Example 8: In vitro biological property of X/TGFβR2 fusion proteins with targeting solid tumors antigen as targeting portion and truncated TGFβR2 (fusion protein 6)

### 8.1 X/TGFβR2 fusion protein Binding TGF-β1 Assay

The binding ability of the X/TGFβR2 fusion protein to TGF-β1 was measured by ELISA according to Example 3.4. As shown in FIG. 33, the ability of the X/TGFβR2 fusion protein with the preferred truncated form of TGFβR2 to bind TGF-β1 is slightly lower than that of the X/TGFβR2 fusion protein with the full-length form of TGFβR2.

### 8.2 X/TGFβR2 fusion protein neutralizing TGF-β assay

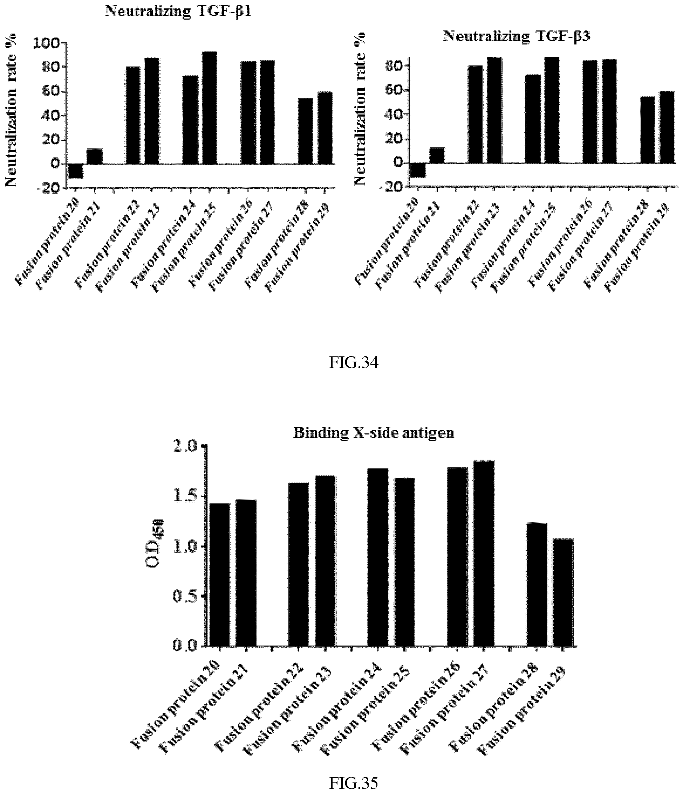

The ability of the X/TGFβR2 fusion protein to neutralize TGF-β1 and TGF-β3 was tested with reference to Example 3.5. As shown in FIG. 34, the ability of the X/TGFβR2 fusion protein with the preferred truncated form of TGFβR2 to neutralize both TGF-β1 and TGF-β3 was superior to that of the X/TGFβR2 fusion protein with the full-length form of TGFβR2.

### 8.3 Binding assay of X/TGFβR2 fusion protein for the target of X portion

The antigens ERBB2-his, VEGF165, VEGFR2-His, CTLA4-his, and EGFR-His, which are X portion target, at final concentrations of 10 ng/mL, 5 ng/mL, 80 ng/mL, 80 ng/mL, and 40 ng/mL, respectively, were coated on 96-well plates at 100 L/well. The plates were coated overnight at 4 °C. The plates were washed the next day, blocked at room temperature for 1 h, and incubated with 100 µL of X/TGFβR2 fusion protein at a final concentration of 13.89 nM for 1 h. The plates were washed to remove unbound antibodies, incubated with secondary antibody Goat anti-hIgG Fc/HRP, and repeated the wash. Finally, the substrate chromogenic solution was added for color development, and the OD₄₅₀ was read by the a microplate reader after termination . As shown in FIG. 35, the X/TGFβR2 fusion protein with the elected truncated TGFβR2 has a similar ability to bind the corresponding antigen on the X side as the X/TGFβR2 fusion protein containing the full-length form of TGFβR2.

### SEQUENCE LISTING

| Numbe r | Name | Sequence |
|---|---|---|
| SEQ ID NO.1 | The amino acid sequence of Human EGFRprotein(UniProtKB-P35968)extracellular domain Metl-Ser645 | MRPSGTAGAALLALLAALCPASRALEEKKVCQGTSNKLTQLGTFE DHFLSLQRMFNNCEVVLGNLEITYVQRNYDLSFLKTIQEVAGYVLI ALNTVERIPLENLQIIRGNMYYENSYALAVLSNYDANKTGLKELP MRNLQEILHGAVRFSNNPALCNVESIQWRDIVSSDFLSNMSMDFQ NHLGSCQKCDPSCPNGSCWGAGEENCQKLTKIICAQQCSGRCRGK SPSDCCHNQCAAGCTGPRESDCLVCRKFRDEATCKDTCPPLMLYN PTTYQMDVNPEGKYSFGATCVKKCPRNYVVTDHGSCVRACGADS YEMEEDGVRKCKKCEGPCRKVCNGIGIGEFKDSLSINATNIKHFKN CTSISGDLHILPVAFRGDSFTHTPPLDPQELDILKTVKEITGFLLIQA WPENRTDLHAFENLEIIRGRTKQHGQFSLAVVSLNITSLGLRSLKEI SDGDVIISGNKNLCYANTINWKKLFGTSGQKTKIISNRGENSCKAT GQVCHALCSPEGCWGPEPRDCVSCRNVSRGRECVDKCNLLEGEP REFVENSECIQCHPECLPQAMNITCTGRGPDNCIQCAHYIDGPHCV KTCPAGVMGENNTLVWKYADAGHVCHLCHPNCTYGCTGPGLEG CPTNGPKIPS |
| SEQ ID NO.2 | The nucleotide sequence encoding the linker used in the construction of the phage antibody library for the linkage of the murine antibody scFv | TCTAGTGGTGGCGGTGGTTCGGGCGGTGGTGGAGGTGGTAGTT CTAGATCTTCC |
| SEQ ID NO.3 | The nucleotide sequence encoding murine antibody scFv used in the construction of antibody EGFR-mhPA8 | The nucleotide sequence encoding EGFR-mhPA8 light chain variable region (SEQ ID NO:5): |
| | | GATATCCAGATGACCCAGTCTCCAGCCTCCCTGGCTGCATCTGT GGGAGAAACTGTCACCATCACATGTCGAGCAAGTGAGAACATT TACTACAGTTTAGCTTGGTATCAGCAGAAGGAAGGGAAATCTCC TCAGCTCCTGATCTATATTACAAACGGCTTGGCAGATGGTGTCCC ATCGAGGTTCAGTGGCAGTGGATCTGGGACACAGTATTCTATGA AGATCGACAGCATGCAGCCTGAAGATACCGCAACTTATTTCTGT AAACAGTCTTATGACGTTCCGCTCACGTTCGGTGCTGGGACCAA GCTGGAGATGAAA |
| | | Linker (SEQ ID NO:2): |
| | | TCTAGTGGTGGCGGTGGTTCGGGCGGTGGTGGAGGTGGTAGTT CTAGATCTTCC |
| | | The nucleotide sequence encoding EGFR-mhPA8 light heavy variable region (SEQ ID NO:4): |
| | | CAGGTGCAGCTGCAGCAATCTGGACCTGATTTGGTGAAGCCTG GGGCTTCAGTGAGGATATCTTGCAAGGTTTCTGGCTACACCTTC ACAACCTACTATACACACTGGGTGAAGCAGAGGCCTGGACGGG GACTTGAATGGATTGGATGGATTTATCCTGGAGATGTTAATACGA AGTACAATGAGAAATTCAAGGGCAAGGCCACACTGACTGCAGA CAAAACCTCCAGCACAGCCTACATGCAGCTCAGCAGCCTGACC |
| | | TCTGAGGACTCTGCGGTCTATTTCTGTGCAAGAGAAGACCCCGG TAGTAACTACTTTGACTACTGGGGCCAAGGCACCACTCTCACAG TCTCCTCA |
| SEQ ID NO:4 | The nucleotide sequence encoding the heavy chain variable region of murine antibody EGFR-mhPA8 | CAGGTGCAGCTGCAGCAATCTGGACCTGATTTGGTGAAGCCTG GGGCTTCAGTGAGGATATCTTGCAAGGTTTCTGGCTACACCTTC ACAACCTACTATACACACTGGGTGAAGCAGAGGCCTGGACGGG GACTTGAATGGATTGGATGGATTTATCCTGGAGATGTTAATACGA AGTACAATGAGAAATTCAAGGGCAAGGCCACACTGACTGCAGA CAAAACCTCCAGCACAGCCTACATGCAGCTCAGCAGCCTGACC TCTGAGGACTCTGCGGTCTATTTCTGTGCAAGAGAAGACCCCGG TAGTAACTACTTTGACTACTGGGGCCAAGGCACCACTCTCACAG TCTCCTCA |
| SEQ ID NO:5 | The nucleotide sequence encoding the light chain variable region of murine antibody EGFR-mhPA8 | GATATCCAGATGACCCAGTCTCCAGCCTCCCTGGCTGCATCTGT GGGAGAAACTGTCACCATCACATGTCGAGCAAGTGAGAACATT TACTACAGTTTAGCTTGGTATCAGCAGAAGGAAGGGAAATCTCC TCAGCTCCTGATCTATATTACAAACGGCTTGGCAGATGGTGTCCC ATCGAGGTTCAGTGGCAGTGGATCTGGGACACAGTATTCTATGA AGATCGACAGCATGCAGCCTGAAGATACCGCAACTTATTTCTGT AAACAGTCTTATGACGTTCCGCTCACGTTCGGTGCTGGGACCAA GCTGGAGATGAAA |
| SEQ ID NO:6 | Nucleotide sequence of human IgG1 heavy chain constant region | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGTAAATGA |
| SEQ ID NO:7 | Nucleotide sequence of human kappa light chain constant region | CGTACGGTGGCTGCACCATCTGTCTTCATCTTCCCGCCAT CTGATGAGCAGTTGAAATCTGGAACTGCCTCTGTTGTGTGCCTG CTGAATAACTTCTATCCCAGAGAGGCCAAAGTACAGTGGAAGG TGGATAACGCCCTCCAATCGGGTAACTCCCAGGAGAGTGTCACA GAGCAGGACAGCAAGGACAGCACCTACAGCCTCAGCAGCACC CTGACGCTGAGCAAAGCAGACTACGAGAAACACAAAGTCTACG CCTGCGAAGTCACCCATCAGGGCCTGAGCTCGCCCGTCACAAA GAGCTTCAACAGGGGAGAGTGTTAG |
| SEQ ID NO:8 | The amino acid sequence of the heavy chain variable region of murine antibody EGFR-mhPA8 | QVQLQQSGPDLVKPGASVRISCKVSGYTFTTYYTHWVKQRPGRG LEWIGWIYPGDVNTKYNEKFKGKATLTADKTSSTAYMQLSSLTSE DSAVYFCAREDPGSNYFDYWGQGTTLTVSS |
| SEQ ID NO:9 | The amino acid sequence of the light chain variable region of murine antibody EGFR-mhPA8 | DIQMTQSPASLAASVGETVTITCRASENIYYSLAWYQQKEGKSPQL LIYITNGLADGVPSRFSGSGSGTQYSMKIDSMQPEDTATYFCKQSY DVPLTFGAGTKLEMK |
| SEQ ID NO:10 | The amino acid of sequence murine antibody EGFR-mhPA8 light chain CDR1 | RASENIYYSLA |
| SEQ ID NO:11 | The amino acid sequence of murine antibody EGFR-mhPA8 light chain CDR2 | ITNGLAD |
| SEQ ID NO:12 | The amino acid of sequence murine antibody EGFR-mhPA8 light chain CDR3 | KQSYDVPLT |
| SEQ ID NO:13 | The amino acid of sequence murine antibody EGFR-mhPA8 heavy chain CDR1 | GYTFTTYYTH |
| SEQ ID NO:14 | The amino acid of sequence murine antibody EGFR-mhPA8 heavy chain CDR2 | WIYPGDVNTKYNEKFKG |
| SEQ ID NO:15 | The amino acid of sequence murine antibody EGFR-mhPA8 heavy chain CDR3 | AREDPGSNYFDY |
| SEQ ID NO:16 | The amino acid sequence of humanized antibody EGFR-HPA8 light chain CDR1 | RASENIYYSLA |
| SEQ ID NO:17 | The amino acid sequence of humanized antibody EGFR-HPA8 light chain CDR2 | ITDGLAD |
| SEQ ID NO:18 | The amino acid sequence of humanized antibody EGFR-HPA8 light chain CDR3 | KQSYDVPLT |
| SEQ ID NO:19 | The amino acid sequence of humanized antibody EGFR-HPA8 heavy chain CDR1 | GYTFTTYYTH |
| SEQ ID NO:20 | The amino acid sequence of humanized antibody EGFR-HPA8 heavy chain CDR2 | WIYPGDVNTKYNEKFKG |
| SEQ ID NO:21 | The amino acid sequence of humanized antibody EGFR-HPA8 heavy chain CDR3 | AREDPGSNYFDY |
| SEQ ID NO:22 | The amino acid sequence of humanized antibody EGFR-HPA8 heavy chain | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:30): |
| | | |
| SEQ ID NO:23 | The amino acid sequence of humanized antibody EGFR-HPA8 light chain | The amino acid sequence of the light chain variable region (SEQ ID NO:29): |
| | | DIQMTQSPSSLSASVGDRVTITCRASENIYYSLAWYQQKPGKAPQL LIYITDGLADGVPSRFSGSGSGTDYTLKIDSLQPEDFATYYCKQSY DVPLTFGGGTKVEIK |
| | | The amino acid sequence of the light chain constant regio (SEQ ID NO:31): |
| | | |
| SEQ ID NO:24 | The amino acid sequence of humanized antibody EGFR-HPA8 heavy chain containing the signal peptide | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:30): |
| | | |
| SEQ ID NO:25 | The amino acid sequence of humanized antibody EGFR-HPA8 light chain containing a signal peptide | The amino acid sequence of the light chain signal peptide (SEQ ID NO:27): |
| | | MGWSCIILFLVATATGVHS |
| | | The amino acid sequence of the light chain variable region (SEQ ID NO:29): |
| | | DIQMTQSPSSLSASVGDRVTITCRASENIYYSLAWYQQKPGKAPQL LIYITDGLADGVPSRFSGSGSGTDYTLKIDSLQPEDFATYYCKQSY DVPLTFGGGTKVEIK |
| | | The amino acid sequence of the light chain constant region (SEQ ID NO:31): |
| | | |
| SEQ ID NO:26 | The amino acid sequence of humanized antibodyEGFR-HPA8 heavy chain signal peptide | MGWSLILLFLVAVATRVLS |
| SEQ ID NO:27 | The amino acid sequence of humanized antibody EGFR-HPA8light chain signal peptide | MGWSCIILFLVATATGVHS |
| SEQ ID NO:28 | The amino acid sequence of the humanized antibody EGFR-HPA8 heavy chain variable region | EVQLVQSGAEVKKPGATVKISCKVSGYTFTTYYTHWVKQAPGKG LEWIGWIYPGDVNTKYNEKFKGRVTLTADTSTDTAYMELSSLRSE |
| | | [?] DTAVYYCAREDPGSNYFDYWGQGTLVTVSS 1 |
| SEQ ID NO:29 | The amino acid sequence of the humanized antibody EGFR-HPA8 light chain variable region | DIQMTQSPSSLSASVGDRVTITCRASENIYYSLAWYQQKPGKAPQL LIYITDGLADGVPSRFSGSGSGTDYTLKIDSLQPEDFATYYCKQSY DVPLTFGGGTKVEIK |
| SEQ ID NO:30 | The amino acid sequence of the humanized antibody EGFR-HPA8heavy chain constant region | |
| SEQ ID NO:31 | The amino acid sequence of the humanized antibody EGFR-HPA8light chain constant region | e  |
| SEQ ID NO:32 | The nucleotide sequence encoding humanized antibody EGFR-HPA8 heavy chain containing a signal peptide | The nucleotide sequence encoding the heavy chain signal peptide (SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain varia ble region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:38): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT |
| | | CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGTAAATGA |
| SEQ ID NO:33 | The nucleotide sequence encoding humanized antibody EGFR-HPA8 light chain containing a signal peptide | The nucleotide sequence encoding the light chain signal peptide (SEQ ID NO:35): |
| | | ATGGGCTGGTCCTGTATCATCCTGTTCCTGGTGGCTACAGCCAC AGGAGTGCATAGT |
| | | The nucleotide sequence encoding the light chain variable region (SEQ ID NO:37): |
| | | GACATCCAGATGACCCAGAGCCCATCCTCCCTGTCTGCCTCTGT GGGAGACAGGGTGACCATCACTTGTAGGGCATCTGAGAACATC TACTACTCCCTGGCTTGGTATCAACAGAAGCCTGGCAAGGCTCC ACAGCTGCTGATTTACATCACCGACGGACTGGCTGATGGAGTGC CAAGCAGGTTCTCTGGCTCTGGCTCTGGCACAGACTACACCCTG AAGATCGACTCCCTCCAACCTGAGGACTTTGCCACCTACTACTG TAAGCAGTCCTATGATGTGCCACTGACCTTTGGAGGAGGCACCA AGGTGGAGATTAAG |
| | | The nucleotide sequence encoding the light chain constant region (SEQ ID NO:39): |
| | | CGTACGGTGGCTGCACCATCTGTCTTCATCTTCCCGCCATCTGAT GAGCAGTTGAAATCTGGAACTGCCTCTGTTGTGTGCCTGCTGAA TAACTTCTATCCCAGAGAGGCCAAAGTACAGTGGAAGGTGGATA ACGCCCTCCAATCGGGTAACTCCCAGGAGAGTGTCACAGAGCA GGACAGCAAGGACAGCACCTACAGCCTCAGCAGCACCCTGACG CTGAGCAAAGCAGACTACGAGAAACACAAAGTCTACGCCTGCG AAGTCACCCATCAGGGCCTGAGCTCGCCCGTCACAAAGAGCTT CAACAGGGGAGAGTGTTGA |
| SEQ ID NO:34 | The nucleotide sequence encoding humanized antibody EGFR-HPA8 heavy chain signal peptide | ATGGGCTGGTCCCTGATTCTGCTGTTCCTGGTGGCTGTGGCTAC CAGGGTGCTGAGT |
| SEQ ID NO:35 | The nucleotide sequence encoding humanized antibody EGFR-HPA8 light chain signal peptide | ATGGGCTGGTCCTGTATCATCCTGTTCCTGGTGGCTACAGCCAC AGGAGTGCATAGT |
| SEQ ID NO:36 | The nucleotide sequence encoding humanized antibody EGFR-HPA8 heavy chain variable region | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| SEQ ID NO:37 | The nucleotide sequence encoding humanized antibody EGFR-HPA8light chain variable region | GACATCCAGATGACCCAGAGCCCATCCTCCCTGTCTGCCTCTGT GGGAGACAGGGTGACCATCACTTGTAGGGCATCTGAGAACATC TACTACTCCCTGGCTTGGTATCAACAGAAGCCTGGCAAGGCTCC ACAGCTGCTGATTTACATCACCGACGGACTGGCTGATGGAGTGC CAAGCAGGTTCTCTGGCTCTGGCTCTGGCACAGACTACACCCTG AAGATCGACTCCCTCCAACCTGAGGACTTTGCCACCTACTACTG TAAGCAGTCCTATGATGTGCCACTGACCTTTGGAGGAGGCACCA AGGTGGAGATTAAG |
| SEQ ID NO:38 | The nucleotide sequence encoding humanized antibody EGFR-HPA8 heavy chain constant region | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA |
| | | AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGTAAATGA |
| SEQ ID NO:39 | The nucleotide sequence encoding humanized antibody EGFR-HPA8 light chain constant region | [?] CGTACGGTGGCTGCACCATCTGTCTTCATCTTCCCGCCATCTGAT [?] GAGCAGTTGAAATCTGGAACTGCCTCTGTTGTGTGCCTGCTGAA TAACTTCTATCCCAGAGAGGCCAAAGTACAGTGGAAGGTGGATA ACGCCCTCCAATCGGGTAACTCCCAGGAGAGTGTCACAGAGCA GGACAGCAAGGACAGCACCTACAGCCTCAGCAGCACCCTGACG CTGAGCAAAGCAGACTACGAGAAACACAAAGTCTACGCCTGCG AAGTCACCCATCAGGGCCTGAGCTCGCCCGTCACAAAGAGCTT CAACAGGGGAGAGTGTTGA |
| SEQ ID NO:40 | The amino acid sequence of murine antibody scFv used in the construction of antibody EGFR-mhPA8 | The amino acid sequence of the EGFR-mhPA8 light chain variable region (SEQ ID NO:9): |
| | | DIQMTQSPASLAASVGETVTITCRASENIYYSLAWYQQKEGKSPQL LIYITNGLADGVPSRFSGSGSGTQYSMKIDSMQPEDTATYFCKQSY DVPLTFGAGTKLEMK |
| | | Linker (SEQ ID NO:41): |
| | | SSGGGGSGGGGGGSSRSS |
| | | The amino acid sequence of the EGFR-mhPA8 heavy chain variable region (SEQ ID NO:8): |
| | | QVQLQQSGPDLVKPGASVRISCKVSGYTFTTYYTHWVKQRPGRG LEWIGWIYPGDVNTKYNEKFKGKATLTADKTSSTAYMQLSSLTSE DSAVYFCAREDPGSNYFDYWGQGTTLTVSS |
| SEQ ID NO:41 | The amino acid sequence of the linker used in the construction of the phage antibody library for the linkage of the murine antibody scFv | SSGGGGSGGGGGGSSRSS |
| SEQ ID NO:42 | The nucleotide sequence encoding human-mouse chimeric antibody EGFR-mhPA8 heavy chain containing the signal peptide | The nucleotide sequence encoding the heavy chain signal peptide (SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region (SEQ ID NO:4): |
| | | CAGGTGCAGCTGCAGCAATCTGGACCTGATTTGGTGAAGCCTG GGGCTTCAGTGAGGATATCTTGCAAGGTTTCTGGCTACACCTTC ACAACCTACTATACACACTGGGTGAAGCAGAGGCCTGGACGGG GACTTGAATGGATTGGATGGATTTATCCTGGAGATGTTAATACGA |
| | | AGTACAATGAGAAATTCAAGGGCAAGGCCACACTGACTGCAGA CAAAACCTCCAGCACAGCCTACATGCAGCTCAGCAGCCTGACC TCTGAGGACTCTGCGGTCTATTTCTGTGCAAGAGAAGACCCCGG TAGTAACTACTTTGACTACTGGGGCCAAGGCACCACTCTCACAG TCTCCTCA |
| | | The nucleotide sequence encoding the heavy chain constant region (SEQ ID NO:6): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGTAAATGA |
| SEQ ID NO:43 | The nucleotide sequence encoding human-mouse chimeric antibody EGFR-mhPA8 light chain containing the signal peptide | The nucleotide sequence encoding the light chain signal peptide (SEQ ID NO:35): |
| | | ATGGGCTGGTCCTGTATCATCCTGTTCCTGGTGGCTACAGCCAC AGGAGTGCATAGT |
| | | The nucleotide sequence encoding the light chain variable region (SEQ ID NO:5): |
| | | GATATCCAGATGACCCAGTCTCCAGCCTCCCTGGCTGCATCTGT GGGAGAAACTGTCACCATCACATGTCGAGCAAGTGAGAACATT TACTACAGTTTAGCTTGGTATCAGCAGAAGGAAGGGAAATCTCC TCAGCTCCTGATCTATATTACAAACGGCTTGGCAGATGGTGTCCC ATCGAGGTTCAGTGGCAGTGGATCTGGGACACAGTATTCTATGA AGATCGACAGCATGCAGCCTGAAGATACCGCAACTTATTTCTGT |
| | | AAACAGTCTTATGACGTTCCGCTCACGTTCGGTGCTGGGACCAA GCTGGAGATGAAA |
| | | The nucleotide sequence encoding the light chain constant region (SEQ ID NO:7): |
| | | CGTACGGTGGCTGCACCATCTGTCTTCATCTTCCCGCCATCTGAT GAGCAGTTGAAATCTGGAACTGCCTCTGTTGTGTGCCTGCTGAA TAACTTCTATCCCAGAGAGGCCAAAGTACAGTGGAAGGTGGATA ACGCCCTCCAATCGGGTAACTCCCAGGAGAGTGTCACAGAGCA GGACAGCAAGGACAGCACCTACAGCCTCAGCAGCACCCTGACG CTGAGCAAAGCAGACTACGAGAAACACAAAGTCTACGCCTGCG AAGTCACCCATCAGGGCCTGAGCTCGCCCGTCACAAAGAGCTT CAACAGGGGAGAGTGTTAG |
| SEQ ID NO:44 | The amino acid sequence of human-mouse chimeric antibody EGFR-mhPA8 heavy chain containing the signal peptide | The amino acid sequence of the heavy chain signal peptide (SEQ ID NO:26): |
| | | MGWSLILLFLVAVATRVLS |
| | | The amino acid sequence of the heavy chain variable region (SEQ ID NO:8): |
| | | QVQLQQSGPDLVKPGASVRISCKVSGYTFTTYYTHWVKQRPGRG LEWIGWIYPGDVNTKYNEKFKGKATLTADKTSSTAYMQLSSLTSE DSAVYFCAREDPGSNYFDYWGQGTTLTVSS |
| | | The amino acid sequence of the heavy chain constant region (SEQ ID NO:30): |
| | | |
| SEQ ID NO:45 | The amino acid sequence of human-mouse chimeric antibody EGFR-mhPA8 light chain containing the signal peptide | The amino acid sequence of the light chain signal peptide (SEQ ID NO:27): |
| | | MGWSCIILFLVATATGVHS |
| | | The amino acid sequence of the light chain variable region (SEQ ID NO:9): |
| | | DIQMTQSPASLAASVGETVTITCRASENIYYSLAWYQQKEGKSPQL LIYITNGLADGVPSRFSGSGSGTQYSMKIDSMQPEDTATYFCKQSY DVPLTFGAGTKLEMK |
| | | The amino acid sequence of the light chain constant region (SEQ ID NO:31): |
| | | |
| SEQ ID NO:46 | The amino acid sequence of humanized antibody EGFR-HPA8 heavy chain constant region with C-erminal lysine deletion | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| SEQ ID NO:47 | The amino acid sequence of the full-length TGFβR2 extracellular domain | IPPHVQKSVNNDMIVTDNNGAVKFPQLCKFCDVRFSTCDNQKSC MSNCSITSICEKPQEVCVAVWRKNDENITLETVCHDPKLPYHDFIL EDAASPKCIMKEKKKPGETFFMCSCSSDECNDNIIFSEEYNTSNPD |
| SEQ ID NO:48 | The amino acid sequence of truncated TGFβR2 extracellular domain with a deletion of N-terminal amino acids at position (6-16) | IPPHVNNGAVKFPQLCKFCDVRFSTCDNQKSCMSNCSITSICEKPQ EVCVAVWRKNDENITLETVCHDPKLPYHDFILEDAASPKCIMKEK KKPGETFFMCSCSSDECNDNIIFSEEYNTSNPD |
| SEQ ID NO:49 | The amino acid sequence of truncated TGFβR2 extracellular domain with a deletion of N-terminal amino acids at position (6-19) | IPPHVGAVKFPQLCKFCDVRFSTCDNQKSCMSNCSITSICEKPQEV CVAVWRKNDENITLETVCHDPKLPYHDFILEDAASPKCIMKEKKK PGETFFMCSCSSDECNDNIIFSEEYNTSNPD |
| SEQ ID NO:50 | The amino acid sequence of truncated TGFβR2 extracellular domain with a deletion of N-terminal amino acids at position (6-21) | IPPHVVKFPQLCKFCDVRFSTCDNQKSCMSNCSITSICEKPQEVCV AVWRKNDENITLETVCHDPKLPYHDFILEDAASPKCIMKEKKKPG ETFFMCSCSSDECNDNIIFSEEYNTSNPD |
| SEQ ID NO:51 | The amino acid sequence of truncated TGFβR2 extracellular domain with a deletion of N-terminal amino acids at position (6-25) | IPPHVQLCKFCDVRFSTCDNQKSCMSNCSITSICEKPQEVCVAVWR KNDENITLETVCHDPKLPYHDFILEDAASPKCIMKEKKKPGETFFM CSCSSDECNDNIIFSEEYNTSNPD |
| SEQ ID NO:52 | The amino acid sequence of truncated TGFβR2 extracellular domain with a deletion of N-terminal amino acids at position (6-26) | IPPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:53 | The amino acid sequence of truncated TGFβR2 extracellular domain with a deletion of N-terminal amino acids at position (6-27) | IPPHVCKFCDVRFSTCDNQKSCMSNCSITSICEKPQEVCVAVWRKN DENITLETVCHDPKLPYHDFILEDAASPKCIMKEKKKPGETFFMCS CSSDECNDNIIFSEEYNTSNPD |
| SEQ ID NO:54 | The amino acid sequence of truncated TGFβR2 extracellular domain with a deletion of N-terminal amino acids at position (7-26) | IPPHVQLCKFCDVRFSTCDNQKSCMSNCSITSICEKPQEVCVAVWR KNDENITLETVCHDPKLPYHDFILEDAASPKCIMKEKKKPGETFFM CSCSSDECNDNIIFSEEYNTSNPD |
| SEQ ID NO:55 | The amino acid sequence of truncated TGFβR2 extracellular domain with a deletion of N-terminal amino acids at position (5-26) | IPPHLCKFCDVRFSTCDNQKSCMSNCSITSICEKPQEVCVAVWRKN DENITLETVCHDPKLPYHDFILEDAASPKCIMKEKKKPGETFFMCS CSSDECNDNIIFSEEYNTSNPD |
| SEQ ID NO:56 | The amino acid sequence of truncated TGFβR2 extracellular domain with a ieletion of N-terminal amino acids at position (4-26) | IPPLCKFCDVRFSTCDNQKSCMSNCSITSICEKPQEVCVAVWRKND ENITLETVCHDPKLPYHDFILEDAASPKCIMKEKKKPGETFFMCSC SSDECNDNIIFSEEYNTSNPD |
| SEQ ID NO:57 | The amino acid sequence of truncated TGFβR2 extracellular domain with a deletion of N-terminal amino acids at position (3-26) | IPLCKFCDVRFSTCDNQKSCMSNCSITSICEKPQEVCVAVWRKNDE NITLETVCHDPKLPYHDFILEDAASPKCIMKEKKKPGETFFMCSCS SDECNDNIIFSEEYNTSNPD |
| SEQ ID NO:58 | The amino acid sequence of truncated TGFβR2 extracellular domain with a deletion of N-terminal amino acids at position (2-26) | ILCKFCDVRFSTCDNQKSCMSNCSITSICEKPQEVCVAVWRKNDEN ITLETVCHDPKLPYHDFILEDAASPKCIMKEKKKPGETFFMCSCSS DECNDNIIFSEEYNTSNPD |
| SEQ ID NO:59 | The amino acid sequence of truncated TGFβR2 extracellular domain with a deletion of N-terminal amino acids at position (1,6-26) | PPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:60 | The amino acid sequence of truncated TGFβR2 extracellular domain with a deletion of N-terminal amino acids at position (1-2,6-26) | PHVLCKFCDVRFSTCDNQKSCMSNCSITSICEKPQEVCVAVWRKN DENITLETVCHDPKLPYHDFILEDAASPKCIMKEKKKPGETFFMCS CSSDECNDNIIFSEEYNTSNPD |
| SEQ ID NO:61 | The amino acid sequence of truncated TGFβR2 extracellular domain with a deletion of N-terminal amino acids at position (1-3,6-26) | HVLCKFCDVRFSTCDNQKSCMSNCSITSICEKPQEVCVAVWRKND ENITLETVCHDPKLPYHDFILEDAASPKCIMKEKKKPGETFFMCSC SSDECNDNIIFSEEYNTSNPD |
| SEQ ID NO:62 | The amino acid sequence of truncated TGFβR2 extracellular domain with a deletion of N-terminal amino acids at position (1-4,6-26) | VLCKFCDVRFSTCDNQKSCMSNCSITSICEKPQEVCVAVWRKNDE NITLETVCHDPKLPYHDFILEDAASPKCIMKEKKKPGETFFMCSCS SDECNDNIIFSEEYNTSNPD |
| SEQ ID NO:63 | The amino acid sequence of truncated TGFβR2 extracellular domain with a deletion of N-terminal amino acids at position (1-19) | GAVKFPQLCKFCDVRFSTCDNQKSCMSNCSITSICEKPQEVCVAVW RKNDENITLETVCHDPKLPYHDFILEDAASPKCIMKEKKKPGETFF MCSCSSDECNDNIIFSEEYNTSNPD |
| SEQ ID NO:64 | The amino acid sequence of truncated TGFβR2 extracellular domain with a deletion of N-terminal amino acids at position (1-21) | VKFPQLCKFCDVRFSTCDNQKSCMSNCSITSICEKPQEVCVAVWR KNDENITLETVCHDPKLPYHDFILEDAASPKCIMKEKKKPGETFFM CSCSSDECNDNIIFSEEYNTSNPD |
| SEQ ID NO:65 | The amino acid sequence of truncated TGFβR2 extracellular domain with a deletion of N-terminal amino acids at position (1-26) | LCKFCDVRFSTCDNQKSCMSNCSITSICEKPQEVCVAVWRKNDENI TLETVCHDPKLPYHDFILEDAASPKCIMKEKKKPGETFFMCSCSSD ECNDNIIFSEEYNTSNPD |
| SEQ ID NO:66 | The amino acid sequence of Linker linking the heavy chain C-terminal and TGFβR2 extracellular domain in fusion protein | GGGGSGGGGSGGGGSGGGGS |
| SEQ ID NO:67 | The nucleotide sequence encoding humanized antibody EGFR-HPA8 heavy chain constant region with C-erminal lysine deletion | [?] GCAAGCACCAAGGGCCCATCGGTCTTCCCCCTGGCACCCTCCTC [?] CAAGAGCACCTCTGGGGGCACAGCGGCCCTGGGCTGCCTGGTC [?] AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| SEQ ID NO:68 | The nucleotide sequence encoding the full-length extracellular domain of TGFβR2 | ATCCCACCGCACGTTCAGAAGTCGGTTAATAACGACATGATAGT CACTGACAACAACGGTGCAGTCAAGTTTCCACAACTGTGTAAA TTTTGTGATGTGAGATTTTCCACCTGTGACAACCAGAAATCCTG CATGAGCAACTGCAGCATCACCTCCATCTGTGAGAAGCCACAG GAAGTCTGTGTGGCTGTATGGAGAAAGAATGACGAGAACATAA CACTAGAGACAGTTTGCCATGACCCCAAGCTCCCCTACCATGAC TTTATTCTGGAAGATGCTGCTTCTCCAAAGTGCATTATGAAGGA AAAAAAAAAGCCTGGTGAGACTTTCTTCATGTGTTCCTGTAGCT CTGATGAGTGCAATGACAACATCATCTTCTCAGAAGAATATAAC ACCAGCAATCCTGACTAAA |
| SEQ ID NO:69 | The nucleotide sequence encoding runcated TGFβR2 extracellular domain with a deletion of N-terminal amino acids at position (6-16) | ATCCCACCGCACGTTAACAACGGTGCAGTCAAGTTTCCACAACT GTGTAAATTTTGTGATGTGAGATTTTCCACCTGTGACAACCAGA AATCCTGCATGAGCAACTGCAGCATCACCTCCATCTGTGAGAAG CCACAGGAAGTCTGTGTGGCTGTATGGAGAAAGAATGACGAGA ACATAACACTAGAGACAGTTTGCCATGACCCCAAGCTCCCCTAC CATGACTTTATTCTGGAAGATGCTGCTTCTCCAAAGTGCATTATG |
| | | AAGGAAAAAAAAAAGCCTGGTGAGACTTTCTTCATGTGTTCCT GTAGCTCTGATGAGTGCAATGACAACATCATCTTCTCAGAAGAA TATAACACCAGCAATCCTGACTAAA |
| SEQ ID NO:70 | The nucleotide sequence encoding runcated TGFβR2 extracellular domain with a deletion of N-terminal amino acids at position (6-19) | ATCCCACCGCACGTTGGTGCAGTCAAGTTTCCACAACTGTGTAA ATTTTGTGATGTGAGATTTTCCACCTGTGACAACCAGAAATCCT GCATGAGCAACTGCAGCATCACCTCCATCTGTGAGAAGCCACA GGAAGTCTGTGTGGCTGTATGGAGAAAGAATGACGAGAACATA ACACTAGAGACAGTTTGCCATGACCCCAAGCTCCCCTACCATGA CTTTATTCTGGAAGATGCTGCTTCTCCAAAGTGCATTATGAAGGA AAAAAAAAAGCCTGGTGAGACTTTCTTCATGTGTTCCTGTAGCT CTGATGAGTGCAATGACAACATCATCTTCTCAGAAGAATATAAC ACCAGCAATCCTGACTAAA |
| SEQ ID NO:71 | The nucleotide sequence encoding runcated TGFβR2 extracellular domain with a deletion of N-terminal amino acids at position (6-21) | ATCCCACCGCACGTTGTCAAGTTTCCACAACTGTGTAAATTTTG TGATGTGAGATTTTCCACCTGTGACAACCAGAAATCCTGCATGA GCAACTGCAGCATCACCTCCATCTGTGAGAAGCCACAGGAAGT CTGTGTGGCTGTATGGAGAAAGAATGACGAGAACATAACACTA GAGACAGTTTGCCATGACCCCAAGCTCCCCTACCATGACTTTAT TCTGGAAGATGCTGCTTCTCCAAAGTGCATTATGAAGGAAAAAA AAAAGCCTGGTGAGACTTTCTTCATGTGTTCCTGTAGCTCTGAT GAGTGCAATGACAACATCATCTTCTCAGAAGAATATAACACCAG CAATCCTGACTAAA |
| SEQ ID NO:72 | The nucleotide sequence encoding runcated TGFβR2 extracellular domain with a deletion of N-terminal amino acids at position (6-25) | ATCCCACCGCACGTTCAACTGTGTAAATTTTGTGATGTGAGATTT TCCACCTGTGACAACCAGAAATCCTGCATGAGCAACTGCAGCAT CACCTCCATCTGTGAGAAGCCACAGGAAGTCTGTGTGGCTGTAT GGAGAAAGAATGACGAGAACATAACACTAGAGACAGTTTGCCA TGACCCCAAGCTCCCCTACCATGACTTTATTCTGGAAGATGCTG CTTCTCCAAAGTGCATTATGAAGGAAAAAAAAAAGCCTGGTGA GACTTTCTTCATGTGTTCCTGTAGCTCTGATGAGTGCAATGACA ACATCATCTTCTCAGAAGAATATAACACCAGCAATCCTGACTAA A |
| SEQ ID NO:73 | The nucleotide sequence encoding runcated TGFβR2 extracellular domain with a deletion of N-terminal amino acids at position (6-26) | ATCCCACCGCACGTTCTGTGTAAATTTTGTGATGTGAGATTTTCC ACCTGTGACAACCAGAAATCCTGCATGAGCAACTGCAGCATCA CCTCCATCTGTGAGAAGCCACAGGAAGTCTGTGTGGCTGTATGG AGAAAGAATGACGAGAACATAACACTAGAGACAGTTTGCCATG ACCCCAAGCTCCCCTACCATGACTTTATTCTGGAAGATGCTGCTT CTCCAAAGTGCATTATGAAGGAAAAAAAAAAGCCTGGTGAGAC TTTCTTCATGTGTTCCTGTAGCTCTGATGAGTGCAATGACAACAT CATCTTCTCAGAAGAATATAACACCAGCAATCCTGACTAAA |
| SEQ ID NO:74 | The nucleotide sequence encoding runcated TGFβR2 extracellular domain with a deletion of N-terminal amino acids at position (6-27) | ATCCCACCGCACGTTTGTAAATTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC |
| | | CAAAGTGCATTATGAAGGAAAAAAAAAAGCCTGGTGAGACTTT CTTCATGTGTTCCTGTAGCTCTGATGAGTGCAATGACAACATCAT CTTCTCAGAAGAATATAACACCAGCAATCCTGACTAAA |
| SEQ ID NO:75 | The nucleotide sequence encoding runcated TGFβR2 extracellular domain with a deletion of N-terminal amino acids at position (7-26) | ATCCCACCGCACGTTCAGCTGTGTAAATTTTGTGATGTGAGATTT TCCACCTGTGACAACCAGAAATCCTGCATGAGCAACTGCAGCAT CACCTCCATCTGTGAGAAGCCACAGGAAGTCTGTGTGGCTGTAT GGAGAAAGAATGACGAGAACATAACACTAGAGACAGTTTGCCA TGACCCCAAGCTCCCCTACCATGACTTTATTCTGGAAGATGCTG CTTCTCCAAAGTGCATTATGAAGGAAAAAAAAAAGCCTGGTGA GACTTTCTTCATGTGTTCCTGTAGCTCTGATGAGTGCAATGACA ACATCATCTTCTCAGAAGAATATAACACCAGCAATCCTGACTAA A |
| SEQ ID NO:76 | The nucleotide sequence encoding runcated TGFβR2 extracellular domain with a deletion of N-terminal amino acids at position (5-26) | ATCCCACCGCACCTGTGTAAATTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC CAAAGTGCATTATGAAGGAAAAAAAAAAGCCTGGTGAGACTTT CTTCATGTGTTCCTGTAGCTCTGATGAGTGCAATGACAACATCAT CTTCTCAGAAGAATATAACACCAGCAATCCTGACTAAA |
| SEQ ID NO:77 | The nucleotide sequence encoding runcated TGFβR2 extracellular domain with a deletion of N-terminal amino acids at position (4-26) | ATCCCACCGCTGTGTAAATTTTGTGATGTGAGATTTTCCACCTGT GACAACCAGAAATCCTGCATGAGCAACTGCAGCATCACCTCCAT CTGTGAGAAGCCACAGGAAGTCTGTGTGGCTGTATGGAGAAAG AATGACGAGAACATAACACTAGAGACAGTTTGCCATGACCCCA AGCTCCCCTACCATGACTTTATTCTGGAAGATGCTGCTTCTCCAA AGTGCATTATGAAGGAAAAAAAAAAGCCTGGTGAGACTTTCTT CATGTGTTCCTGTAGCTCTGATGAGTGCAATGACAACATCATCTT CTCAGAAGAATATAACACCAGCAATCCTGACTAAA |
| SEQ ID NO:78 | The nucleotide sequence encoding runcated TGFβR2 extracellular domain with a deletion of N-terminal amino acids at position (3-26) | ATCCCACTGTGTAAATTTTGTGATGTGAGATTTTCCACCTGTGAC AACCAGAAATCCTGCATGAGCAACTGCAGCATCACCTCCATCTG TGAGAAGCCACAGGAAGTCTGTGTGGCTGTATGGAGAAAGAAT GACGAGAACATAACACTAGAGACAGTTTGCCATGACCCCAAGC TCCCCTACCATGACTTTATTCTGGAAGATGCTGCTTCTCCAAAGT GCATTATGAAGGAAAAAAAAAAGCCTGGTGAGACTTTCTTCAT GTGTTCCTGTAGCTCTGATGAGTGCAATGACAACATCATCTTCTC AGAAGAATATAACACCAGCAATCCTGACTAAA |
| SEQ ID NO:79 | The nucleotide sequence encoding runcated TGFβR2 extracellular domain with a deletion of N-terminal amino acids at position (2-26) | ATCCTGTGTAAATTTTGTGATGTGAGATTTTCCACCTGTGACAAC CAGAAATCCTGCATGAGCAACTGCAGCATCACCTCCATCTGTGA GAAGCCACAGGAAGTCTGTGTGGCTGTATGGAGAAAGAATGAC GAGAACATAACACTAGAGACAGTTTGCCATGACCCCAAGCTCC CCTACCATGACTTTATTCTGGAAGATGCTGCTTCTCCAAAGTGCA TTATGAAGGAAAAAAAAAAGCCTGGTGAGACTTTCTTCATGTGT TCCTGTAGCTCTGATGAGTGCAATGACAACATCATCTTCTCAGA |
| | | AGAATATAACACCAGCAATCCTGACTAAA |
| SEQ ID NO:80 | The nucleotide sequence encoding runcated TGFβR2 extracellular domain with a deletion of N-terminal amino acids at position (1,6-26) | CCACCGCACGTTCTGTGTAAATTTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC CAAAGTGCATTATGAAGGAAAAAAAAAAGCCTGGTGAGACTTT CTTCATGTGTTCCTGTAGCTCTGATGAGTGCAATGACAACATCAT CTTCTCAGAAGAATATAACACCAGCAATCCTGACTAAA |
| SEQ ID NO:81 | The nucleotide sequence encoding runcated TGFβR2 extracellular domain with a deletion of N-terminal amino acids at position (1-2,6-26) | CCGCACGTTCTGTGTAAATTTTGTGATGTGAGATTTTCCACCTGT GACAACCAGAAATCCTGCATGAGCAACTGCAGCATCACCTCCAT CTGTGAGAAGCCACAGGAAGTCTGTGTGGCTGTATGGAGAAAG AATGACGAGAACATAACACTAGAGACAGTTTGCCATGACCCCA AGCTCCCCTACCATGACTTTATTCTGGAAGATGCTGCTTCTCCAA AGTGCATTATGAAGGAAAAAAAAAAGCCTGGTGAGACTTTCTT CATGTGTTCCTGTAGCTCTGATGAGTGCAATGACAACATCATCTT CTCAGAAGAATATAACACCAGCAATCCTGACTAAA |
| SEQ ID NO:82 | The nucleotide sequence encoding runcated TGFβR2 extracellular domain with a deletion of N-terminal amino acids at position (1-3,6-26) | CACGTTCTGTGTAAATTTTGTGATGTGAGATTTTCCACCTGTGAC AACCAGAAATCCTGCATGAGCAACTGCAGCATCACCTCCATCTG TGAGAAGCCACAGGAAGTCTGTGTGGCTGTATGGAGAAAGAAT GACGAGAACATAACACTAGAGACAGTTTGCCATGACCCCAAGC TCCCCTACCATGACTTTATTCTGGAAGATGCTGCTTCTCCAAAGT GCATTATGAAGGAAAAAAAAAAGCCTGGTGAGACTTTCTTCAT GTGTTCCTGTAGCTCTGATGAGTGCAATGACAACATCATCTTCTC AGAAGAATATAACACCAGCAATCCTGACTAAA |
| SEQ ID NO:83 | The nucleotide sequence encoding truncated TGFβR2 extracellular domain with a deletion of N-terminal amino acids at position (1-4,6-26) | GTTCTGTGTAAATTTTGTGATGTGAGATTTTCCACCTGTG ACAACCAGAAATCCTGCATGAGCAACTGCAGCATCACCTCCATC TGTGAGAAGCCACAGGAAGTCTGTGTGGCTGTATGGAGAAAGA ATGACGAGAACATAACACTAGAGACAGTTTGCCATGACCCCAA GCTCCCCTACCATGACTTTATTCTGGAAGATGCTGCTTCTCCAAA GTGCATTATGAAGGAAAAAAAAAAGCCTGGTGAGACTTTCTTC ATGTGTTCCTGTAGCTCTGATGAGTGCAATGACAACATCATCTTC TCAGAAGAATATAACACCAGCAATCCTGACTAAA |
| SEQ ID NO:84 | The nucleotide sequence encoding truncated TGFβR2 extracellular domain with a deletion of N-terminal amino acids at position (1-19) | [?] GGTGCAGTCAAGTTTCCACAACTGTGTAAATTTTGTGAT [?] GTGAGATTTTCCACCTGTGACAACCAGAAATCCTGCATGAGCAA [?] CTGCAGCATCACCTCCATCTGTGAGAAGCCACAGGAAGTCTGT [?] GTGGCTGTATGGAGAAAGAATGACGAGAACATAACACTAGAGA CAGTTTGCCATGACCCCAAGCTCCCCTACCATGACTTTATTCTGG AAGATGCTGCTTCTCCAAAGTGCATTATGAAGGAAAAAAAAAA GCCTGGTGAGACTTTCTTCATGTGTTCCTGTAGCTCTGATGAGT GCAATGACAACATCATCTTCTCAGAAGAATATAACACCAGCAAT CCTGACTAAA |
| SEQ ID NO:85 | The nucleotide sequence encoding truncated TGFβR2 extracellular domain with a deletion of N-terminal amino acids at position (1-21) | GTCAAGTTTCCACAACTGTGTAAATTTGTGATGTGAGAT |
| | | TTTCCACCTGTGACAACCAGAAATCCTGCATGAGCAACTGCAGC ATCACCTCCATCTGTGAGAAGCCACAGGAAGTCTGTGTGGCTGT ATGGAGAAAGAATGACGAGAACATAACACTAGAGACAGTTTGC CATGACCCCAAGCTCCCCTACCATGACTTTATTCTGGAAGATGCT GCTTCTCCAAAGTGCATTATGAAGGAAAAAAAAAAGCCTGGTG AGACTTTCTTCATGTGTTCCTGTAGCTCTGATGAGTGCAATGAC AACATCATCTTCTCAGAAGAATATAACACCAGCAATCCTGACTA AA |
| SEQ ID NO:86 | The nucleotide sequence encoding runcated TGFβR2 extracellular domain with a deletion of N-terminal amino acids at position (1-26) | CTGTGTAAATTTTGTGATGTGAGATTTTCCACCTGTGACAACCA GAAATCCTGCATGAGCAACTGCAGCATCACCTCCATCTGTGAGA AGCCACAGGAAGTCTGTGTGGCTGTATGGAGAAAGAATGACGA GAACATAACACTAGAGACAGTTTGCCATGACCCCAAGCTCCCCT ACCATGACTTTATTCTGGAAGATGCTGCTTCTCCAAAGTGCATTA TGAAGGAAAAAAAAAAGCCTGGTGAGACTTTCTTCATGTGTTC CTGTAGCTCTGATGAGTGCAATGACAACATCATCTTCTCAGAAG AATATAACACCAGCAATCCTGACTAAA |
| SEQ ID NO:87 | The nucleotide sequence encoding Linker linking the heavy chain C-erminal and TGFβR2 extracellular domain in the fusion protein | GGTGGTGGCGGTTCAGGCGGAGGTGGCTCTGGAGGTGGAGGTT CAGGAGGTGGTGGTTCT |
| SEQ ID NO:88 | The amino acid sequence of the signal peptide bearing heavy chain of fusion protein 1 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:47): |
| | | IPPHVQKSVNNDMIVTDNNGAVKFPQLCKFCDVRFSTCDNQKSC MSNCSITSICEKPQEVCVAVWRKNDENITLETVCHDPKLPYHDFIL EDAASPKCIMKEKKKPGETFFMCSCSSDECNDNIIFSEEYNTSNPD |
| SEQ ID NO:89 | The amino acid sequence of the signal peptide bearing heavy chain of fusion protein 2 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:48): |
| | | IPPHVNNGAVKFPQLCKFCDVRFSTCDNQKSCMSNCSITSICEKPQ EVCVAVWRKNDENITLETVCHDPKLPYHDFILEDAASPKCIMKEK KKPGETFFMCSCSSDECNDNIIFSEEYNTSNPD |
| SEQ ID NO:90 | The amino acid sequence of the signal peptide bearing heavy chain of fusion protein 3 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:49): |
| | | IPPHVGAVKFPQLCKFCDVRFSTCDNQKSCMSNCSITSICEKPQEV CVAVWRKNDENITLETVCHDPKLPYHDFILEDAASPKCIMKEKKK PGETFFMCSCSSDECNDNIIFSEEYNTSNPD |
| SEQ ID NO:91 | The amino acid sequence of the signal peptide bearing heavy chain of fusion protein4 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:50): |
| | | IPPHVVKFPQLCKFCDVRFSTCDNQKSCMSNCSITSICEKPQEVCV AVWRKNDENITLETVCHDPKLPYHDFILEDAASPKCIMKEKKKPG ETFFMCSCSSDECNDNIIFSEEYNTSNPD |
| SEQ ID NO:92 | The amino acid sequence of the signal peptide bearing heavy chain of fusion protein5 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:51): |
| | | IPPHVQLCKFCDVRFSTCDNQKSCMSNCSITSICEKPQEVCVAVWR KNDENITLETVCHDPKLPYHDFILEDAASPKCIMKEKKKPGETFFM CSCSSDECNDNIIFSEEYNTSNPD |
| SEQ ID NO:93 | The amino acid sequence of the signal peptide bearing heavy chain of fusion protein6 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK |
| | | VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:52): |
| | | IPPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:94 | The amino acid sequence of the signal peptide bearing heavy chain of fusion protein7 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:53): |
| | | IPPHVCKFCDVRFSTCDNQKSCMSNCSITSICEKPQEVCVAVWRKN DENITLETVCHDPKLPYHDFILEDAASPKCIMKEKKKPGETFFMCS CSSDECNDNIIFSEEYNTSNPD |
| SEQ ID NO:95 | The amino acid sequence of the signal peptide bearing heavy chain of fusion protein8 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:54): |
| | | IPPHVQLCKFCDVRFSTCDNQKSCMSNCSITSICEKPQEVCVAVWR KNDENITLETVCHDPKLPYHDFILEDAASPKCIMKEKKKPGETFFM CSCSSDECNDNIIFSEEYNTSNPD |
| SEQ ID NO:96 | The amino acid sequence of the signal peptide bearing heavy chain of fusion protein 9 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY |
| | | KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:55): |
| | | IPPHLCKFCDVRFSTCDNQKSCMSNCSITSICEKPQEVCVAVWRKN DENITLETVCHDPKLPYHDFILEDAASPKCIMKEKKKPGETFFMCS CSSDECNDNIIFSEEYNTSNPD |
| SEQ ID NO:97 | The amino acid sequence of the signal peptide bearing heavy chain of fusion protein 10 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:56): |
| | | IPPLCKFCDVRFSTCDNQKSCMSNCSITSICEKPQEVCVAVWRKND ENITLETVCHDPKLPYHDFILEDAASPKCIMKEKKKPGETFFMCSC SSDECNDNIIFSEEYNTSNPD |
| SEQ ID NO:98 | The amino acid sequence of the signal peptide bearing heavy chain of fusion protein 11 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:57): |
| | | IPLCKFCDVRFSTCDNQKSCMSNCSITSICEKPQEVCVAVWRKNDE NITLETVCHDPKLPYHDFILEDAASPKCIMKEKKKPGETFFMCSCS SDECNDNIIFSEEYNTSNPD |
| SEQ ID NO:99 | The amino acid sequence of the signal peptide bearing heavy chain of fusion protein 12 | The amino acid sequence of the heavy chain signal peptide (SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:58): |
| | | ILCKFCDVRFSTCDNQKSCMSNCSITSICEKPQEVCVAVWRKNDEN ITLETVCHDPKLPYHDFILEDAASPKCIMKEKKKPGETFFMCSCSS DECNDNIIFSEEYNTSNPD |
| SEQ ID NO: 100 | The amino acid sequence of the signal peptide bearing heavy chain of fusion protein 13 | The amino acid sequence of the heavy chain signal peptide C SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:59): |
| | | PPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:101 | The amino acid sequence of the signal peptide bearing heavy chain of fusion protein 14 | The amino acid sequence of the heavy chain signal peptide C SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:60): |
| | | PHVLCKFCDVRFSTCDNQKSCMSNCSITSICEKPQEVCVAVWRKN DENITLETVCHDPKLPYHDFILEDAASPKCIMKEKKKPGETFFMCS CSSDECNDNIIFSEEYNTSNPD |
| SEQ ID NO:102 | The amino acid sequence of the signal peptide bearing heavy chain of fusion protein 15 | The amino acid sequence of the heavy chain signal peptide C SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:61): |
| | | HVLCKFCDVRFSTCDNQKSCMSNCSITSICEKPQEVCVAVWRKND ENITLETVCHDPKLPYHDFILEDAASPKCIMKEKKKPGETFFMCSC SSDECNDNIIFSEEYNTSNPD |
| SEQ ID NO:103 | The amino acid sequence of the signal peptide bearing heavy chain of fusion protein 16 | The amino acid sequence of the heavy chain signal peptide C SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:62): |
| | | VLCKFCDVRFSTCDNQKSCMSNCSITSICEKPQEVCVAVWRKNDE NITLETVCHDPKLPYHDFILEDAASPKCIMKEKKKPGETFFMCSCS SDECNDNIIFSEEYNTSNPD |
| SEQ ID NO:104 | The amino acid sequence of the signal peptide bearing heavy chain of fusion protein 17 | The amino acid sequence of the heavy chain signal peptide C SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region C SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:63): |
| | | GAVKFPQLCKFCDVRFSTCDNQKSCMSNCSITSICEKPQEVCVAVW RKNDENITLETVCHDPKLPYHDFILEDAASPKCIMKEKKKPGETFF MCSCSSDECNDNIIFSEEYNTSNPD |
| SEQ ID NO:105 | The amino acid sequence of the signal peptide bearing heavy chain of fusion protein 18 | The amino acid sequence of the heavy chain signal peptide C SEQ ID NO:26): |
| | | MGWSLILLFLVAVATRVLS |
| | | The amino acid sequence of the heavy chain variable region C SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region C SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:64): |
| | | VKFPQLCKFCDVRFSTCDNQKSCMSNCSITSICEKPQEVCVAVWR KNDENITLETVCHDPKLPYHDFILEDAASPKCIMKEKKKPGETFFM CSCSSDECNDNIIFSEEYNTSNPD |
| SEQ ID NO:106 | The amino acid sequence of the signal peptide bearing heavy chain of fusion protein19 | The amino acid sequence of the heavy chain signal peptide C SEQ ID NO:26): |
| | | MGWSLILLFLVAVATRVLS |
| | | The amino acid sequence of the heavy chain variable region (SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region (SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein (SEQ ID NO:66): |
| | | GGGGSGGGGSGGGGSGGGGS |
| | | The amino acid sequence of the full-length TGFβR2 extracellular domain (SEQ ID NO:65): |
| | | LCKFCDVRFSTCDNQKSCMSNCSITSICEKPQEVCVAVWRKNDENI TLETVCHDPKLPYHDFILEDAASPKCIMKEKKKPGETFFMCSCSSD ECNDNIIFSEEYNTSNPD |
| SEQ ID NO:107 | The nucleotide sequence encoding the signal peptide bearing heavy chain of fusion protein 1 | The nucleotide sequence encoding the heavy chain signal peptide (SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG |
| | | TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:68): |
| | | ATCCCACCGCACGTTCAGAAGTCGGTTAATAACGACATGATAGT CACTGACAACAACGGTGCAGTCAAGTTTCCACAACTGTGTAAA TTTTGTGATGTGAGATTTTCCACCTGTGACAACCAGAAATCCTG CATGAGCAACTGCAGCATCACCTCCATCTGTGAGAAGCCACAG GAAGTCTGTGTGGCTGTATGGAGAAAGAATGACGAGAACATAA CACTAGAGACAGTTTGCCATGACCCCAAGCTCCCCTACCATGAC TTTATTCTGGAAGATGCTGCTTCTCCAAAGTGCATTATGAAGGA |
| | | AAAAAAAAAGCCTGGTGAGACTTTCTTCATGTGTTCCTGTAGCT CTGATGAGTGCAATGACAACATCATCTTCTCAGAAGAATATAAC ACCAGCAATCCTGACTAAA |
| SEQ ID NO: 108 | The nucleotide sequence encoding fusion protein the signal peptide bearing heavy chain of fusion protein2 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG |
| | | GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:69): |
| | | ATCCCACCGCACGTTAACAACGGTGCAGTCAAGTTTCCACAACT GTGTAAATTTTGTGATGTGAGATTTTCCACCTGTGACAACCAGA AATCCTGCATGAGCAACTGCAGCATCACCTCCATCTGTGAGAAG CCACAGGAAGTCTGTGTGGCTGTATGGAGAAAGAATGACGAGA ACATAACACTAGAGACAGTTTGCCATGACCCCAAGCTCCCCTAC CATGACTTTATTCTGGAAGATGCTGCTTCTCCAAAGTGCATTATG AAGGAAAAAAAAAAGCCTGGTGAGACTTTCTTCATGTGTTCCT GTAGCTCTGATGAGTGCAATGACAACATCATCTTCTCAGAAGAA TATAACACCAGCAATCCTGACTAAA |
| SEQ ID NO:109 | The nucleotide sequence encoding the signal peptide bearing heavy chain of fusion protein 3 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA |
| | | ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:70): |
| | | ATCCCACCGCACGTTGGTGCAGTCAAGTTTCCACAACTGTGTAA ATTTTGTGATGTGAGATTTTCCACCTGTGACAACCAGAAATCCT GCATGAGCAACTGCAGCATCACCTCCATCTGTGAGAAGCCACA GGAAGTCTGTGTGGCTGTATGGAGAAAGAATGACGAGAACATA ACACTAGAGACAGTTTGCCATGACCCCAAGCTCCCCTACCATGA CTTTATTCTGGAAGATGCTGCTTCTCCAAAGTGCATTATGAAGGA AAAAAAAAAGCCTGGTGAGACTTTCTTCATGTGTTCCTGTAGCT CTGATGAGTGCAATGACAACATCATCTTCTCAGAAGAATATAAC ACCAGCAATCCTGACTAAA |
| SEQ ID NO:110 | The nucleotide sequence encoding the signal peptide bearing heavy chain of fusion protein 4 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC |
| | | AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:71): |
| | | ATCCCACCGCACGTTGTCAAGTTTCCACAACTGTGTAAATTTTG TGATGTGAGATTTTCCACCTGTGACAACCAGAAATCCTGCATGA GCAACTGCAGCATCACCTCCATCTGTGAGAAGCCACAGGAAGT CTGTGTGGCTGTATGGAGAAAGAATGACGAGAACATAACACTA GAGACAGTTTGCCATGACCCCAAGCTCCCCTACCATGACTTTAT |
| | | TCTGGAAGATGCTGCTTCTCCAAAGTGCATTATGAAGGAAAAAA AAAAGCCTGGTGAGACTTTCTTCATGTGTTCCTGTAGCTCTGAT GAGTGCAATGACAACATCATCTTCTCAGAAGAATATAACACCAG CAATCCTGACTAAA |
| SEQ ID NO:111 | The nucleotide sequence encoding the signal peptide bearing heavy chain of fusion protein5 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG |
| | | GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:72): |
| | | ATCCCACCGCACGTTCAACTGTGTAAATTTTGTGATGTGAGATTT TCCACCTGTGACAACCAGAAATCCTGCATGAGCAACTGCAGCAT CACCTCCATCTGTGAGAAGCCACAGGAAGTCTGTGTGGCTGTAT GGAGAAAGAATGACGAGAACATAACACTAGAGACAGTTTGCCA TGACCCCAAGCTCCCCTACCATGACTTTATTCTGGAAGATGCTG CTTCTCCAAAGTGCATTATGAAGGAAAAAAAAAAGCCTGGTGA GACTTTCTTCATGTGTTCCTGTAGCTCTGATGAGTGCAATGACA ACATCATCTTCTCAGAAGAATATAACACCAGCAATCCTGACTAA A |
| SEQ ID NO:112 | The nucleotide sequence encoding the signal peptide bearing heavy chain of fusion protein6 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA |
| | | ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:73): |
| | | ATCCCACCGCACGTTCTGTGTAAATTTTGTGATGTGAGATTTTCC ACCTGTGACAACCAGAAATCCTGCATGAGCAACTGCAGCATCA CCTCCATCTGTGAGAAGCCACAGGAAGTCTGTGTGGCTGTATGG AGAAAGAATGACGAGAACATAACACTAGAGACAGTTTGCCATG ACCCCAAGCTCCCCTACCATGACTTTATTCTGGAAGATGCTGCTT CTCCAAAGTGCATTATGAAGGAAAAAAAAAAGCCTGGTGAGAC TTTCTTCATGTGTTCCTGTAGCTCTGATGAGTGCAATGACAACAT CATCTTCTCAGAAGAATATAACACCAGCAATCCTGACTAAA |
| SEQ ID NO:113 | The nucleotide sequence encoding the signal peptide bearing heavy chain of fusion protein7 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG |
| | | ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:74): |
| | | ATCCCACCGCACGTTTGTAAATTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC CAAAGTGCATTATGAAGGAAAAAAAAAAGCCTGGTGAGACTTT |
| | | CTTCATGTGTTCCTGTAGCTCTGATGAGTGCAATGACAACATCAT CTTCTCAGAAGAATATAACACCAGCAATCCTGACTAAA |
| SEQ ID NO:114 | The nucleotide sequence encoding the signal peptide bearing heavy chain of fusion protein8 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domainof TGFβR2 (SEQ ID NO:75): |
| | | ATCCCACCGCACGTTCAGCTGTGTAAATTTTGTGATGTGAGATTT TCCACCTGTGACAACCAGAAATCCTGCATGAGCAACTGCAGCAT CACCTCCATCTGTGAGAAGCCACAGGAAGTCTGTGTGGCTGTAT GGAGAAAGAATGACGAGAACATAACACTAGAGACAGTTTGCCA TGACCCCAAGCTCCCCTACCATGACTTTATTCTGGAAGATGCTG CTTCTCCAAAGTGCATTATGAAGGAAAAAAAAAAGCCTGGTGA GACTTTCTTCATGTGTTCCTGTAGCTCTGATGAGTGCAATGACA ACATCATCTTCTCAGAAGAATATAACACCAGCAATCCTGACTAA A |
| SEQ ID NO:115 | The nucleotide sequence encoding the signal peptide bearing heavy chain of fusion protein 9 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAA GCCTGGAGCCACAGTGAAGATTTCCTGTAAGGTGTCTGGCTACA CCTTCACCACCTACTACACCCACTGGGTGAAGCAGGCTCCTGGC AAGGGATTGGAGTGGATTGGCTGGATTTACCCTGGAGATGTGAA CACCAAATACAATGAGAAGTTCAAGGGCAGGGTGACCCTGACA GCAGACACCAGCACAGACACAGCCTATATGGAACTGTCCTCCCT GAGGTCTGAGGACACAGCAGTCTACTACTGTGCCAGGGAGGAC CCTGGCAGCAACTACTTTGACTACTGGGGACAAGGCACCCTGG TGACAGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC |
| | | AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:76): |
| | | ATCCCACCGCACCTGTGTAAATTTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC CAAAGTGCATTATGAAGGAAAAAAAAAAGCCTGGTGAGACTTT CTTCATGTGTTCCTGTAGCTCTGATGAGTGCAATGACAACATCAT CTTCTCAGAAGAATATAACACCAGCAATCCTGACTAAA |
| SEQ ID NO:116 | The nucleotide sequence encoding the signal peptide bearing heavy chain of fusion protein10 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG |
| | | GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:77: |
| | | ATCCCACCGCTGTGTAAATTTGTGATGTGAGATTTTCCACCTGT GACAACCAGAAATCCTGCATGAGCAACTGCAGCATCACCTCCAT CTGTGAGAAGCCACAGGAAGTCTGTGTGGCTGTATGGAGAAAG AATGACGAGAACATAACACTAGAGACAGTTTGCCATGACCCCA AGCTCCCCTACCATGACTTTATTCTGGAAGATGCTGCTTCTCCAA AGTGCATTATGAAGGAAAAAAAAAAGCCTGGTGAGACTTTCTT CATGTGTTCCTGTAGCTCTGATGAGTGCAATGACAACATCATCTT CTCAGAAGAATATAACACCAGCAATCCTGACTAAA |
| SEQ ID NO:117 | The nucleotide sequence encoding the signal peptide bearing heavy chain of fusion protein11 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:78): |
| | | ATCCCACTGTGTAAATTTTGTGATGTGAGATTTTCCACCTGTGAC AACCAGAAATCCTGCATGAGCAACTGCAGCATCACCTCCATCTG TGAGAAGCCACAGGAAGTCTGTGTGGCTGTATGGAGAAAGAAT GACGAGAACATAACACTAGAGACAGTTTGCCATGACCCCAAGC TCCCCTACCATGACTTTATTCTGGAAGATGCTGCTTCTCCAAAGT GCATTATGAAGGAAAAAAAAAAGCCTGGTGAGACTTTCTTCAT GTGTTCCTGTAGCTCTGATGAGTGCAATGACAACATCATCTTCTC AGAAGAATATAACACCAGCAATCCTGACTAAA |
| SEQ ID NO:118 | The nucleotide sequence encoding the signal peptide bearing heavy chain of fusion protein12 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT |
| | | GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:79): |
| | | ATCCTGTGTAAATTTTGTGATGTGAGATTTTCCACCTGTGACAAC CAGAAATCCTGCATGAGCAACTGCAGCATCACCTCCATCTGTGA GAAGCCACAGGAAGTCTGTGTGGCTGTATGGAGAAAGAATGAC GAGAACATAACACTAGAGACAGTTTGCCATGACCCCAAGCTCC CCTACCATGACTTTATTCTGGAAGATGCTGCTTCTCCAAAGTGCA TTATGAAGGAAAAAAAAAAGCCTGGTGAGACTTTCTTCATGTGT TCCTGTAGCTCTGATGAGTGCAATGACAACATCATCTTCTCAGA AGAATATAACACCAGCAATCCTGACTAAA |
| SEQ ID NO:119 | The nucleotide sequence encoding the signal peptide bearing heavy chain of fusion protein13 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG |
| | | GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:80): |
| | | CCACCGCACGTTCTGTGTAAATTTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC CAAAGTGCATTATGAAGGAAAAAAAAAAGCCTGGTGAGACTTT CTTCATGTGTTCCTGTAGCTCTGATGAGTGCAATGACAACATCAT CTTCTCAGAAGAATATAACACCAGCAATCCTGACTAAA |
| SEQ ID NO: 120 | The nucleotide sequence encoding the signal peptide bearing heavy chain of fusion protein14 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:81): |
| | | CCGCACGTTCTGTGTAAATTTGTGATGTGAGATTTTCCACCTGT GACAACCAGAAATCCTGCATGAGCAACTGCAGCATCACCTCCAT CTGTGAGAAGCCACAGGAAGTCTGTGTGGCTGTATGGAGAAAG AATGACGAGAACATAACACTAGAGACAGTTTGCCATGACCCCA AGCTCCCCTACCATGACTTTATTCTGGAAGATGCTGCTTCTCCAA AGTGCATTATGAAGGAAAAAAAAAAGCCTGGTGAGACTTTCTT CATGTGTTCCTGTAGCTCTGATGAGTGCAATGACAACATCATCTT CTCAGAAGAATATAACACCAGCAATCCTGACTAAA |
| SEQ ID NO:121 | The nucleotide sequence encoding the signal peptide bearing heavy chain of fusion protein15 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT |
| | | GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:82): |
| | | CACGTTCTGTGTAAATTTTGTGATGTGAGATTTTCCACCTGTGAC AACCAGAAATCCTGCATGAGCAACTGCAGCATCACCTCCATCTG TGAGAAGCCACAGGAAGTCTGTGTGGCTGTATGGAGAAAGAAT GACGAGAACATAACACTAGAGACAGTTTGCCATGACCCCAAGC TCCCCTACCATGACTTTATTCTGGAAGATGCTGCTTCTCCAAAGT GCATTATGAAGGAAAAAAAAAAGCCTGGTGAGACTTTCTTCAT GTGTTCCTGTAGCTCTGATGAGTGCAATGACAACATCATCTTCTC AGAAGAATATAACACCAGCAATCCTGACTAAA |
| SEQ ID NO:122 | The nucleotide sequence encoding the signal peptide bearing heavy chain of fusion protein16 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG |
| | | GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGG AGGTTCAGGAGGTGGTGGTTCT |
| | | The nucleotide sequence encoding the full-length extracellular domain of TGFPR2 (SEQ ID NO:83): |
| | | GTTCTGTGTAAATTTTGTGATGTGAGATTTTCCACCTGTG ACAACCAGAAATCCTGCATGAGCAACTGCAGCATCACCTCCATC TGTGAGAAGCCACAGGAAGTCTGTGTGGCTGTATGGAGAAAGA ATGACGAGAACATAACACTAGAGACAGTTTGCCATGACCCCAA GCTCCCCTACCATGACTTTATTCTGGAAGATGCTGCTTCTCCAAA GTGCATTATGAAGGAAAAAAAAAAGCCTGGTGAGACTTTCTTC ATGTGTTCCTGTAGCTCTGATGAGTGCAATGACAACATCATCTTC TCAGAAGAATATAACACCAGCAATCCTGACTAAA |
| SEQ ID NO: 123 | The nucleotide sequence encoding the signal peptide bearing heavy chain of fusion protein17 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:84): |
| | | GGTGCAGTCAAGTTTCCACAACTGTGTAAATTTTGTGATGTGAG ATTTTCCACCTGTGACAACCAGAAATCCTGCATGAGCAACTGCA GCATCACCTCCATCTGTGAGAAGCCACAGGAAGTCTGTGTGGCT GTATGGAGAAAGAATGACGAGAACATAACACTAGAGACAGTTT GCCATGACCCCAAGCTCCCCTACCATGACTTTATTCTGGAAGAT GCTGCTTCTCCAAAGTGCATTATGAAGGAAAAAAAAAAGCCTG GTGAGACTTTCTTCATGTGTTCCTGTAGCTCTGATGAGTGCAATG ACAACATCATCTTCTCAGAAGAATATAACACCAGCAATCCTGAC TAAA |
| SEQ ID NO:124 | The nucleotide sequence encoding the signal peptide bearing heavy chain of fusion protein18 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region C SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC |
| | | AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:85): |
| | | GTCAAGTTTCCACAACTGTGTAAATTTTGTGATGTGAGATTTTCC ACCTGTGACAACCAGAAATCCTGCATGAGCAACTGCAGCATCA CCTCCATCTGTGAGAAGCCACAGGAAGTCTGTGTGGCTGTATGG AGAAAGAATGACGAGAACATAACACTAGAGACAGTTTGCCATG ACCCCAAGCTCCCCTACCATGACTTTATTCTGGAAGATGCTGCTT CTCCAAAGTGCATTATGAAGGAAAAAAAAAAGCCTGGTGAGAC TTTCTTCATGTGTTCCTGTAGCTCTGATGAGTGCAATGACAACAT CATCTTCTCAGAAGAATATAACACCAGCAATCCTGACTAAA |
| SEQ ID NO: 125 | The nucleotide sequence encoding the signal peptide bearing heavy chain of fusion protein19 | The nucleotide sequence encoding the heavy chain signal peptide C SEQ ID NO:34): |
| | | ATGGGCTGGTCCCTGATTCTGCTGTTCCTGGTGGCTGTGGCTAC CAGGGTGCTGAGT |
| | | The nucleotide sequence encoding the heavy chain variable region C SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG |
| | | GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:86): |
| | | CTGTGTAAATTTTGTGATGTGAGATTTTCCACCTGTGACAACCA GAAATCCTGCATGAGCAACTGCAGCATCACCTCCATCTGTGAGA AGCCACAGGAAGTCTGTGTGGCTGTATGGAGAAAGAATGACGA GAACATAACACTAGAGACAGTTTGCCATGACCCCAAGCTCCCCT ACCATGACTTTATTCTGGAAGATGCTGCTTCTCCAAAGTGCATTA TGAAGGAAAAAAAAAAGCCTGGTGAGACTTTCTTCATGTGTTC CTGTAGCTCTGATGAGTGCAATGACAACATCATCTTCTCAGAAG AATATAACACCAGCAATCCTGACTAAA |
| SEQ ID NO:126 | The amino acid sequence of the signal peptide bearing light chain of fusion protein 20,21 | The amino acid sequence of the light chain signal peptide: |
| | | MGWSLILLFLVAVATRVLS |
| | | The amino acid sequence of the light chain variable region: |
| | | |
| | | The amino acid sequence of the light chain constant region: |
| | | |
| SEQ ID NO:127 | The amino acid sequence of the signal peptide bearing heavy chain of fusion protein 20 | The amino acid sequence of the heavy chain signal peptide: |
| | | MGWSLILLFLVAVATRVLS |
| | | The amino acid sequence of the heavy chain variable region: |
| | | |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:47): |
| | | IPPHVQKSVNNDMIVTDNNGAVKFPQLCKFCDVRFSTCDNQKSC MSNCSITSICEKPQEVCVAVWRKNDENITLETVCHDPKLPYHDFIL EDAASPKCIMKEKKKPGETFFMCSCSSDECNDNIIFSEEYNTSNPD |
| SEQ ID NO:128 | The amino acid sequence of the signal peptide bearing heavy chain of fusion protein21 | The amino acid sequence of the heavy chain signal peptide: |
| | | MGWSLILLFLVAVATRVLS |
| | | The amino acid sequence of the heavy chain variable region: |
| | | |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:52): |
| | | IPPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:129 | The amino acid sequence of the signal peptide bearing light chain of fusion protein 22,23 | The amino acid sequence of the light chain signal peptide: |
| | | MGWSLILLFLVAVATRVLS |
| | | The amino acid sequence of the light chain variable region: |
| | | |
| | | The amino acid sequence of the light chain constant region: |
| | | |
| SEQ ID NO:130 | The amino acid sequence of the signal peptide bearing heavy chain of fusion protein22 | The amino acid sequence of the heavy chain signal peptide: |
| | | MGWSLILLFLVAVATRVLS |
| | | The amino acid sequence of the heavy chain variable region: |
| | | |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:47): |
| | | IPPHVQKSVNNDMIVTDNNGAVKFPQLCKFCDVRFSTCDNQKSC MSNCSITSICEKPQEVCVAVWRKNDENITLETVCHDPKLPYHDFIL EDAASPKCIMKEKKKPGETFFMCSCSSDECNDNIIFSEEYNTSNPD |
| SEQ ID NO:131 | The amino acid sequence of the signal peptide bearing heavy chain of fusion protein23 | The amino acid sequence of the heavy chain signal peptide: |
| | | MGWSLILLFLVAVATRVLS |
| | | The amino acid sequence of the heavy chain variable region: |
| | | |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:52): |
| | | IPPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:132 | The amino acid sequence of the signal peptide bearing light chain of fusion protein 24,25 | The amino acid sequence of the light chain signal peptide: |
| | | MGWSLILLFLVAVATRVLS |
| | | The amino acid sequence of the light chain variable region: |
| | | |
| | | The amino acid sequence of the light chain constant region: |
| | | |
| SEQ ID NO:133 | The amino acid sequence of the signal peptide bearing heavy chain of fusion protein24 | The amino acid sequence of the heavy chain signal peptide: |
| | | MGWSLILLFLVAVATRVLS |
| | | The amino acid sequence of the heavy chain variable region: |
| | | |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:47): |
| | | IPPHVQKSVNNDMIVTDNNGAVKFPQLCKFCDVRFSTCDNQKSC MSNCSITSICEKPQEVCVAVWRKNDENITLETVCHDPKLPYHDFIL EDAASPKCIMKEKKKPGETFFMCSCSSDECNDNIIFSEEYNTSNPD |
| SEQ ID NO:134 | The amino acid sequence of the signal peptide bearing heavy chain of fusion protein25 | The amino acid sequence of the heavy chain signal peptide: |
| | | MGWSLILLFLVAVATRVLS |
| | | The amino acid sequence of the heavy chain variable region: |
| | | |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:52): |
| | | IPPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:135 | The amino acid sequence of the signal peptide bearing light chain of fusion protein 26,27 | The amino acid sequence of the light chain signal peptide: |
| | | MGWSLILLFLVAVATRVLS |
| | | The amino acid sequence of the light chain variable region: |
| | | EIVLTQSPGTLSLSPGERATLSCRASQSVGSSYLAWYQQKPGQAPR LLIYGAFSRATGIPDRFSGSGSGTDFTLTISRLEPEDFAVYYCQQYGS SPWTFGQGTKVEIK |
| | | The amino acid sequence of the light chain constant region: |
| | | |
| SEQ ID NO:136 | The amino acid sequence of the signal peptide bearing heavy chain of fusion protein26 | The amino acid sequence of the heavy chain signal peptide: |
| | | MGWSLILLFLVAVATRVLS |
| | | The amino acid sequence of the heavy chain variable region: |
| | | QVQLVESGGGVVQPGRSLRLSCAASGFTFSSYTMHWVRQAPGKG LEWVTFISYDGNNKYYADSVKGRFTISRDNSKNTLYLQMNSLRAE DTAIYYCARTGWLGPFDYWGQGTLVTVSS |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:47): |
| | | IPPHVQKSVNNDMIVTDNNGAVKFPQLCKFCDVRFSTCDNQKSC MSNCSITSICEKPQEVCVAVWRKNDENITLETVCHDPKLPYHDFIL EDAASPKCIMKEKKKPGETFFMCSCSSDECNDNIIFSEEYNTSNPD |
| SEQ ID NO:137 | The amino acid sequence of the signal peptide bearing heavy chain of fusion protein27 | The amino acid sequence of the heavy chain signal peptide: |
| | | MGWSLILLFLVAVATRVLS |
| | | The amino acid sequence of the heavy chain variable region: |
| | | QVQLVESGGGVVQPGRSLRLSCAASGFTFSSYTMHWVRQAPGKG LEWVTFISYDGNNKYYADSVKGRFTISRDNSKNTLYLQMNSLRAE DTAIYYCARTGWLGPFDYWGQGTLVTVSS |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:52): |
| | | IPPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:138 | The amino acid sequence of the signal peptide bearing light chain of fusion protein 28,29 | The amino acid sequence of the light chain signal peptide: |
| | | MGWSLILLFLVAVATRVLS |
| | | The amino acid sequence of the light chain variable region: |
| | | DIQMTQSPAALSASVGDRVTITCQASQDISNYLNWYQQKPGKAPK LLIYDASNLETGVPSRFSGSGSGTDFTFTISSLQPEDIATYFCQHFDH LPLAFGGGTKVEIK |
| | | The amino acid sequence of the light chain constant region: |
| | | |
| SEQ ID NO:139 | The amino acid sequence of the signal peptide bearing heavy chain of fusion protein28 | The amino acid sequence of the heavy chain signal peptide: |
| | | MGWSLILLFLVAVATRVLS |
| | | The amino acid sequence of the heavy chain variable region: |
| | | |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY |
| | | RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:47): |
| | | IPPHVQKSVNNDMIVTDNNGAVKFPQLCKFCDVRFSTCDNQKSC MSNCSITSICEKPQEVCVAVWRKNDENITLETVCHDPKLPYHDFIL EDAASPKCIMKEKKKPGETFFMCSCSSDECNDNIIFSEEYNTSNPD |
| SEQ ID NO:140 | The amino acid sequence of the signal peptide bearing heavy chain of fusion protein29 | The amino acid sequence of the heavy chain signal peptide: |
| | | MGWSLILLFLVAVATRVLS |
| | | The amino acid sequence of the heavy chain variable region: |
| | | |
| | | The amino acid sequence of the heavy chain constant region |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:52): |
| | | IPPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:141 | The amino acid sequence of fusion protein 6 heavy chain | The amino acid sequence of the heavy chain variable region C SEQ ID NO:28): |
| | | EVQLVQSGAEVKKPGATVKISCKVSGYTFTTYYTHWVKQAPGKG LEWIGWIYPGDVNTKYNEKFKGRVTLTADTSTDTAYMELSSLRSE DTAVYYCAREDPGSNYFDYWGQGTLVTVSS |
| | | The amino acid sequence of the heavy chain constant region C SEQ ID NO:46): |
| | | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSRDELTKNQVSLTCLVKGFYPSDIAVEWESNGQPENNY KTTPPVLDSDGSFFLYSKLTVDKSRWQQGNVFSCSVMHEALHNH YTQKSLSLSPG |
| | | The amino acid sequence of Linker linking the heavy chain C-terminal and TGFβR2 extracellular domain in the fusion protein C SEQ ID NO:66): |
| | | GGGGSGGGGSGGGGSGGGGS |
| | | The amino acid sequence of the full-length TGFβR2 extracellular domain (SEQ ID NO:52): |
| | | IPPHVLCKFCDVRFSTCDNQKSCMSNCSITSICEKPQEVCVAVWRK NDENITLETVCHDPKLPYHDFILEDAASPKCIMKEKKKPGETFFMC SCSSDECNDNIIFSEEYNTSNPD |
| SEQ ID NO:142 | The nucleotide sequence of plasmid pCMV3-mFlt3L | ATGACAGTGCTGGCGCCAGCCTGGAGCCCAAATTCCTCCCTGTT GCTGCTGTTGCTGCTGCTGAGTCCTTGCCTGCGGGGGACACCTG ACTGTTACTTCAGCCACAGTCCCATCTCCTCCAACTTCAAAGTG AAGTTTAGAGAGTTGACTGACCACCTGCTTAAAGATTACCCAGT CACTGTGGCCGTCAATCTTCAGGACGAGAAGCACTGCAAGGCC TTGTGGAGCCTCTTCCTAGCCCAGCGCTGGATAGAGCAACTGAA GACTGTGGCAGGGTCTAAGATGCAAACGCTTCTGGAGGACGTC AACACCGAGATACATTTTGTCACCTCATGTACCTTCCAGCCCCTA CCAGAATGTCTGCGATTCGTCCAGACCAACATCTCCCACCTCCT GAAGGACACCTGCACACAGCTGCTTGCTCTGAAGCCCTGTATC GGGAAGGCCTGCCAGAATTTCTCTCGGTGCCTGGAGGTGCAGT GCCAGCCGGACTCCTCCACCCTGCTGCCCCCAAGGAGTCCCATA GCCCTAGAAGCCACGGAGCTCCCAGAGCCTCGGCCCAGGCAGC TGTTGCTCCTGCTGCTGCTGCTGCTGCCTCTCACACTGGTGCTG CTGGCAGCCGCCTGGGGCCTTCGCTGGCAAAGGGCAAGAAGG AGGGGGGAGCTCCACCCTGGGGTGCCCCTCCCCTCCCATCCCTA A |
| SEQ ID NO:143 | The nucleotide sequence of plasmid pCMV3-mCSF2 | ATGTGGCTGCAGAATTTACTTTTCCTGGGCATTGTGGTCTACAGC CTCTCAGCACCCACCCGCTCACCCATCACTGTCACCCGGCCTTG GAAGCATGTAGAGGCCATCAAAGAAGCCCTGAACCTCCTGGAT GACATGCCTGTCACGTTGAATGAAGAGGTAGAAGTCGTCTCTAA CGAGTTCTCCTTCAAGAAGCTAACATGTGTGCAGACCCGCCTGA AGATATTCGAGCAGGGTCTACGGGGCAATTTCACCAAACTCAAG |
| | | GGCGCCTTGAACATGACAGCCAGCTACTACCAGACATACTGCCC CCCAACTCCGGAAACGGACTGTGAAACACAAGTTACCACCTAT GCGGATTTCATAGACAGCCTTAAAACCTTTCTGACTGATATCCCC TTTGAATGCAAAAAACCAGGCCAAAAATAA |
| SEQ ID NO: 144 | The nucleotide sequence of plasmid pGS6-EGFR-TT-WPRE | ATGCGACCCTCCGGGACGGCCGGGGCAGCGCTCCTGGCGCTGC TGGCTGCGCTCTGCCCGGCGAGTCGGGCTCTGGAGGAAAAGAA AGTTTGCCAAGGCACGAGTAACAAGCTCACGCAGTTGGGCACT TTTGAAGATCATTTTCTCAGCCTCCAGAGGATGTTCAATAACTGT GAGGTGGTCCTTGGGAATTTGGAAATTACCTATGTGCAGAGGAA TTATGATCTTTCCTTCTTAAAGACCATCCAGGAGGTGGCTGGTTA TGTCCTCATTGCCCTCAACACAGTGGAGCGAATTCCTTTGGAAA ACCTGCAGATCATCAGAGGAAATATGTACTACGAAAATTCCTATG CCTTAGCAGTCTTATCTAACTATGATGCAAATAAAACCGGACTGA AGGAGCTGCCCATGAGAAATTTACAGGAAATCCTGCATGGCGCC GTGCGGTTCAGCAACAACCCTGCCCTGTGCAACGTGGAGAGCA TCCAGTGGCGGGACATAGTCAGCAGTGACTTTCTCAGCAACATG TCGATGGACTTCCAGAACCACCTGGGCAGCTGCCAAAAGTGTG ATCCAAGCTGTCCCAATGGGAGCTGCTGGGGTGCAGGAGAGGA GAACTGCCAGAAACTGACCAAAATCATCTGTGCCCAGCAGTGC TCCGGGCGCTGCCGTGGCAAGTCCCCCAGTGACTGCTGCCACA ACCAGTGTGCTGCAGGCTGCACAGGCCCCCGGGAGAGCGACTG CCTGGTCTGCCGCAAATTCCGAGACGAAGCCACGTGCAAGGAC ACCTGCCCCCCACTCATGCTCTACAACCCCACCACGTACCAGAT GGATGTGAACCCCGAGGGCAAATACAGCTTTGGTGCCACCTGC GTGAAGAAGTGTCCCCGTAATTATGTGGTGACAGATCACGGCTC GTGCGTCCGAGCCTGTGGGGCCGACAGCTATGAGATGGAGGAA GACGGCGTCCGCAAGTGTAAGAAGTGCGAAGGGCCTTGCCGCA AAGTGTGTAACGGAATAGGTATTGGTGAATTTAAAGACTCACTC TCCATAAATGCTACGAATATTAAACACTTCAAAAACTGCACCTCC ATCAGTGGCGATCTCCACATCCTGCCGGTGGCATTTAGGGGTGA CTCCTTCACACATACTCCTCCTCTGGATCCACAGGAACTGGATAT TCTGAAAACCGTAAAGGAAATCACAGGGTTTTTGCTGATTCAGG CTTGGCCTGAAAACAGGACGGACCTCCATGCCTTTGAGAACCT AGAAATCATACGCGGCAGGACCAAGCAACATGGTCAGTTTTCTC TTGCAGTCGTCAGCCTGAACATAACATCCTTGGGATTACGCTCC CTCAAGGAGATAAGTGATGGAGATGTGATAATTTCAGGAAACAA AAATTTGTGCTATGCAAATACAATAAACTGGAAAAAACTGTTTG GGACCTCCGGTCAGAAAACCAAAATTATAAGCAACAGAGGTGA AAACAGCTGCAAGGCCACAGGCCAGGTCTGCCATGCCTTGTGC TCCCCCGAGGGCTGCTGGGGCCCGGAGCCCAGGGACTGCGTCT CTTGCCGGAATGTCAGCCGAGGCAGGGAATGCGTGGACAAGTG CAACCTTCTGGAGGGTGAGCCAAGGGAGTTTGTGGAGAACTCT |
| | | GAGTGCATACAGTGCCACCCAGAGTGCCTGCCTCAGGCCATGA ACATCACCTGCACAGGACGGGGACCAGACAACTGTATCCAGTG TGCCCACTACATTGACGGCCCCCACTGCGTCAAGACCTGCCCGG CAGGAGTCATGGGAGAAAACAACACCCTGGTCTGGAAGTACGC AGACGCCGGCCATGTGTGCCACCTGTGCCATCCAAACTGCACCT ACGGATGCACTGGGCCAGGTCTTGAAGGCTGTCCAACGAATGG GCCTAAGATCCCGTCCATCGCCACTGGGATGGTGGGGGCCCTCC TCTTGCTGCTGGTGGTGGCCCTGGGGATCGGCCTCTTCATGCGA AGGCGCCACATCGTTCGGAAGCGCACGCTGCGGAGGCTGCTGC AGGAGAGGGAGCTTGTGGAGCCTCTTACACCCAGTGGAGAAGC TCCCAACCAAGCTCTCTTGAGGATCTTGAAGGAAACTGAATTCA AAAAGATCAAAGTGCTGGGCTCCGGTGCGTTCGGCACGGTGTA TAAGGGACTCTGGATCCCAGAAGGTGAGAAAGTTAAAATTCCC GTCGCTATCAAGGAATTAAGAGAAGCAACATCTCCGAAAGCCA ACAAGGAAATCCTCGATGAAGCCTACGTGATGGCCAGCGTGGA CAACCCCCACGTGTGCCGCCTGCTGGGCATCTGCCTCACCTCCA CCGTGCAACTCATCACGCAGCTCATGCCCTTCGGCTGCCTCCTG GACTATGTCCGGGAACACAAAGACAATATTGGCTCCCAGTACCT GCTCAACTGGTGTGTGCAGATCGCAAAGGGCATGAACTACTTG GAGGACCGTCGCTTGGTGCACCGCGACCTGGCAGCCAGGAACG TACTGGTGAAAACACCGCAGCATGTCAAGATCACAGATTTTGGG CTGGCCAAACTGCTGGGTGCGGAAGAGAAAGAATACCATGCAG AAGGAGGCAAAGTGCCTATCAAGTGGATGGCATTGGAATCAATT TTACACAGAATCTATACCCACCAGAGTGATGTCTGGAGCTACGG GGTGACCGTTTGGGAGTTGATGACCTTTGGATCCAAGCCATATG ACGGAATCCCTGCCAGCGAGATCTCCTCCATCCTGGAGAAAGG AGAACGCCTCCCTCAGCCACCCATATGTACCATCGATGTCTACAT GATCATGGTCAAGTGCTGGATGATAGACGCAGATAGTCGCCCAA AGTTCCGTGAGTTGATCATCGAATTCTCCAAAATGGCCCGAGAC CCCCAGCGCTACCTTGTCATTCAGGGGGATGAAAGAATGCATTT GCCAAGTCCTACAGACTCCAACTTCTACCGTGCCCTGATGGATG AAGAAGACATGGACGACGTGGTGGATGCCGACGAGTACCTCAT CCCACAGCAGGGCTTCTTCAGCAGCCCCTCCACGTCACGGACT CCCCTCCTGAGCTCTCTGAGTGCAACCAGCAACAATTCCACCGT GGCTTGCATTGATAGAAATGGGCTGCAAAGCTGTCCCATCAAGG AAGACAGCTTCTTGCAGCGATACAGCTCAGACCCCACAGGCGC CTTGACTGAGGACAGCATAGACGACACCTTCCTCCCAGTGCCTG AATACATAAACCAGTCCGTTCCCAAAAGGCCCGCTGGCTCTGTG CAGAATCCTGTCTATCACAATCAGCCTCTGAACCCCGCGCCCAG CAGAGACCCACACTACCAGGACCCCCACAGCACTGCAGTGGGC AACCCCGAGTATCTCAACACTGTCCAGCCCACCTGTGTCAACAG CACATTCGACAGCCCTGCCCACTGGGCCCAGAAAGGCAGCCAC CAAATTAGCCTGGACAACCCTGACTACCAGCAGGACTTCTTTCC CAAGGAAGCCAAGCCAAATGGCATCTTTAAGGGCTCCACAGCT |
| | | GAAAATGCAGAATACCTAAGGGTCGCGCCACAAAGCAGTGAAT TTATTGGAGCA |
| SEQ ID NO: 145 | The amino acid sequence encoded by plasmid pCMV3-mFlt3L | MTVLAPAWSPNSSLLLLLLLLLSPCLRGTPDCYFSHSPISSNFKVKFR ELTDHLLKDYPVTVAVNLQDEKHCKALWSLFLAQRWIEQLKTVAG SKMQTLLEDVNTEIHFVTSCTFQPLPECLRFVQTNISHLLKDTCTQ LLALKPCIGKACQNFSRCLEVQCQPDSSTLLPPRSPIALEATELPEP RPRQLLLLLLLLLPLTLVLLAAAWGLRWQRARRRGELHPGVPLPS HP* |
| SEQ ID NO: 146 | The amino acid sequence encoded by plasmid pCMV3-mCSF2 | MWLQNLLFLGIVVYSLSAPTRSPITVTRPWKHVEAIKEALNLLDD MPVTLNEEVEVVSNEFSFKKLTCVQTRLKIFEQGLRGNFTKLKGA LNMTASYYQTYCPPTPETDCETQVTTYADFIDSLKTFLTDIPFECKK PGQK* |
| SEQ ID NO: 147 | The amino acid sequence encoded by plasmid pGS6-EGFR-TT- WPRE | |
| SEQ ID NO: 148 | The nucleotide sequence encoding fusion protein 1 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:68): |
| | | ATCCCACCGCACGTTCAGAAGTCGGTTAATAACGACATGATAGT CACTGACAACAACGGTGCAGTCAAGTTTCCACAACTGTGTAAA TTTTGTGATGTGAGATTTTCCACCTGTGACAACCAGAAATCCTG CATGAGCAACTGCAGCATCACCTCCATCTGTGAGAAGCCACAG GAAGTCTGTGTGGCTGTATGGAGAAAGAATGACGAGAACATAA CACTAGAGACAGTTTGCCATGACCCCAAGCTCCCCTACCATGAC TTTATTCTGGAAGATGCTGCTTCTCCAAAGTGCATTATGAAGGA AAAAAAAAAGCCTGGTGAGACTTTCTTCATGTGTTCCTGTAGCT CTGATGAGTGCAATGACAACATCATCTTCTCAGAAGAATATAAC ACCAGCAATCCTGACTAAA |
| SEQ ID NO149 | The nucleotide sequence encoding fusion protein 2 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT |
| | | CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:69): |
| | | ATCCCACCGCACGTTAACAACGGTGCAGTCAAGTTTCCACAACT GTGTAAATTTTGTGATGTGAGATTTTCCACCTGTGACAACCAGA AATCCTGCATGAGCAACTGCAGCATCACCTCCATCTGTGAGAAG CCACAGGAAGTCTGTGTGGCTGTATGGAGAAAGAATGACGAGA ACATAACACTAGAGACAGTTTGCCATGACCCCAAGCTCCCCTAC CATGACTTTATTCTGGAAGATGCTGCTTCTCCAAAGTGCATTATG AAGGAAAAAAAAAAGCCTGGTGAGACTTTCTTCATGTGTTCCT GTAGCTCTGATGAGTGCAATGACAACATCATCTTCTCAGAAGAA TATAACACCAGCAATCCTGACTAAA |
| SEQ ID NO:150 | The nucleotide sequence encoding fusion protein 3 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG |
| | | AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:70): |
| | | ATCCCACCGCACGTTGGTGCAGTCAAGTTTCCACAACTGTGTAA ATTTTGTGATGTGAGATTTTCCACCTGTGACAACCAGAAATCCT GCATGAGCAACTGCAGCATCACCTCCATCTGTGAGAAGCCACA GGAAGTCTGTGTGGCTGTATGGAGAAAGAATGACGAGAACATA ACACTAGAGACAGTTTGCCATGACCCCAAGCTCCCCTACCATGA CTTTATTCTGGAAGATGCTGCTTCTCCAAAGTGCATTATGAAGGA AAAAAAAAAGCCTGGTGAGACTTTCTTCATGTGTTCCTGTAGCT CTGATGAGTGCAATGACAACATCATCTTCTCAGAAGAATATAAC ACCAGCAATCCTGACTAAA |
| SEQ ID NO:151 | The nucleotide sequence encoding fusion protein 4 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:71): |
| | | ATCCCACCGCACGTTGTCAAGTTTCCACAACTGTGTAAATTTTG TGATGTGAGATTTTCCACCTGTGACAACCAGAAATCCTGCATGA GCAACTGCAGCATCACCTCCATCTGTGAGAAGCCACAGGAAGT CTGTGTGGCTGTATGGAGAAAGAATGACGAGAACATAACACTA GAGACAGTTTGCCATGACCCCAAGCTCCCCTACCATGACTTTAT TCTGGAAGATGCTGCTTCTCCAAAGTGCATTATGAAGGAAAAAA AAAAGCCTGGTGAGACTTTCTTCATGTGTTCCTGTAGCTCTGAT GAGTGCAATGACAACATCATCTTCTCAGAAGAATATAACACCAG CAATCCTGACTAAA |
| SEQ ID NO:152 | The nucleotide sequence encoding fusion protein 5 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:72): |
| | | ATCCCACCGCACGTTCAACTGTGTAAATTTTGTGATGTGAGATTT TCCACCTGTGACAACCAGAAATCCTGCATGAGCAACTGCAGCAT CACCTCCATCTGTGAGAAGCCACAGGAAGTCTGTGTGGCTGTAT GGAGAAAGAATGACGAGAACATAACACTAGAGACAGTTTGCCA TGACCCCAAGCTCCCCTACCATGACTTTATTCTGGAAGATGCTG CTTCTCCAAAGTGCATTATGAAGGAAAAAAAAAAGCCTGGTGA GACTTTCTTCATGTGTTCCTGTAGCTCTGATGAGTGCAATGACA ACATCATCTTCTCAGAAGAATATAACACCAGCAATCCTGACTAA A |
| SEQ ID NO:153 | The nucleotide sequence encoding fusion protein 6 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG |
| | | GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:73): |
| | | ATCCCACCGCACGTTCTGTGTAAATTTTGTGATGTGAGATTTTCC ACCTGTGACAACCAGAAATCCTGCATGAGCAACTGCAGCATCA CCTCCATCTGTGAGAAGCCACAGGAAGTCTGTGTGGCTGTATGG AGAAAGAATGACGAGAACATAACACTAGAGACAGTTTGCCATG ACCCCAAGCTCCCCTACCATGACTTTATTCTGGAAGATGCTGCTT CTCCAAAGTGCATTATGAAGGAAAAAAAAAAGCCTGGTGAGAC TTTCTTCATGTGTTCCTGTAGCTCTGATGAGTGCAATGACAACAT CATCTTCTCAGAAGAATATAACACCAGCAATCCTGACTAAA |
| SEQ ID NO:154 | The nucleotide sequence encoding fusion protein 7 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC |
| | | CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:74): |
| | | ATCCCACCGCACGTTTGTAAATTTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC CAAAGTGCATTATGAAGGAAAAAAAAAAGCCTGGTGAGACTTT CTTCATGTGTTCCTGTAGCTCTGATGAGTGCAATGACAACATCAT CTTCTCAGAAGAATATAACACCAGCAATCCTGACTAAA |
| SEQ ID NO:155 | The nucleotide sequence encoding fusion protein 8 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG |
| | | GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:75): |
| | | ATCCCACCGCACGTTCAGCTGTGTAAATTTTGTGATGTGAGATTT TCCACCTGTGACAACCAGAAATCCTGCATGAGCAACTGCAGCAT CACCTCCATCTGTGAGAAGCCACAGGAAGTCTGTGTGGCTGTAT GGAGAAAGAATGACGAGAACATAACACTAGAGACAGTTTGCCA TGACCCCAAGCTCCCCTACCATGACTTTATTCTGGAAGATGCTG CTTCTCCAAAGTGCATTATGAAGGAAAAAAAAAAGCCTGGTGA GACTTTCTTCATGTGTTCCTGTAGCTCTGATGAGTGCAATGACA ACATCATCTTCTCAGAAGAATATAACACCAGCAATCCTGACTAA A |
| SEQ ID NO156 | The nucleotide sequence encoding fusion protein 9 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC |
| | | AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:76): |
| | | ATCCCACCGCACCTGTGTAAATTTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC |
| | | CAAAGTGCATTATGAAGGAAAAAAAAAAGCCTGGTGAGACTTT CTTCATGTGTTCCTGTAGCTCTGATGAGTGCAATGACAACATCAT CTTCTCAGAAGAATATAACACCAGCAATCCTGACTAAA |
| SEQ ID NO:157 | The nucleotide sequence encoding fusion protein 10 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:77): |
| | | ATCCCACCGCTGTGTAAATTTTGTGATGTGAGATTTTCCACCTGT GACAACCAGAAATCCTGCATGAGCAACTGCAGCATCACCTCCAT CTGTGAGAAGCCACAGGAAGTCTGTGTGGCTGTATGGAGAAAG AATGACGAGAACATAACACTAGAGACAGTTTGCCATGACCCCA AGCTCCCCTACCATGACTTTATTCTGGAAGATGCTGCTTCTCCAA AGTGCATTATGAAGGAAAAAAAAAAGCCTGGTGAGACTTTCTT CATGTGTTCCTGTAGCTCTGATGAGTGCAATGACAACATCATCTT CTCAGAAGAATATAACACCAGCAATCCTGACTAAA |
| SEQ ID NO158 | The nucleotide sequence encoding fusion protein 11 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG |
| | | GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:78): |
| | | ATCCCACTGTGTAAATTTTGTGATGTGAGATTTTCCACCTGTGAC AACCAGAAATCCTGCATGAGCAACTGCAGCATCACCTCCATCTG TGAGAAGCCACAGGAAGTCTGTGTGGCTGTATGGAGAAAGAAT GACGAGAACATAACACTAGAGACAGTTTGCCATGACCCCAAGC TCCCCTACCATGACTTTATTCTGGAAGATGCTGCTTCTCCAAAGT GCATTATGAAGGAAAAAAAAAAGCCTGGTGAGACTTTCTTCAT GTGTTCCTGTAGCTCTGATGAGTGCAATGACAACATCATCTTCTC AGAAGAATATAACACCAGCAATCCTGACTAAA |
| SEQ ID NO159 | The nucleotide sequence encoding fusion protein 12 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG |
| | | AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:79): |
| | | ATCCTGTGTAAATTTTGTGATGTGAGATTTTCCACCTGTGACAAC CAGAAATCCTGCATGAGCAACTGCAGCATCACCTCCATCTGTGA GAAGCCACAGGAAGTCTGTGTGGCTGTATGGAGAAAGAATGAC GAGAACATAACACTAGAGACAGTTTGCCATGACCCCAAGCTCC CCTACCATGACTTTATTCTGGAAGATGCTGCTTCTCCAAAGTGCA TTATGAAGGAAAAAAAAAAGCCTGGTGAGACTTTCTTCATGTGT TCCTGTAGCTCTGATGAGTGCAATGACAACATCATCTTCTCAGA AGAATATAACACCAGCAATCCTGACTAAA |
| SEQ ID NO:160 | The nucleotide sequence encoding fusion protein 13 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:80): |
| | | CCACCGCACGTTCTGTGTAAATTTGTGATGTGAGATTTTCCACC TGTGACAACCAGAAATCCTGCATGAGCAACTGCAGCATCACCTC CATCTGTGAGAAGCCACAGGAAGTCTGTGTGGCTGTATGGAGA AAGAATGACGAGAACATAACACTAGAGACAGTTTGCCATGACC CCAAGCTCCCCTACCATGACTTTATTCTGGAAGATGCTGCTTCTC CAAAGTGCATTATGAAGGAAAAAAAAAAGCCTGGTGAGACTTT CTTCATGTGTTCCTGTAGCTCTGATGAGTGCAATGACAACATCAT CTTCTCAGAAGAATATAACACCAGCAATCCTGACTAAA |
| SEQ ID NO:161 | The nucleotide sequence encoding fusion protein 14 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:81): |
| | | CCGCACGTTCTGTGTAAATTTTGTGATGTGAGATTTTCCACCTGT GACAACCAGAAATCCTGCATGAGCAACTGCAGCATCACCTCCAT CTGTGAGAAGCCACAGGAAGTCTGTGTGGCTGTATGGAGAAAG AATGACGAGAACATAACACTAGAGACAGTTTGCCATGACCCCA AGCTCCCCTACCATGACTTTATTCTGGAAGATGCTGCTTCTCCAA AGTGCATTATGAAGGAAAAAAAAAAGCCTGGTGAGACTTTCTT CATGTGTTCCTGTAGCTCTGATGAGTGCAATGACAACATCATCTT CTCAGAAGAATATAACACCAGCAATCCTGACTAAA |
| SEQ ID NO:162 | The nucleotide sequence encoding fusion protein 15 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG |
| | | GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:82): |
| | | CACGTTCTGTGTAAATTTTGTGATGTGAGATTTTCCACCTGTGAC AACCAGAAATCCTGCATGAGCAACTGCAGCATCACCTCCATCTG TGAGAAGCCACAGGAAGTCTGTGTGGCTGTATGGAGAAAGAAT GACGAGAACATAACACTAGAGACAGTTTGCCATGACCCCAAGC TCCCCTACCATGACTTTATTCTGGAAGATGCTGCTTCTCCAAAGT GCATTATGAAGGAAAAAAAAAAGCCTGGTGAGACTTTCTTCAT GTGTTCCTGTAGCTCTGATGAGTGCAATGACAACATCATCTTCTC AGAAGAATATAACACCAGCAATCCTGACTAAA |
| SEQ ID NO:163 | The nucleotide sequence encoding fusion protein 16 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC |
| | | CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:83): |
| | | GTTCTGTGTAAATTTTGTGATGTGAGATTTTCCACCTGTGACAAC CAGAAATCCTGCATGAGCAACTGCAGCATCACCTCCATCTGTGA GAAGCCACAGGAAGTCTGTGTGGCTGTATGGAGAAAGAATGAC GAGAACATAACACTAGAGACAGTTTGCCATGACCCCAAGCTCC CCTACCATGACTTTATTCTGGAAGATGCTGCTTCTCCAAAGTGCA TTATGAAGGAAAAAAAAAAGCCTGGTGAGACTTTCTTCATGTGT TCCTGTAGCTCTGATGAGTGCAATGACAACATCATCTTCTCAGA AGAATATAACACCAGCAATCCTGACTAAA |
| SEQ ID NO:164 | The nucleotide sequence encoding fusion protein 17 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG |
| | | GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:84): |
| | | GGTGCAGTCAAGTTTCCACAACTGTGTAAATTTTGTGATGTGAG ATTTTCCACCTGTGACAACCAGAAATCCTGCATGAGCAACTGCA GCATCACCTCCATCTGTGAGAAGCCACAGGAAGTCTGTGTGGCT GTATGGAGAAAGAATGACGAGAACATAACACTAGAGACAGTTT GCCATGACCCCAAGCTCCCCTACCATGACTTTATTCTGGAAGAT GCTGCTTCTCCAAAGTGCATTATGAAGGAAAAAAAAAAGCCTG GTGAGACTTTCTTCATGTGTTCCTGTAGCTCTGATGAGTGCAATG ACAACATCATCTTCTCAGAAGAATATAACACCAGCAATCCTGAC TAAA |
| SEQ ID NO:165 | The nucleotide sequence encoding fusion protein 18 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC |
| | | AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:85): |
| | | GTCAAGTTTCCACAACTGTGTAAATTTTGTGATGTGAGATTTTCC ACCTGTGACAACCAGAAATCCTGCATGAGCAACTGCAGCATCA CCTCCATCTGTGAGAAGCCACAGGAAGTCTGTGTGGCTGTATGG AGAAAGAATGACGAGAACATAACACTAGAGACAGTTTGCCATG ACCCCAAGCTCCCCTACCATGACTTTATTCTGGAAGATGCTGCTT |
| | | CTCCAAAGTGCATTATGAAGGAAAAAAAAAAGCCTGGTGAGAC TTTCTTCATGTGTTCCTGTAGCTCTGATGAGTGCAATGACAACAT CATCTTCTCAGAAGAATATAACACCAGCAATCCTGACTAAA |
| SEQ ID NO: 166 | The nucleotide sequence encoding fusion protein 19 heavy chain | The nucleotide sequence encoding the heavy chain variable region (SEQ ID NO:36): |
| | | GAGGTCCAACTTGTCCAGTCTGGAGCAGAGGTGAAGAAGCCTG GAGCCACAGTGAAGATTTCCTGTAAGGTGTCTGGCTACACCTTC ACCACCTACTACACCCACTGGGTGAAGCAGGCTCCTGGCAAGG GATTGGAGTGGATTGGCTGGATTTACCCTGGAGATGTGAACACC AAATACAATGAGAAGTTCAAGGGCAGGGTGACCCTGACAGCAG ACACCAGCACAGACACAGCCTATATGGAACTGTCCTCCCTGAGG TCTGAGGACACAGCAGTCTACTACTGTGCCAGGGAGGACCCTG GCAGCAACTACTTTGACTACTGGGGACAAGGCACCCTGGTGAC AGTGTCCAGC |
| | | The nucleotide sequence encoding the heavy chain constant region (SEQ ID NO:67): |
| | | GCAAGCACCAAGGGCCCATCGGTCTTCCCCCTGGCACCCTCCTC CAAGAGCACCTCTGGGGGCACAGCGGCCCTGGGCTGCCTGGTC AAGGACTACTTCCCCGAACCGGTGACGGTGTCGTGGAACTCAG GCGCCCTGACCAGCGGCGTGCACACCTTCCCGGCTGTCCTACA GTCCTCAGGACTCTACTCCCTCAGCAGCGTGGTGACCGTGCCCT CCAGCAGCTTGGGCACCCAGACCTACATCTGCAACGTGAATCA CAAGCCCAGCAACACCAAGGTGGACAAGAAAGTTGAGCCCAA ATCTTGTGACAAAACTCACACATGCCCACCGTGCCCAGCACCTG AACTCCTGGGGGGACCGTCAGTCTTCCTCTTCCCCCCAAAACCC AAGGACACCCTCATGATCTCCCGGACCCCTGAGGTCACgTGCGT GGTGGTGGACGTGAGCCACGAAGACCCcGAGGTCAAGTTCAAC TGGTACGTGGACGGCGTGGAGGTGCATAATGCCAAGACAAAGC CGCGGGAGGAGCAGTACAACAGCACGTACCGTGTGGTCAGCGT CCTCACCGTCCTGCACCAGGACTGGCTGAATGGCAAGGAGTAC AAGTGCAAGGTCTCCAACAAAGCCCTCCCAGCCCCCATCGAGA AAACCATCTCCAAAGCCAAAGGGCAGCCCCGAGAACCACAGGT GTACACCCTGCCCCCATCCCGGGATGAGCTGACCAAGAACCAG GTCAGCCTGACCTGCCTGGTCAAAGGCTTCTATCCCAGCGACAT CGCCGTGGAGTGGGAGAGCAATGGGCAGCCGGAGAACAACTA CAAGACCACGCCTCCCGTGCTGGACTCCGACGGCTCCTTCTTCC TCTACAGCAAGCTCACCGTGGACAAGAGCAGGTGGCAGCAGG GGAACGTCTTCTCATGCTCCGTGATGCATGAGGCTCTGCACAAC CACTACACGCAGAAGAGCCTCTCCCTGTCTCCGGGT |
| | | The nucleotide sequence encoding Linker linking the heavy chain C-terminal and TGFβR2 extracellular domain in the fusion protein (SEQ ID NO:87): |
| | | GGTGGTGGCGGTTCAGGCGGAGGTGGCTCTGGAGGTGGAGGTT CAGGAGGTGGTGGTTCT |
| | | The nucleotide sequence encoding the full-length extracellular domain of TGFβR2 (SEQ ID NO:86): |
| | | CTGTGTAAATTTTGTGATGTGAGATTTTCCACCTGTGACAACCA GAAATCCTGCATGAGCAACTGCAGCATCACCTCCATCTGTGAGA AGCCACAGGAAGTCTGTGTGGCTGTATGGAGAAAGAATGACGA GAACATAACACTAGAGACAGTTTGCCATGACCCCAAGCTCCCCT ACCATGACTTTATTCTGGAAGATGCTGCTTCTCCAAAGTGCATTA TGAAGGAAAAAAAAAAGCCTGGTGAGACTTTCTTCATGTGTTC CTGTAGCTCTGATGAGTGCAATGACAACATCATCTTCTCAGAAG AATATAACACCAGCAATCCTGACTAAA |

### REFERENCES

1. Xie, F., et al., TGF-beta signaling in cancer metastasis. Acta Biochim Biophys Sin (Shanghai), 2018. 50(1): p. 121-132.
2. Colak, S. and P. Ten Dijke, Targeting TGF-beta Signaling in Cancer. Trends Cancer, 2017. 3(1): p. 56-71.
3. Fabregat, I., et al., TGF-beta signaling in cancer treatment. Curr Pharm Des, 2014. 20(17): p. 2934-47.
4. Batlle, E. and J. Massague, Transforming Growth Factor-beta Signaling in Immunity and Cancer. Immunity, 2019. 50(4): p. 924-940.
5. Laskin, J.J. and A.B. Sandler, Epidermal growth factor receptor: a promising target in solid tumours. Cancer treatment reviews, 2004. 30(1): p. 1-17.
6. Hynes, N., et al., The ErbB receptor tyrosine family as signal integrators. Endocrine-related cancer, 2001. 8(3): p. 151-159.
7. Zandi, R., et al., Mechanisms for oncogenic activation of the epidermal growth factor receptor. Cellular signalling, 2007. 19(10): p. 2013-2023.
8. Seshacharyulu, P., et al., Targeting the EGFR signaling pathway in cancer therapy. Expert opinion on therapeutic targets, 2012. 16(1): p. 15-31.
9. Zhao, Y, et al., TGF -β transactivates EGFR and facilitates breast cancer migration and invasion through canonical Smad3 and ERK/Sp1 signaling pathways. Molecular oncology, 2018. 12(3): p. 305-321.
10. Wendt, M.K., J.A. Smith, and W.P. Schiemann, Transforming growth factor-β-induced epithelial-mesenchymal transition facilitates epidermal growth factor-dependent breast cancer progression. Oncogene, 2010. 29(49): p. 6485.
11. Lee, E., et al., Transforming growth factorβ1 transactivates EGFR via an H2O2-dependent mechanism in squamous carcinoma cell line. Cancer letters, 2010. 290(1): p. 43-48.
12. Dunfield, L.D. and M.W. Nachtigal, Inhibition of the antiproliferative effect of TGFβ by EGF in primary human ovarian cancer cells. Oncogene, 2003. 22(30): p. 4745.
13. Kretzschmar, M., et al., A mechanism of repression of TGFβ/Smad signaling by oncogenic Ras. Genes & development, 1999. 13(7): p. 804-816.
14. ten Dijke, P., K. Miyazono, and C.-H. Heldin, Signaling inputs converge on nuclear effectors in TGF-β signaling. Trends in biochemical sciences, 2000. 25(2): p. 64-70.
15. Funaba, M., C.M. Zimmerman, and L.S. Mathews, Modulation of Smad2-mediated signaling by extracellular signal-regulated kinase. Journal of Biological Chemistry, 2002. 277(44): p. 41361-41368.
16. Richter, P., et al., EGF/TGFbeta1 co-stimulation of oral squamous cell carcinoma cells causes an epithelial-mesenchymal transition cell phenotype expressing laminin 332. J Oral Pathol Med, 2011. 40(1): p. 46-54.
17. Uttamsingh, S., et al., Synergistic effect between EGF and TGF-beta1 in inducing oncogenic properties of intestinal epithelial cells. Oncogene, 2008. 27(18): p. 2626-34.
18. Xu, Z., et al., TGFbeta and EGF synergistically induce a more invasive phenotype of epithelial ovarian cancer cells. Biochem Biophys Res Commun, 2010. 401(3): p. 376-81.
19. Xiong, J., et al., Epidermal growth factor promotes transforming growth factor-beta1-induced epithelial-mesenchymal transition in HK-2 cells through a synergistic effect on Snail. Mol Biol Rep, 2014. 41(1): p. 241-50.
20. Buonato, J.M., I.S. Lan, and M.J. Lazzara, EGF augments TGFbeta-induced epithelial-mesenchymal transition by promoting SHP2 binding to GAB1. J Cell Sci, 2015. 128(21): p. 3898-909.
21. Wang, T., et al., The TGFβ-miR-499a-SHKBP1 pathway induces resistance to EGFR inhibitors in osteosarcoma cancer stem cell-like cells. Journal of Experimental & Clinical Cancer Research, 2019. 38(1): p. 226.
22. Jie, H.B., et al., CTLA-4(+) Regulatory T Cells Increased in Cetuximab-Treated Head and Neck Cancer Patients Suppress NK Cell Cytotoxicity and Correlate with Poor Prognosis. Cancer Res, 2015. 75(11): p. 2200-10.
23. Bedi, A., et al., Inhibition of TGF-beta enhances the in vivo antitumor efficacy of EGF receptor-targeted therapy. Mol Cancer Ther, 2012. 11(11): p. 2429-39.
24. Zhang, Y., et al., The canonical TGF-beta/Smad signalling pathway is involved in PD-L1-induced primary resistance to EGFR-TKIs in EGFR-mutant non-small-cell lung cancer. Respir Res, 2019. 20(1): p. 164.
25. Jones, S.T. and M.M. Bendig, Rapid PCR-cloning of full-length mouse immunoglobulin variable regions. Biotechnology (N Y), 1991. 9(6): p. 579.
26. Kabat, E.A., et al., Sequences of proteins of immunological interest. 1992: DIANE publishing.
27. Jones, P.T., et al., Replacing the complementarity-determining regions in a human antibody with those from a mouse. Nature, 1986. 321(6069): p. 522.
28. Verhoeyen, M. and L. Riechmann, Engineering of antibodies. BioEssays, 1988. 8(2 - 3): p. 74-78.

## Claims

1. A truncated TGFβR2 extracellular domain molecule that, compared to its natural form,
a) at least the amino acid residues at positions 6-16 thereof are deleted, and further optionally, the amino acid residues at positions 17- 17+n thereof are deleted, where n is an integer of 1-10; preferably, n is 2, 4, 8, 9 or 10; most preferably, n is 9; or
b) on the basis of the deletion of amino acid residues thereof at positions 6-26, furthermore, the amino acid residues thereof at positions 5, 4-5, 3-5, 2-5, 1, 1-2, 1-3, or 1-4 are deleted; or
c) the amino acid residues at positions 7-26 are deleted.

2. The molecule of claim 1,the amino acid sequence comprises any of SEQ ID NO: 48-62.

3. A fusion protein comprising the molecule of claim 1 or 2.

4. The fusion protein of claim 3, comprising
a) the truncated TGFβR2 extracellular domain molecule of claim 1 or 2; and
b) a targeting portion.

5. The fusion protein of claim 4, the targeting portion is a cancer cell-specific targeting portion selected from an antibody or antigen-binding fragment thereof, a functional ligand or Fc fusion protein thereof, and a receptor protein or Fc fusion protein thereof.

6. The fusion protein of claim 5, the targeting portion is an anti-EGFR antibody or an antigen-binding fragment thereof.

7. The fusion protein of any one of claims 4-6, wherein the N-terminal of the truncated TGFβR2 extracellular domain molecule is linked to the C-terminal of the heavy chain of the targeting portion; and
optionally, linked by a linker.

8. The fusion protein of claim 7, wherein the linker is a GaS flexible peptide linker, preferably a (G₄S)₄ peptied linker.

9. An isolated antibody binding to EGFRor an antigen-binding fragment thereof, comprising
(a) a heavy chain variable region comprising heavy chain CDR1, heavy chain CDR2, and heavy chain CDR3 domains comprising SEQ ID NOs: 19, 20, and 21, respectively, and/or
(b) a light chain variable region comprising a light chain CDR1, light chain CDR2, and light chain CDR3 domain comprising SEQ ID NOs: 16, 17, and 18, respectively.

10. The antibody or an antigen-binding fragment of claim 9, comprising
a) a heavy chain variable region comprising a sequence comprising SEQ ID NO:28 or a sequence having at least 85%, 88%, 90%, 95%, 98%, or 99% sequence identity therewith; and/or
(b) a light chain variable region comprising a sequence comprising SEQ ID NO: 29 or a sequence having at least 85%, 88%, 90%, 95%, 98%, or 99% sequence identity therewith.

11. The antibody or antigen-binding fragment of claim 9 or 10, wherein said antibody further comprises:
a) a heavy chain constant region, preferably comprising a sequence comprising SEQ ID NO: 30 or a sequence having at least 85%, 88%, 90%, 95%, 98%, or 99% sequence identity therewith; and/or
b) a light chain constant region, preferably comprising a sequence comprising SEQ ID NO: 31 or a sequence having at least 85%, 88%, 90%, 95%, 98%, or 99% sequence identity therewith.

12. The fusion protein of claims 3-8, wherein the targeting portion is selected from the anti-EGFR antibodyof any one of claims 9-11, Trastuzumab, Bevacizumab, Ramucirumab, Ipilimumab, or Panitumumab.

13. The fusion protein of claim 7, wherein
a) the amino acid sequence of a heavy chain comprises SEQ ID NO: 141 or a sequence having at least 85%, 88%, 90%, 95%, 98%, or 99% sequence identity therewith; and
b) the amino acid sequence of a light chain comprising SEQ ID NO:23 or a sequence having at least 85%, 88%, 90%, 95%, 98%, or 99% sequence identity therewith;
wherein the fusion proteincomprises two heavy chains and two light chains; a disulfide bond is formed between a first light chain and a first heavy chain thereof, a disulfide bond is formed between a second light chain and a second heavy chain thereof, and a disulfide bond is formed between a first heavy chain and a second heavy chain thereof.

14. The fusion protein of claim 13,
having a K_{D} value of 2.92 pM-26.3 pM,preferably 7 pM-9 pM, most preferably 8.77 pMfor binding affinity to human EGFR protein, and
having a K_{D} value of 23 pM -288.3 pM, preferably 64 pM-144 pM , most preferably 96.1 pMfor binding affinityto human TGF-β1 protein.

15. A conjugate comprising the truncated TGFβR2 extracellular domain molecule of claim 1 or 2, the fusion protein of any one of claims 3-8 and 12-14, the antibody or antigen-binding fragment thereof of any one of claims 9-11 and an additional therapeutic agent, preferably said antibody or antigen-binding fragment thereof and the additional therapeutic agent are linked by a linker.

16. A nucleic acid encoding the truncated TGFβR2 extracellular domain molecule of claim
1 or 2, the fusion protein of any one of claims 3-8 and 12-14, the antibody or antigen-binding fragment thereof of any one of claims 9-11, which is mRNA and/or DNA.

17. The nucleic acid of claim 16, comprising
any one of SEQ ID NOs: 32 to 39; or
any one of SEQ ID NOs: 67-84; or
any one of SEQ ID NOs: 148-163, or an functional variant thereof.

18. An expression vector comprising the nucleic acid of claim 16 or 17.

19. A host cell comprising the nucleic acid of claim 16 or 17 or the expression vector of claim 18.

20. A method for producing thetruncated TGFβR2 extracellular domain molecule of claim 1 or 2, the fusion protein of any one of claims 3-8 and 12-14, the antibody or antigen-binding fragment thereof of any one of claims 9-11, comprising culturing the host cell of claim 19 under conditions suitable for the expression of the preceding protein molecule, and recovering the expressed product from the culture medium.

21. A pharmaceutical composition comprising
a) thetruncated TGFβR2 extracellular domain molecule of claim 1 or 2, the fusion protein of any one of claims 3-8 and 12-14, the antibody or antigen-binding fragment thereof of any one of claims 9-11, the conjugate as claimed in claim 15, the nucleic acid of any one of claims 16-17, or the expression vector of claim 18, and
b) a pharmaceutically acceptable carrier; optionally
c) one or more additional therapeutic agents.

22. Use of the truncated TGFβR2 extracellular domain molecule of claim 1 or 2, the fusion protein of any one of claims 3-8 and 12-14, the antibody or antigen-binding fragment thereof of any one of claims 9-11, the conjugate as claimed in claim 15, the nucleic acid of any one of claims 16-17, the expression vector of claim 18, or the pharmaceutical composition of claim 21, for the prevention and treatment of cancer, preferably for the treatment of gastric cancer.

23. Use of the truncated TGFβR2 extracellular domain molecule of claim 1 or 2, the fusion protein of any one of claims 3-8 and 12-14, the antibody or antigen-binding fragment thereof of any one of claims 9-11, the conjugate as claimed in claim 15, the nucleic acid of any one of claims 16-17, the expression vector of claim 18, or the pharmaceutical composition of claim 21, for the preparation of a medicament for the prevention and treatment of cancer, preferably for the treatment of gastric cancer.

24. A pharmaceutical combination comprising
the truncated TGFβR2 extracellular domain molecule of claim 1 or 2, the fusion protein of any one of claims 3-8 and 12-14, the antibody or antigen-binding fragment thereof of any one of claims 9-11, the conjugate as claimed in claim 15, the nucleic acid of any one of claims 16-17, the expression vector of claim 18, or the pharmaceutical composition of claim 21, and
one or more additional therapeutic agent(s).

25. A kit comprising
thetruncated TGFβR2 extracellular domain molecule of claim 1 or 2, the fusion protein of any one of claims 3-8 and 12-14, the antibody or antigen-binding fragment thereof of any one of claims 9-11, the conjugate as claimed in claim 15, the nucleic acid of any one of claims 16-17, the expression vector of claim 18, or the pharmaceutical composition of claim 21; preferably,
further comprising a device for administering the medicament.

26. A method of preventing and treating a neoplastic disease comprising administering to a subjectthe truncated TGFβR2 extracellular domain molecule of claim 1 or 2, the fusion protein of any one of claims 3-8 and 12-14, the antibody or antigen-binding fragment thereof of any one of claims 9-11, the conjugate as claimed in claim 15, the nucleic acid of any one of claims 16-17, the expression vector of claim 18, the pharmaceutical composition of claim 21, the pharmaceutical combination of claim 24, or the kit of claim 25.
